# EUROPEAN PATENT APPLICATION

(11) **EP 4 039 806 A1**
(43) Date of publication of application: **10.08.2022**
(21) Application number: 21155245.0
(22) Date of filing: 04.02.2021
(51) Int. Cl.: C12N 9/42, C11D 3/386, C12N 9/88, C12N 9/96

(54) **DETERGENT COMPOSITION COMPRISING XANTHAN LYASE AND ENDOGLUCANASE VARIANTS WITH IM-PROVED STABILITY**

(71) Applicant: Henkel AG & Co. KGaA, 40589 Düsseldorf (DE); Novozymes A/S, 2880 Bagsvaerd (DK)
(72) Inventor: Degering, Christian, 40699 Erkrath (DE); Vockenroth, Inga Kerstin, 40227 Düsseldorf (DE); Laufs, Brian, 41363 Jüchen (DE); Tkacz, Piotr, 40789 Monheim am Rhein Düsseldorf (DE); Pedersen, Asbjørn Toftgaard, 2880 Bagsvaerd (DK)

(57) **Abstract**

The present invention relates to a detergent composition comprising endoglucanase variants and xanthan lyase variants and methods for use of said compositions.

SEQUENCE LISTING

<110>  Henkel AG & Co. KGaA

<120>  Detergent  composition comprising xanthan lyase and endoglucanase
        variants with improved stability

<130>  2020P00336EP

<160>  2

<170>  PatentIn version 3.5

<210>  1
<211>  1050
<212>  PRT
<213>  Paenibacillus sp.

<400>  1

Ile Ala Gly Val Val Gln Ser Val Asn Val Ser Gln Ala Gly Tyr Ser
1               5                   10                  15

Ser Asn Asp Phe Lys Thr Ala Thr Val Thr Ala Ser Asp Lys Leu Ser
            20                  25                  30

Asp Thr Ser Tyr Gln Ile Leu Gln Gly Thr Thr Val Ile Ala Thr Gly
        35                  40                  45

Thr Met Lys Asp Glu Gly Tyr Val Trp Gly Lys Tyr Val Tyr Ser Ile
    50                  55                  60

Asp Phe Ser Ser Val Thr Ala Thr Gly Thr Asn Phe Thr Ile Arg Ser
65                  70                  75                  80

Asn Gly Val Ser Ser Tyr Thr Phe Pro Ile Gln Thr Asn Met Trp Asn
                85                  90                  95

Glu Tyr Lys Asp Glu Met Thr Ala Phe Tyr Arg Leu Leu Arg Thr Thr
            100                 105                 110

Asp Thr Phe Ala Ala Tyr Pro Ala Gly Tyr Ser Asn Ile Ala Pro Ser
        115                 120                 125

Asn Lys Ile Leu His Pro Asp Ser Phe Leu Asp Asp Ala Phe Ser Pro
    130                 135                 140

Asp Arg Thr Thr His Tyr Asp Leu Thr Gly Gly Trp Phe Asp Ala Gly
145                 150                 155                 160

Asp Tyr Gly Lys Tyr Gly Gly Asn Gln Trp Val Gln Gly Asn Ile Ala
                165                 170                 175

Ile Ser Tyr Leu Arg His Ala Ser Ser Ala Ala Val Asn Phe Asp Lys
            180                 185                 190

Asp Thr Asn Gly Ile Pro Asp Leu Val Asp Glu Ala Ile Phe Gly Ser
        195                 200                 205

Gln Tyr Leu Val Lys Phe Ala Asn Gln Leu Gly Gly Ala Ile His Asn
    210                 215                 220

Ile Leu Arg Lys Gly Gly Phe Val Leu Pro His Lys Val Thr Asp Asn
225                 230                 235                 240

Val Pro Gly Asn Thr Asp Asp Arg Ala Leu Glu Ala Val Glu Ala Val
                245                 250                 255

Gly Gly Ser Gly Lys Ser Ser Gly Ser Leu Ala Ala Thr Ala Arg Ala
            260                 265                 270

Ile Arg Thr Ala Ile Ala Gly Gly Lys Val Ala Ala Asn Lys Val Ala
        275                 280                 285

Gln Leu Gln Thr Leu Ala Asn Glu Phe Gln Ala Ala Ala Ile Ile Phe
    290                 295                 300

Tyr Asn Tyr Thr Leu Thr His Gln Ser Gly Asn His Gly Ser Tyr Gly
305                 310                 315                 320

Thr Met Asn Asn Gly Gly Ile Ala Asn Pro Leu Leu Trp Ala Glu Val
                325                 330                 335

Gln Leu Tyr Leu Leu Thr Gly Asp Ala Ala Tyr Lys Thr Gln Ala Gln
            340                 345                 350

Thr Arg Ile Asn Ala Ile Asn Glu Ala Tyr Val Ser Ser Thr Asn Tyr
        355                 360                 365

Trp Asp Met His Pro Ile Ala Leu Ala Glu Phe Tyr Pro Val Ala Asp
    370                 375                 380

Ser Ala Ile Lys Thr Lys Ile Gln Ser Ile Leu Lys His Gln Ala Tyr
385                 390                 395                 400

Tyr Phe Ile Thr Leu Met Asp Glu Thr Pro Tyr Gly Val Leu Asn Gln
                405                 410                 415

Phe Gly Asn Phe Gly Val Asn Glu Pro His Ala Ser Tyr Met Ala Asp
            420                 425                 430

Leu Leu Arg Tyr Tyr Glu Leu Phe Asn Asp Pro Val Ala Leu Arg Ala
        435                 440                 445

Ala Lys Lys Ala Leu Tyr Trp Ile Val Gly Asn Asn Pro Trp Asn Ile
    450                 455                 460

Ser Trp Val Ser Gly Val Gly Ser Asn Phe Thr Asp Phe Leu His Thr
465                 470                 475                 480

Arg Leu Asp Glu Glu Ala Tyr Ser Gln Thr Asn Thr Gly Val Val Leu
                485                 490                 495

Pro Gly Ala Met Val Ser Gly Pro Asn Ile Lys Asp Pro Asn Asn Lys
            500                 505                 510

Leu Ser Ser Ser Pro Trp Tyr Glu Asp Lys Pro Ile Trp Ala Asp Asp
        515                 520                 525

Thr Asn Gln Trp Arg Tyr Asn Glu Tyr Ser Val Ser Ile Gln Thr Gly
    530                 535                 540

Leu Phe Tyr Thr Ile Met Gly Leu Ser Ala Leu Gly Gly Asn Ala Ser
545                 550                 555                 560

Thr Gly Gly Ala Glu Pro Val Lys Leu Pro Ile Thr Trp Pro Ile Ile
                565                 570                 575

Gly Asp Tyr Val Thr Gly Asp Val Thr Val Phe Ala Gln Pro Glu Gly
            580                 585                 590

Ser Leu Ser Asn Val Ser Ala Asn Gly Ile Val Leu Ser Pro Ser Asp
        595                 600                 605

Gly Val Tyr Thr Thr Thr Val Ser Thr Ser Ala Asp Ala Pro Tyr Thr
    610                 615                 620

Glu Arg Lys Val Gln Ile Lys Gly Thr Asp Asp Ser Gly Phe Thr Thr
625                 630                 635                 640

Tyr Ser Asn Thr His Phe Thr Val Ala Pro Ala Leu Pro Asp Pro Ser
                645                 650                 655

His Pro Leu Leu Phe Asp Asp Phe Asn Gln Lys Gly Ile Trp Gly Ser
            660                 665                 670

Gln Lys Leu Asp Trp Val Asn Trp Tyr Asn Gln Asn Gly Gly Thr Ala

        675                 680                 685

Ser Tyr Thr Arg Thr Thr Val Asp Thr Arg Thr Val Gly Lys Phe Ala
    690                 695                 700

His Thr Pro Ala Ala Thr Thr Ser Lys Ala Lys Phe Gln Pro Trp Lys
705                 710                 715                 720

Tyr Asn Ala Asn Leu Asn Gly Tyr Arg Tyr Leu Asn Phe Thr Met Lys
                725                 730                 735

Asn Pro Gly Tyr Pro Asn Thr Lys Ile Arg Ile Ala Ala Asn Asp Gly
            740                 745                 750

Thr Lys Ser Val Asn Leu Thr Ser Gly Glu Val Ala Ile Ser Ser Thr
        755                 760                 765

Trp Thr Thr Tyr Gln Tyr Asp Leu Asn Leu His Pro Thr Leu Asn Lys
    770                 775                 780

Ser Asn Val Leu Ile Glu Val Trp Leu Ser Asn Pro Thr Ala Gly Ala
785                 790                 795                 800

Tyr Gly Glu Ile Leu Ile Asp Glu Ile Ser Ala Val Asn Thr Asn Ser
                805                 810                 815

Gly Thr Ala Pro Thr Leu Ser Ala Thr Gly Val Asn Ala Ser Ile Gly
            820                 825                 830

Asn Gln Ser Thr Val Phe Thr Tyr Thr Ala Thr Tyr Thr Asp Ala Asn
        835                 840                 845

Asn Gln Ala Pro Phe Asp Val Gln Val Val Ile Asp Gly Val Ile Arg
    850                 855                 860

Ser Met Thr Ala Ala Asp Pro Thr Asp Thr Thr Tyr Ser Asp Gly Arg
865                 870                 875                 880

Val Tyr Thr Tyr Ala Thr Thr Leu Pro Val Gly Thr His Lys Phe Tyr
                885                 890                 895

Phe Arg Thr Thr Asp Thr Thr Thr Asn Phe Val Ser Thr Ser Val Gln
            900                 905                 910

Thr Gly Pro Thr Val Ile Arg Asn Lys Leu Glu Ala Glu Val Leu Ser
        915                 920                 925

Ile Asn Leu Thr Asn Tyr Thr His Ala Val Lys Asp Asn Ala Asp Ala
    930                 935                 940

Ser Gly Gly Lys Tyr Arg Leu Phe Asn Gly Arg Gln Ala Asn Asp Tyr
945                 950                 955                 960

Ile Glu Tyr Ala Val Asn Val Pro Lys Ala Gly Thr Tyr Gln Val Ser
                965                 970                 975

Ala Arg Ala Met Arg Leu Ser Asp Asn Gly Ile Tyr Gln Leu Gln Ile
            980                 985                 990

Asn Gly Ser Asn Gln Gly Thr Pro Phe Asp Thr Tyr Gln Ser Ser Gly
        995                 1000                1005

Lys Tyr Leu Asp Tyr Ala Leu Gly Asn Val Thr Ile Thr Ser Pro
    1010                1015                1020

Gly Thr Gln Leu Phe Arg Phe Lys Val Thr Gly Lys Asn Ala Ser
    1025                1030                1035

Ser Leu Gly Tyr Lys Leu Pro Leu Asp Phe Ile Gln
    1040                1045                1050

<210> 2
<211> 1037
<212> PRT
<213> Paenibacillus sp.

<400> 2

Asp Glu Phe Asp Thr Leu Arg Glu Lys Tyr Lys Ala Met Leu Asn Gly
1               5                   10                  15

Gly Thr Thr Tyr Asn Leu Ser Asp Pro Asp Ile Ala Ala Arg Val Asn
            20                  25                  30

Ala Ile Thr Val Thr Ala Gln Gly Tyr Trp Asp Ser Met Leu Lys Asp
        35                  40                  45

Pro Asn Arg Asn Arg Leu Trp Asn Asp Ala Pro Phe Gly Ser Asp Ser
    50                  55                  60

Thr Ser Ile Thr Thr Thr Tyr Arg His Leu Tyr Asp Met Ala Leu Ala
65                  70                  75                  80

Tyr Thr Thr Tyr Gly Ser Ser Leu Gln Gly Asn Ala Ala Leu Lys Ala
                85                  90                  95

Asp Ile Ile Ser Gly Leu Asp Trp Met Asn Ala Asn Gln Phe Tyr Asn
            100                 105                 110

Gly Cys Ser Gln Tyr Gln Asn Trp Trp His Trp Gln Ile Gly Gly Pro
        115                 120                 125

Met Ala Leu Asn Asp Ile Val Ala Leu Met Tyr Thr Glu Leu Thr Ala
    130                 135                 140

Thr Gln Ile Ser Asn Tyr Met Ala Ala Ile Tyr Tyr Thr Gln Ala Ser
145                 150                 155                 160

Val Thr Met Thr Gly Ala Asn Arg Leu Trp Glu Ser Gln Val Ile Ala
                165                 170                 175

Ile Ser Gly Ile Leu Asn Lys Asp Ser Ala Arg Val Ala Ala Gly Arg
            180                 185                 190

Asp Gly Ile Ser Ala Leu Leu Pro Tyr Val Ala Lys Gly Asp Gly Phe
        195                 200                 205

Tyr Asn Asp Gly Ser Phe Val Gln His Thr Tyr Tyr Ala Tyr Asn Gly
    210                 215                 220

Gly Tyr Gly Ser Glu Leu Leu Ser Gly Ile Ala Asp Leu Ile Phe Ile
225                 230                 235                 240

Leu Asn Gly Ser Ser Trp Gln Val Thr Asp Pro Asn Lys Asn Asn Val
                245                 250                 255

Tyr Arg Trp Ile Tyr Asp Ser Tyr Glu Pro Phe Ile Tyr Lys Gly Asn
            260                 265                 270

Leu Met Asp Met Val Arg Gly Arg Glu Ile Ser Arg His Gly Leu Gln
        275                 280                 285

Asp Asp Lys Ala Ala Val Thr Val Met Ala Ser Ile Ile Arg Leu Ser
    290                 295                 300

Gln Thr Ala Ala Ser Ala Asp Ala Thr Ala Phe Lys Arg Met Val Lys
305                 310                 315                 320

Tyr Trp Leu Leu Leu Asp Thr Asp Lys Thr Phe Leu Lys Ala Val Ser
                325                 330                 335

Ile Asp Leu Ile Ile Ala Ala Asn Gln Leu Val Asn Asp Ser Thr Val
            340                 345                 350

Thr Ser Arg Gly Glu Leu Val Lys Tyr Lys Gln Phe Ser Gly Met Asp
        355                 360                 365

Arg Ala Val Gln Leu Arg Pro Gly Phe Gly Phe Gly Leu Ser Met Phe
    370                 375                 380

Ser Ser Arg Ile Gly Asn Tyr Glu Ser Ile Asn Ala Glu Asn Asn Lys
385                 390                 395                 400

Gly Trp His Thr Gly Asp Gly Met Thr Tyr Leu Tyr Asn Thr Asp Leu
                405                 410                 415

Ser Gln Phe Asn Asp His Phe Trp Ala Thr Val Asp Asn Tyr Arg Leu
            420                 425                 430

Pro Gly Thr Thr Val Leu Gln Asn Thr Thr Gln Thr Ala Asn Ser Arg
        435                 440                 445

Ser Asp Lys Ser Trp Ala Gly Gly Thr Asp Ile Leu Gly Gln Tyr Gly
    450                 455                 460

Val Ser Gly Met Glu Leu His Thr Val Gly Lys Ser Leu Thr Ala Lys
465                 470                 475                 480

Lys Ser Trp Phe Met Phe Asp Asp Glu Ile Val Ala Leu Gly Ser Gly
                485                 490                 495

Ile Ala Ser Thr Asp Gly Ile Ala Thr Glu Thr Ile Val Glu Asn Arg
            500                 505                 510

Lys Leu Asn Ser Ser Gly Asn Asn Ala Leu Ile Val Asn Gly Thr Ala
        515                 520                 525

Lys Pro Gly Ser Leu Gly Trp Ser Glu Thr Met Thr Gly Thr Asn Tyr
    530                 535                 540

Ile His Leu Ala Gly Ser Val Pro Gly Ser Asp Ile Gly Tyr Tyr Phe
545                 550                 555                 560

Pro Gly Gly Ala Ala Val Lys Gly Leu Arg Glu Ala Arg Ser Gly Ser
                565                 570                 575

Trp Ser Ser Leu Asn Ser Ser Ala Ser Trp Lys Asp Ser Thr Leu His
            580                 585                 590

Thr Arg Asn Phe Met Thr Leu Trp Phe Asp His Gly Met Asn Pro Thr
        595                 600                 605

Asn Gly Ser Tyr Ser Tyr Val Leu Leu Pro Asn Lys Thr Ser Ser Ala
    610                 615                 620

Val Ala Ser Tyr Ala Ala Thr Pro Gln Ile Ser Ile Leu Glu Asn Ser
625                 630                 635                 640

Ser Ser Ala Gln Ala Val Lys Glu Thr Gln Leu Asn Val Thr Gly Ile
                645                 650                 655

Asn Phe Trp Asn Asp Glu Pro Thr Thr Val Gly Leu Val Thr Ser Asn
            660                 665                 670

Arg Lys Ala Ser Val Met Thr Lys Glu Thr Ala Ser Asp Phe Glu Ile
        675                 680                 685

Ser Val Ser Asp Pro Thr Gln Ser Asn Val Gly Thr Ile Tyr Ile Asp
    690                 695                 700

Val Asn Lys Ser Ala Thr Gly Leu Ile Ser Lys Asp Asn Glu Ile Thr
705                 710                 715                 720

Val Ile Gln Tyr Tyr Pro Thr Met Lys Phe Lys Val Asn Val Asn Asn
                725                 730                 735

Ser Gly Gly Lys Ser Tyr Lys Val Lys Phe Ser Leu Thr Gly Thr Pro
            740                 745                 750

Gly Ser Asn Pro Ser Pro Ile Pro Ile Pro Asn Pro Tyr Glu Ala Glu
        755                 760                 765

Ala Leu Pro Ile Asn Ala Leu Thr Asp Thr Pro Val Val Tyr Asn Asp
    770                 775                 780

Ala Asn Ala Ser Gly Gly Lys Lys Leu Gly Phe Asn Asn Asn Ala Val
785                 790                 795                 800

Asp Asp Tyr Val Glu Phe Ser Leu Asp Val Thr Gln Pro Gly Thr Tyr
                805                 810                 815

Asp Val Lys Ser Arg Ile Met Lys Ser Thr Asn Ser Gly Ile Tyr Gln
            820                 825                 830

Leu Ser Ile Asn Gly Thr Asn Val Gly Ser Ala Gln Asp Met Phe Trp
        835                 840                 845

Thr Thr Ser Glu Leu Ser Lys Glu Phe Thr Met Gly Ser Tyr Ser Phe

850                 855                 860

Ser Thr Pro Gly Ser Tyr Leu Phe Arg Leu Lys Thr Thr Gly Lys Asn
865                 870                 875                 880

Val Ser Ser Ser Gly Tyr Lys Leu Met Leu Asp Asn Phe Ser Leu Val
                885                 890                 895

Ser Thr Gly Ile Asp Thr Thr Val Ile Val Asp Asn Ala Asp Ala Ala
            900                 905                 910

Gly Val Thr Lys Val Gly Thr Trp Thr Gly Thr Asn Thr Gln Thr Asp
        915                 920                 925

Arg Tyr Gly Ala Asp Tyr Ile His Asp Gly Asn Thr Gly Lys Gly Thr
    930                 935                 940

Lys Ser Val Thr Phe Thr Pro Asn Val Pro Ile Ser Gly Thr Tyr Gln
945                 950                 955                 960

Val Tyr Met Met Trp Ala Ala His Thr Asn Arg Ala Thr Asn Val Pro
                965                 970                 975

Val Asp Val Thr His Ser Gly Gly Thr Ala Thr Leu Asn Val Asn Gln
            980                 985                 990

Gln Gly Asn Gly Gly Val Trp Asn  Leu Leu Gly Thr Tyr  Ser Phe Asn
        995                 1000                1005

Ala Gly  Ser Thr Gly Ala Ile  Lys Ile Arg Thr Asp  Ala Thr Asn
    1010                1015                1020

Gly Tyr  Val Val Ala Asp Ala  Val Lys Leu Val Lys  Val Pro
    1025                1030                1035

## Description

### Reference to a Sequence Listing

This application contains a Sequence Listing in computer readable form, which is incorporated herein by reference.

### BACKGROUND OF THE INVENTION

### Field of the Invention

The present invention relates to a detergent composition, such as laundry compositions and dish wash compositions, including hand wash and automatic dish wash compositions, comprising xanthan lyase or endoglucanase or mixtures thereof and at least one enzyme stabilizing component, in order to improve at least one or more properties including detergent stability (e.g. improved stability in a detergent composition) and/or storage stability (e.g. improved storage stability in a detergent composition). The invention also relates to methods for producing and using such compositions as well as to the use of specific enzyme stabilizing components for improving storage stability of xanthan lyase or endoglucanase or mixtures thereof. The variants described herein are particularly suitable for use in cleaning processes and detergent compositions, in particular laundry detergents.

### Description of the Related Art

Xanthan gum is a polysaccharide derived from the bacterial coat of *Xanthomonas campestris.* It is produced by the fermentation of glucose, sucrose, or lactose by the *Xanthomonas campestris* bacterium. After a fermentation period, the polysaccharide is precipitated from a growth medium with isopropyl alcohol, dried, and ground into a fine powder. Later, it is added to a liquid medium to form the gum. Xanthan gum is a natural polysaccharide consisting of different sugars which are connected by several different bonds, such as β-D-mannosyl-β-D-1,4-glucuronosyl bonds and β-D-glucosyl-β-D-1,4-glucosyl bonds. Xanthan gum is at least partly soluble in water and forms highly viscous solutions or gels. Complete enzymatic degradation of xanthan gum requires several enzymatic activities including xanthan lyase activity and endo-β-1,4-glucanase activity. Xanthan lyases are enzymes that cleave the β-D-mannosyl-β-D-1,4-glucuronosyl bond of xanthan and have been described in the literature. Xanthan lyases are known in the art, e.g. two xanthan lyases have been isolated from *Paenibacillus alginolyticus* XL-1 (e.g. Ruijssenaars et al. (1999) 'A pyruvated mannose-specific xanthan lyase involved in xanthan degradation by Paenibacillus alginolyticus XL-1', Appl. Environ. Microbiol. 65(6): 2446-2452, and Ruijssenaars et al. (2000), 'A novel gene encoding xanthan lyase of Paenibacillus alginolyticus strain XL-1', Appl. Environ. Microbiol. 66(9): 3945-3950).

Glycoside hydrolases are enzymes that catalyze the hydrolysis of the glycosyl bond to release smaller sugars. There are over 100 classes of glycoside hydrolases which have been classified, see e.g. Henrissat et al. (1991) 'A classification of glycosyl hydrolases based on amino-acid sequence similarities', J. Biochem. 280: 309-316 and the Uniprot website at www.cazy.org. The glycoside hydrolase family 9 (GH9) consists of over 70 different enzymes that are mostly endoglucanases (EC 3.2.1.4), cellobiohydrolases (EC 3.2.1.91), β-glucosidases (EC 3.2.1.21) and exo-β-glucosaminidase (EC 3.2.1.165).

In recent years, xanthan gum has been used as an ingredient in many consumer products including foods (e.g. as thickening agent in salad dressings and dairy products) and cosmetics (e.g. as stabilizer and thickener in toothpaste and make-up, creams and lotions to prevent ingredients from separating and to provide the right texture of the product). Further xanthan gum has found use in the oil industry as an additive to regulate the viscosity of drilling fluids etc. The widespread use of xanthan gum has led to a desire to degrade solutions, gels or mixtures containing xanthan gum thereby allowing easier removal of the byproducts. Endoglucanases and xanthan lyases for the degradation of xanthan gum and the use of such enzymes for cleaning purposes, such as the removal of xanthan gum containing stains, and in the drilling and oil industries are known in the art, e.g. WO 2013/167581, WO 2018/037064, WO 2018/037065 or WO 2019/162000.

The known endoglucanase having SEQ ID NO:1 and the known xanthan lyase having SEQ ID NO:2 were both found to be sensitive to the presence of detergents, in particular with respect to certain detergent components such as chelators and/or surfactants and/or further enzymes such as proteases. To improve the applicability and/or cost and/or performance of such enzymes, there is an ongoing search for variants with altered properties, such as increased stability, e.g. improved stability in a detergent composition as such or improved stability after storage. However, mutagenesis of large enzymes followed by purification and functional analysis of mutant libraries can be very expensive and laborious. Hence, there is ongoing need for stabilized endoglucanase variants and/or xanthan lyase variants for the use in detergent compositions resp. for detergent compositions with improved enzyme stabilization, in particular for detergent compositions in which the enzyme mix containing endoglucanase and/or xanthan lyase shows improved stabilization.

### SUMMARY OF THE INVENTION

In some aspects, the present invention relates to a detergent composition comprising
(A) an endoglucanase variant, comprising an alteration, preferably a substitution, at one or more positions in one or more regions selected from the group consisting of region 7 corresponding to amino acids 612 to 660 of SEQ ID NO:1, region 1 corresponding to amino acids 95 to 105 of SEQ ID NO:1, region 2 corresponding to amino acids 115 to 138 of SEQ ID NO:1, region 3 corresponding to amino acids 210 to 251 of SEQ ID NO:1, region 4 corresponding to amino acids 267 to 301 of SEQ ID NO:1, region 5 corresponding to amino acids 339 to 361 of SEQ ID NO:1, region 6 corresponding to amino acids 547 to 595 of SEQ ID NO:1, region 8 corresponding to amino acids 806 to 828 of SEQ ID NO:1, region 9 corresponding to amino acids 839 to 1042 of SEQ ID NO:1, region 10 corresponding to amino acids 1 to 94 of SEQ ID NO:1, region 11 corresponding to amino acids 106 to 114 of SEQ ID NO:1, region 12 corresponding to amino acids 139 to 209 of SEQ ID NO:1, region 13 corresponding to amino acids 252 to 266 of SEQ ID NO:1, region 14 corresponding to amino acids 302 to 338 of SEQ ID NO:1, region 15 corresponding to amino acids 362 to 546 of SEQ ID NO:1, region 16 corresponding to amino acids 596 to 611 of SEQ ID NO:1, region 17 corresponding to amino acids 661 to 805 of SEQ ID NO:1, region 18 corresponding to amino acids 829 to 838 of SEQ ID NO:1, and region 19 corresponding to amino acids 1043 to 1055 of SEQ ID NO:1; and/or
(B) a xanthan lyase variant, comprising an alteration, preferably a substitution, at one or more positions in one or more regions selected from the group consisting of region 3 corresponding to amino acids 731 to 803 of SEQ ID NO:2, region 1 corresponding to amino acids 154 to 176 of SEQ ID NO:2, region 2 corresponding to amino acids 614 to 658 of SEQ ID NO:2, region 4 corresponding to amino acids 807 to 846 of SEQ ID NO:2, region 5 corresponding to amino acids 872 to 885 of SEQ ID NO:2, region 6 corresponding to amino acids 903 to 1004 of SEQ ID NO:2, region 7 corresponding to amino acids 1 to 153 of SEQ ID NO:2, region 8 corresponding to amino acids 177 to 613 of SEQ ID NO:2, region 9 corresponding to amino acids 659 to 730 of SEQ ID NO:2, region 10 corresponding to amino acids 804 to 806 of SEQ ID NO:2, region 11 corresponding to amino acids 847 to 871 of SEQ ID NO:2, region 12 corresponding to amino acids 886 to 902 of SEQ ID NO:2, and region 13 corresponding to amino acids 1005 to 1037 of SEQ ID NO:2; and
(C) an enzyme stabilizing component.

In various aspects, the endoglucanase variant (A) has at least 60% and less than 100% sequence identity to SEQ ID NO:1; preferably said endoglucanase variant has activity on xanthan gum pre-treated with xanthan lyase; and/or the xanthan lyase variant (B) has at least 60% and less than 100% sequence identity to SEQ ID NO:2, preferably said xanthan lyase variant having an activity on xanthan gum.

In further aspects, the enzyme stabilizing component is selected from the group consisting of polyols such as glycerol or 1,2-propylene glycol, alditols such as non-cyclic polyols having the formula HOCH₂[CH(OH)]ₙCH₂OH, mannitol, isomalt, lactit, sorbitol, xylitol, threitol, erythritol or arabitol, alkanol amines such as ethanol amine, or formates such as sodium formate, potassium formate, calcium formate or magnesium formate, preferably said enzyme stabilizing component is selected from glycerol and sodium formate, more preferably being sodium formate.

In some aspects, the detergent composition of the present invention, as defined herein, comprises an endoglucanase variant having at least 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% and less than 100% sequence identity to SEQ ID NO:1.

In some aspects, the detergent composition of the present invention, as defined herein, comprises a xanthan lyase variant having at least 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% and less than 100% sequence identity to SEQ ID NO:2.

In some aspects, the detergent composition of the present invention comprises an endoglucanase variant comprising an alteration, preferably a substitution, at one or more positions selected from the group consisting of 4, 17, 18, 20, 51, 53, 55, 56, 60, 63, 71, 79, 87, 92, 99, 120, 125, 126, 130, 137, 182, 186, 189, 192, 213, 216, 221, 226, 228, 230, 231, 232, 233, 235, 240, 243, 247, 249, 278, 279, 281, 283, 285, 289, 292, 294, 298, 302, 311, 313, 333, 346, 353, 358, 386, 387, 388, 390, 403, 408, 410, 416, 441, 448, 451, 471, 472, 476, 489, 507, 512, 515, 538, 555, 556, 557, 558, 559, 560, 561, 562, 563, 564, 567, 568, 570, 575, 578, 579, 580, 581, 583, 589, 590, 591, 592, 593, 595, 598, 599, 602, 603, 605, 607, 609, 616, 627, 630, 631, 635, 636, 638, 639, 640, 641, 642, 643, 644, 651, 676, 683, 688, 690, 694, 698, 699, 706, 711, 713, 719, 720, 744, 749, 754, 756, 760, 781, 786, 797, 810, 811, 812, 815, 823, 824, 825, 827, 828, 833, 834, 835, 837, 843, 848, 868, 869, 870, 871, 872, 873, 874, 880, 881, 883, 884, 885, 887, 888, 890, 892, 894, 898, 905, 906, 912, 920, 921, 924, 926, 927, 928, 932, 933, 934, 935, 937, 938, 939, 940, 941, 942, 943, 946, 948, 950, 952, 953, 954, 956, 957, 960, 966, 971, 972, 980, 989, 991, 994, 995, 998, 999, 1006, 1009, 1010, 1011, 1029, 1030, 1031, 1032, 1035, 1037, 1038, 1040, 1041, 1042, 1044, 1045 and 1048, preferably at one or more positions selected from the group consisting of 17, 20, 216, 283, 302, 311, 313, 408, 476, 579, 602, 627, 636, 638, 651, 688, 697, 719, 756, 880, 881, 883, 887, 906, 921, 934, 937, and 956, wherein said positions correspond to amino acid positions of SEQ ID NO:1.

In some aspects, the detergent composition of the present invention comprises a xanthan lyase variant comprising an alteration, preferably a substitution, at one or more positions selected from the group consisting of 9, 15, 18, 46, 58, 66, 89, 95, 100, 106, 109, 155, 159, 183, 188, 190, 203, 204, 221, 229, 234, 238, 240, 242, 243, 257, 258, 284, 291, 293, 316, 317, 320, 324, 329, 333, 339, 341, 352, 354, 360, 372, 377, 399, 400, 419, 440, 450, 451, 454, 458, 481, 492, 505, 533, 567, 568, 576, 578, 579, 582, 620, 624, 626, 631, 635, 649, 650, 656, 664, 672, 703, 722, 726, 727, 728, 738, 745, 752, 753, 754, 757, 769, 775, 777, 779, 782, 786, 789, 792, 796, 799, 801, 819, 824, 843, 851, 855, 856, 867, 875, 887, 892, 899, 900, 901, 902, 903, 911, 912, 915, 919, 921, 923, 925, 927, 928, 930, 933, 941, 966, 991, 1008 and 1016, wherein said positions correspond to amino acid positions of SEQ ID NO:2.

In some aspects, the aforementioned variants have at least 60% and less than 100% sequence identity to SEQ ID NO:2, preferably said xanthan lyase variant having an activity on xanthan gum.

In some aspects, the detergent composition of the present invention comprises an enzyme stabilizing component being selected from the group consisting of polyols such as glycerol or 1,2-propylene glycol, alditols such as non-cyclic polyols having the formula HOCH₂[CH(OH)]ₙCH₂OH, mannitol, isomalt, lactit, sorbitol, xylitol, threitol, erythritol or arabitol, alkanol amines such as ethanol amine, or formates such as sodium formate, potassium formate, calcium formate or magnesium formate.

In particular aspects, the detergent composition of the present invention comprises an enzyme stabilizing component being selected from the group consisting of glycerol and sodium formate, more preferably sodium formate.

In some aspects, the detergent composition of the present invention comprises an endoglucanase variant comprising an alteration, preferably a substitution, at one or more positions in: (i) regions 6 and 17; (ii) regions 6, 15 and 17; (iii) regions 10, 12 and 15; (iv) regions 6, 7, 16, and 17; (v) region 6, 9, 10, 12, 15, and 17; (vi) region 14 and 15; (vii) region 9; (viii) 6, 7, 9, 14, 15, 16, and 17; or (ix) 3, 6, 7, 9, 14, 15, 16, and 17; wherein said variant preferably has no alternation in the other regions besides those mentioned.

In further aspects, the detergent composition of the present invention comprises an endoglucanase variant comprising one or more amino acid substitutions selected from the group consisting of V4T, S17A, N18G, F20P, F20N, F20G, F20Y, K51Q, K51H, E53Y, E53P, E53G, Y55M, Y55D, V56M, Y60F, S63F, A71E, T87R, T92S, K99R, A120P, 1125V, A126R, S128X, N129D, K130R, F137L, H182Y, A186P, N189K, K192N, K213R, N216D, N216Q, N216R, A221R, L226K, K228E, K228I, G230F, G230L, G230A, G230H, G230N, G230W, G230T, F231Y, F231N, V232R, V232G, L233H, H235D, N240Q, G243K, G243R, D247N, A249N, A278S, G279E, K281F, K281V, K281Y, K281H, K281Q, K281N, K281W, K281T, K281R, A283D, N285G, N285L, N285M, N285S, N285P, N285T, N285Y, N285H, N285K, N285D, N285W, N285R, Q289E, T292A, T292F, T292L, T292I, T292V, T292S, T292P, T292Y, T292Q, T292N, T292K, T292D, T292G, A294V, F297L, Q298E, I302D, I302H, I302V, I302M, H311 N, S313D, W333L, A346H, A346D, T353D, A386P, I387T, K388R, K390Q, I403Y, E408D, E408N, E408S, E408P, E408A, E408G, P410G, Q416S, Q416D, N441G, A448E, A448W, A448S, K451Q, K451S, G471S, S472Y, D476R, Q489P, K507R, K512P, S515V, S538C, L555Q, G556S, G557R, N558P, N558F, N558K, N558D, N558M, N558Q, N5581, N558Y, N558H, N558E, A559N, A559F, A559M, A559P, A559Y, A559H, A559Q, A559D, A559R, A559G, A559l, A559S, A559K, S560F, S560P, S560K, S560G, S560D, T561E, T561Q, T561S, T561D, T561P, G562W, G562P, G563E, A564I, A564Y, A564H, A564Q, A564K, A564E, A564D, E565M, V567F, V567P, K568R, L569F, L569Y, L569D, L569E, P570F, P570L, P570I, P570M, P570V, P570S, P570T, P570A, P570Y, P570H, P570Q, P570N, P570K, P570E, P570W, P570R, P570G, I575V, I575M, I575A, I575D, I575E, I576F, I576M, I576P, D578R, Y579F, Y579W, V580L, T581M, D583M, Q589G, P590S, P590T, P590E, E591L, G592D, S593P, S593H, S593Q, S593N, S593K, S593D, S593E, S593R, S595L, S598Q, A599S, I602T, I602D, V603P, S605T, S607C, G609E, S616G, S616D, K627L, K627M, K627V, K627S, K627T, K627Q, K627R, I630F, I630V, I630Y, K631R, K631A, T633V, D635L, D635M, D635T, D635A, D635P, D635N, D635K, D635E, D635G, D635W, S636L, S636M, S636A, S636H, S636Q, S636N, S636K, S636R, F638N, F638Y, T639D, F638I, F638V, F638T, F638L, F638H, F638M, T639V, T639S, T639L, T639I, T639M, T639A, T639E, T639W, T639G, T639Y, T639P, T640S, Y641E, S642T, S642N, N643D, N643H, N643T, T644F, A651P, A651S, D676H, Q683E, A688G, Y690F, T694A, T697G, R698W, T699A, T706Q, T711S, T711V, T711Y, K713R, W719R, K720H, K744H, K744Q, A749T, K754R, V756Y, V756H, S760G, T781M, N786K, T797S, S81 0Q, S810R, A811S, V812F, V812I, V812M, V812W, V812R, N815V, N815Y, N815E, N815W, N815R, S823Q, A824T, A824D, T825G, T825N, T825W, T825A, T825D, V827I, V827M, V827S, N828D, N833D, Q834E, S835A, S835D, V837I, T843V, N848D, A868E, A869V, D870F, D870L, D870I, D870M, D870V, D870S, D870T, D870Y, D870H, D870Q, D870N, D870K, D870E, D870W, D870R, D870G, P871F, P871L, P871I, P871M, P871V, P871S, P871T, P871A, P871Y, P871H, P871Q, T872S, T872F, T872A, T872Y, T872H, T872Q, T872N, T872K, T872D, T872E, T872W, T872R, T872G, D873K, D873E, T874V, T874S, T874P, T874A, T874H, T874Q, T874N, T874K, R880K, V881Q, V881T, T883R, T883V, T883C, T883K, Y884H, A885F, A885Q, A885N, T887L, T887I, T887S, T887H, T887R, T887K, L888M, V890R, T892P, T892V, K894E, R898Q, N905D, F906A, Q912V, N920P, N920D, K921R, K921E, K921D, A924D, V926F, V926P, K927R, S928D, T932A, N933V, N933S, Y934G, Y934M, Y934S, Y934A, Y934Q, Y934N, Y934E, Y934W, Y934R, T935W, A937F, A937V, A937S, A937T, A937Q, A937D, A937E, V938l, K939I, K939V, D940E, N941S, N941H, N941D, A942P, A942E, D943Y, D943H, G946R, K948R, K948E, R950V, R950H, R950N, F952S, F952W, N953Y, G954L, Q956Y, Q956S, A957L, A957P, Y960F, A964N, A964C, N966P, N966C, G971A, T972K, Q974K, Q974C, M980I, Q989I, Q991L, Q991I, Q991M, Q991V, Q991T, Q991K, Q991C, G994D, G994N, S995I, S995V, S995Q, S995R, S995C, G998V, G998A, T999R, S1006T, S1006A, S1006K, S1006R, K1009E, Y1010W, L1011 M, L1011S, L1011A, L1011Q, L1011N, L1011D, L1011E, R1029N, F1030M, K1031I, K1031S, K1031T, K1031H, V1032G, K1035A, A1037E, A1037W, S1038L, S1038I, S1038G, L1040N, L1040E, G1041F, G1041R, Y1042N, L1044F, L1044S, L1044N, L1044W, P1045Q, P1045W, and F1048W, wherein said positions correspond to amino acid positions of SEQ ID NO:1.

In further aspects, the detergent composition of the present invention comprises an endoglucanase variant comprising a set of alterations selected from the group consisting of A559K + N558K + S560F + T561P + G562W, A559N + P570Q, A559N + P570T, A559N + Y579F, A559N + Y579F + K627R, A559N + Y579W, A559N + Y579W+ K99R, A559N + Y579W+ S128X + K627R, A559N + Y579W + S128X + S636N, A559N + Y579W + K281R, A559N + Y579W + Q416D + S636N, A559N + Y579W + S560P, A559N + Y579W + A564I, A559N + Y579W + P570N, A559N + Y579W + P570K, A559N + Y579W + P570R, A559N + Y579W + P570A, A559N + Y579W + P570T, A559N + Y579W + P570S, A559N + Y579W+ P570Q, A559N + Y579W+ P570H, A559N + Y579W+ S616D, A559N + Y579W + S616D + K627R, A559N + Y579W + S616D + S636K, A559N + Y579W + S616D + A651P, A559N + Y579W + S616D + K921R, A559N + Y579W + S616D + K921R + Y934G, A559N + Y579W + K627R, A559N + Y579W + K627R + S636N + A651P,A559N + Y579W + K627R + S636N + K921R, A559N + Y579W + K627R + K921R, A559N + Y579W + K627M, A559N + Y579W + K627M + A651P,A559N + Y579W + S636N, A559N + Y579W + S636N + A651P,A559N + Y579W + S636N + K921R, A559N + Y579W + S636K + A651P,A559N + Y579W + S636K + K921R, A559N + Y579W + S636L, A559N + Y579W + F638I, A559N + Y579W + A651P, A559N + Y579W + A651P + K921R + Y934G, A559N + Y579W + A651P + Y934G, A559N + Y579W + D870M, A559N + Y579W + K921R, A559N + Y579W + K921R + A651P, A559N + Y579W + K921R + Y934G, A559N + Y579W + Y934G, A559N + Y579W + K948E, A559N + Y579W + K1009E, A559N + K627R, Y579F + A564E, Y579W+ K99R, Y579W + G562P, Y579W+ A564D, 579W + P570K, Y579W + P570Q, Y579W + K627R, Y579W + S636N, Y579W + F638I, Y579W + A651P, Y579W + K921R, Y579W + Y934G, Y579W + K948E, Y579W+ K1009E, K627R + K51Q, K627R + K451S, K627R + P570Q, K627R + P570T, K627R + S636N, K627R + F638I, K627R + A885F, K627R + Y934G, S636N + P570T, S636N + K921R, S636N + Y934G, F638I + P570K, F638I + P570Q, F638I + P570T, F638I + A651P, F638I + Y934G, F638I + K921R, A651P + P570Q, A651P + P570T, A885F + P570T, A885F + Y934G, K921R + P570Q, K921R + P570T, Y934G + P570T, wherein said positions correspond to amino acid positions of SEQ ID NO:1.

In a preferred aspect, the detergent composition of the present invention comprises an endoglucanase variant comprising an alteration, preferably a substitution, at one or more positions selected from the group consisting of S17A, F20P, N216D, N216Q, A283D, I302D, H311N, S313D, E408D, D476R, Y579W, I602T, K627R, S636N, S636K F638I, F638N, A651P, A688G, T697G, W719R, V756Y, R880K, V881Q, T883R, T887K, F906A, K921R, Y934G, A937E, and Q956Y, wherein said positions correspond to amino acid positions of SEQ ID NO:1.

In a further preferred aspect, the detergent composition of the present invention comprises an endoglucanase variant comprising a set of alterations selected from the group consisting of A559N + Y579W+ T697G, K512P + A559N + Y579W+ T697G, N18G + A71E + A186P + E408D + Y579W + I602T + A651P + A688G + V756Y, N18G + N189K + E408D + A559N + Y579W + A688G + T697G + V756Y + K921R + Y934G, S313D + E408D, R880K + N905D + K921R + Y934G, I302D + S313D + E408D + Y579W + I602T + A651P + T697G + R880K + K921R + Y934G, and N216Q + S313D + E408D + D476R + Y579W + I602T + F638N + A651P + T697G + W719R + R880K + T887K + K921R + Y934G, wherein said positions correspond to amino acid positions of SEQ ID NO:1.

In some aspects, the detergent composition of the present invention comprises an endoglucanase variant having activity on xanthan gum pre-treated with xanthan lyase; preferably said activity comprises endoglucanase EC 3.2.1.4 activity, further preferably said activity is endoglucanase EC 3.2.1.4 activity.

In some aspects, the detergent composition of the present invention comprises an isolated GH9 endoglucanase variant having activity on xanthan gum pre-treated with xanthan lyase.

In some aspects, the xanthan lyase variant, as described herein, is one that comprises an alteration, preferably a substitution, at one or more positions in: (i) regions 3 and 5; (ii) regions 3, 5 and 12; (iii) regions 8, and 9; (iv) regions 2, 3, and 5; (v) regions 2, 3, 5, and 12; (vi) regions 3, 5, 8, 9, and 12; (vii) regions 2, 3, 5, 8, and 9; (viii) 3, 5, 8, 9, and 12; (ix) 2, 3, 5, 8, 9, and 12; (x) region 3; (xi) regions 3, 4 and 5; (xii) regions 7, 8 and 9; (xiii) regions 12 and 13; (xiv) regions 3, 4, 5, 8, 9, and 12; (xv) regions 8, 9, 12, and 13; (xvi) regions 7, 8, 9, 12, and 13; (xvii) regions 3, 4, 5, 7, 8, 9, and 12; and (xviii) regions 3, 4, 5, 7, 8, 9, 12, and 13, wherein said variant preferably has no alteration in the other regions besides those mentioned.

In further aspects, the detergent composition of the present invention comprises a xanthan lyase variant comprising one or more amino acid substitutions selected from the group consisting of K9R, N15T, T18D, L46D, A58L, S66H, Q89Y, K95E, S100D, N106Y, Q109R, Q109D, Q109F, Q109K, Q109A, Y155E, A159P, K183Q, K183R, V188I, A190Q, A203P, K204R, A221P, E229N, E229S, E229V, I234V, I238W, I238L, I238M, I240W, N242S, G243V, Y257W, R258E, R284G, K291R, A293G, A293P, K316R, R317K, K320R, L324Q, K329R, K333R, L339M, I341P, V352I, S354P, K360G, K360R, Q372H, F377Y, N399K, K400R, F419Y, N440K, D450P, K451E, K451R, A454V, D458S, K481R, A492H, A492L, T505I, L533I, K567R, G568A, S578K, S578N, S578R, S579R, S579K, S582K, K620R, A624E, A626Q, T631N, S635E, S635T, T649V, T649K, Q650G, I656V, T664K, N672D, I703L, I722F, P726Q, T727P, M728V, G738L, K745R, P752R, P752K, G753E, G753Q, G753S, S754E, S754L, S754Q, S754R, S757D, S757P, S757E, A769D, A769T, A769R, L775A, L775F, L775I, L775M, L775Q, L775S, L775Y, D777R, P779V, Y782I, N786K, G789R, K792W, K792Y, K792V, K792A, N796Q, A799H, D801G, K819R, K824R, A843P, S851F, K855R, E856D, P867S, K875T, K875E, K887R, N892Y, N892W, N892F, G899S, I900G, D901A, T902F, T903A, T903Q, A911M, A911V, A911S, A912T, A912I, A912Y, T915S, T915V, T915Q, T919D, T919F, T919G, T921R, T921S, T923D, T923H, T925D, T925Q, T925R, T927K, D928W, Y930F, Y930H, Y930L, D933M, G941D, G941E, A966P, N991D, V998K, N1008D and K1016T, wherein said positions correspond to amino acid positions of SEQ ID NO:2.

In a preferred aspect, the detergent composition of the present invention comprises a xanthan lyase variant comprising an alteration at one or more positions selected from the group consisting of 624, 631, 635, 649, 656, 738, 752, 753, 754, 757, 769, 775, 777, 800, 801, 843, 875, 911 and 915, and/or an alteration at one or more positions selected from the group consisting of 89, 100, 190, 229, 234, 352, 360, 399, 440, 458, 492, 567, 582, 664, 672, 703, 728, 892, 1008 and 1016, wherein said positions correspond to amino acid positions of SEQ ID NO:2.

In a further preferred aspect, the detergent composition of the present invention comprises a xanthan lyase variant comprising one or more substitutions selected from the group consisting of Q89Y, S100D, A190Q, E229S, I234V, V352I, K360G, N399K, N440K, D458S, A492H, A492L, K567R, S582K, T664K, N672D, I703L, M728V, N892Y N1008D and K1016T, and/or one or more substitutions selected from the group consisting of A624E, T631N, S635E, T649K, I656V, G738L, P752K, P752R, G753E, S754E, S754R, S757D, A769D, L775A, D777R, V800P, D801G, A843P, K875T, A911V and T915A, wherein said positions correspond to amino acid positions of SEQ ID NO:2.

Non-limiting examples of such variants include: A190Q + E229S + S635E + T649K + 1656V + N672D + 1703L + G753E + S754E + S757D + A769D + L775A + D801G + K875T + N892Y + N1008D, E229S + S635E + T649K + 1656V + N672D + 1703L + G753E + S754E + A769D + L775A + D801G + K875T + N892Y + N1008D, E229S + V3521 + S635E + T649K + I656V + N672D + G753E + S754E + A769D + L775A + V800P + D801G + K875T + N892Y, E229S + K360G + D458S + S582K + N672D + G753E + S754E + A769D + L775A + D801G + K875T + N892Y + N1008D, S100D + E229S + K360G + D458S + S582K + T664K + N672D + G753E + S754E + S757D + A769D + L775A + D801G + A843P + K875T + N892Y + A911V + N1008D + K1016T, E229S + I234V + S582K + N672D + G753E + S754E + A769D + L775A + V800P + D801G + K875T + N892Y, Q89Y + E229S + N440K + S582K + A624E + N672D + G753E + S754E + A769D + L775A + D801G + K875T + N892Y, E229S + S635E + T649K + 1656V + N672D + P752K + G753E + A769D + L775A + D801G + A843P + K875T + N892Y, E229S + S635E + T649K + 1656V + N672D + G753E + S754E + S757D + A769D + L775A + D801G + K875T + N892Y, E229S + N440K + S582K + N672D + G753E + S754E + A769D + L775A + D801G + A843P + K875T + N892Y + N1008D, E229S + N440K + S582K + A624E + N672D + G753E + S754E + A769D + L775A + V800P + D801G + K875T + N892Y, A190Q + E229S + S635E + T649K + 1656V + N672D + P752K + G753E + A769D + L775A + D801G + A843P + K875T + N892Y, A190Q + E229S + S582K + N672D + G753E + S754E + A769D + L775A + D801G + K875T + N892Y, E229S + N440K + S582K + N672D + P752R + G753E + S754E + S757D + A769D + L775A + D801G + K875T + N892Y + N1008D, E229S + S582K + S635E + N672D + P752R + G753E + S754E + A769D + L775A + D801G + K875T + N892Y + N1008D, A190Q + E229S + N440K + S582K + A624E + S635E + N672D + G753E + S754E + A769D + L775A + D801G + K875T + N892Y, E229S + I234V + A492L + S582K + N672D + G753E + S754E + A769D + L775A + D801G + K875T + N892Y, A190Q + E229S + K360G + D458S + S582K + T664K + N672D + G753E + S754E + A769D + L775A + D801G + K875T + N892Y + N1008D, S100D + E229S + K360G + D458S + S582K + N672D + G753E + S754E + S757D + A769D + L775A + D801G + A843P + K875T + N892Y + T915A + N1008D, E229S + N440K + S582K + A624E+ S635E + N672D + G738L + G753E + S754E + S757D + A769D + L775A + D801G + K875T + N892Y, S100D + E229S + K360G + D458S + S582K + N672D + G753E + S754E + A769D + L775A + D801G + K875T + N892Y + N1008D, A190Q + E229S + D458S + T631 N + N672D + G753E + S754E + A769D + L775A + D801G + A843P + K875T + N892Y, A190Q + E229S + K360G + D458S + S582K + N672D + G753E + S754E + A769D + L775A + D801G + K875T + N892Y + N1008D, E229S + S635E + T649K + 1656V + N672D + G753E + S754R + S757D + A769D + L775A + D801G + A843P + K875T + N892Y, E229S + D458S + S582K + T631 N + S635E + N672D + M728V + G753E + S754E + S757D + A769D + L775A + D801G + K875T + N892Y, A190Q + E229S + K360G + D458S + S582K + N672D + G753E + S754E + S757D + A769D + L775A + D801G + K875T + N892Y + N1008D, E229S + A492L + S635E + T649K + 1656V + N672D + G753E + S757D + A769D + L775A + D801G + K875T + N892Y, S100D + A190Q + E229S + K360G + D458S + S582K + N672D + G753E + S754E + A769D + L775A + D801G + K875T + N892Y + N1008D, A190Q + E229S + I234V + S582K + N672D + G753E + S754E + S757D + A769D + L775A + D801G + K875T + N892Y, E229S + N399K + D458S + A492H + K567R + S582K + S635E + T649K + N672D + G753E + S754E + A769D + L775A + D777R + D801G + K875T + N892Y, E229S + D458S + A492L + T631 N + N672D + G753E + S754E + S757D + A769D + L775A + D801G + K875T + N892Y, E229S + D458S + A492H + K567R + S582K + S635E + N672D + G753E + S754E + A769D + L775A + D777R + D801G + K875T + N892Y, S100D + E229S + K360G + D458S + S582K + N672D + G753E + S754E + S757D + A769D + L775A + D801G + A843P + K875T + N892Y + N1008D, E229S + N399K + D458S + K567R + S582K + S635E + N672D + G753E + S754E + A769D + L775A + D777R + D801G + K875T + N892Y.

In a preferred aspect, the detergent composition of the present invention comprises a xanthan lyase variant comprising a set of alterations selected from the group consisting of E229N + N672D + P752K + G753E + A769D + L775A + D801G + K875T + N892Y, E229S + N672D + G753E + S754E + A769D + L775A + D801G + K875T + N892Y, E229S + N672D + P752R + G753E + S754E + A769D + L775A + D801G + K875T + N892Y, A190Q + E229S + I234V + A624E + N672D + G753E + S754E + A769D + L775A + D801G + K875T, A190Q + E229S + T631N + N672D + 1703L + P752K + G753E + A769D + L775A + D801G + K875T, A190Q + E229S + I234V + S582K + N672D + G753E + S754E + S757D + A769D + L775A + D801G + K875T + N892Y, E229S + N440K + S582K + A624E + S635E + N672D + G738L + G753E + S754E + S757D + A769D + L775A + D801G + K875T + N892Y, and S100D + E229S + K360G + D458S + S582K + N672D + G753E + S754E + S757D + A769D + L775A + D801G + A843P + K875T + N892Y + N1008D, wherein said positions correspond to amino acid positions of SEQ ID NO:2.

In some aspects, the detergent composition of the present invention comprises a xanthan lyase variant having activity on xanthan gum; preferably said activity comprises xanthan lyase EC 4.2.2.12 activity, further preferably said activity is xanthan lyase EC 4.2.2.12 activity.

In some aspects, the detergent composition of the present invention, as defined herein, comprising an endoglucanase variant having at least 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NO:1 and comprising a set of alterations selected from the group consisting of A559K + N558K + S560F + T561P + G562W, A559N + P570Q, A559N + P570T, A559N + Y579F, A559N + Y579F + K627R, A559N + Y579W, A559N + Y579W+ K99R, A559N + Y579W+ S128X + K627R, A559N + Y579W+ S128X + S636N, A559N + Y579W+ K281R, A559N + Y579W+ Q416D + S636N, A559N + Y579W + S560P, A559N + Y579W + A564I, A559N + Y579W + P570N, A559N + Y579W + P570K, A559N + Y579W + P570R, A559N + Y579W + P570A, A559N + Y579W + P570T, A559N + Y579W + P570S, A559N + Y579W + P570Q, A559N + Y579W+ P570H, A559N + Y579W + S616D, A559N + Y579W + S616D + K627R, A559N + Y579W + S616D + S636K, A559N + Y579W + S616D + A651P, A559N + Y579W + S616D + K921R, A559N + Y579W+ S616D + K921R + Y934G, A559N + Y579W + K627R, A559N + Y579W + K627R + S636N + A651P,A559N + Y579W + K627R + S636N + K921R, A559N + Y579W + K627R + K921R, A559N + Y579W + K627M, A559N + Y579W + K627M + A651P, A559N + Y579W + S636N, A559N + Y579W + S636N + A651P, A559N + Y579W + S636N + K921R, A559N + Y579W + S636K + A651P, A559N + Y579W + S636K + K921R, A559N + Y579W + S636L, A559N + Y579W + F638I, A559N + Y579W + A651P, A559N + Y579W + A651P + K921R + Y934G, A559N + Y579W + A651P + Y934G, A559N + Y579W + D870M, A559N + Y579W + K921R, A559N + Y579W + K921R + A651P, A559N + Y579W + K921R + Y934G, A559N + Y579W + Y934G, A559N + Y579W + K948E, A559N + Y579W + K1009E, A559N + K627R, Y579F + A564E, Y579W+ K99R, Y579W + G562P, Y579W + A564D, 579W + P570K, Y579W + P570Q, Y579W + K627R, Y579W + S636N, Y579W + F638I, Y579W + A651P, Y579W + K921R, Y579W + Y934G, Y579W + K948E, Y579W+ K1009E, K627R + K51Q, K627R + K451S, K627R + P570Q, K627R + P570T, K627R + S636N, K627R + F638I, K627R + A885F, K627R + Y934G, S636N + P570T, S636N + K921R, S636N + Y934G, F638I + P570K, F638I + P570Q, F638I + P570T, F638I + A651P, F638I + Y934G, F638I + K921R, A651P + P570Q, A651P + P570T, A885F + P570T, A885F + Y934G, K921R + P570Q, K921R + P570T, Y934G + P570T, preferably selected from the group consisting of A559N + Y579W + T697G, K512P + A559N + Y579W + T697G, N18G + A71E + A186P + E408D + Y579W + I602T + A651P + A688G + V756Y, N18G + N189K + E408D + A559N + Y579W + A688G + T697G + V756Y + K921R + Y934G, S313D + E408D, R880K + N905D + K921R + Y934G, I302D + S313D + E408D + Y579W + I602T + A651P + T697G + R880K + K921R + Y934G, and N216Q + S313D + E408D + D476R + Y579W + I602T + F638N + A651P + T697G + W719R + R880K + T887K + K921R + Y934G, wherein said positions correspond to amino acid positions of SEQ ID NO:1.

In a particular aspect, the endoglucanase variant has besides the aforementioned alterations no further alterations relative to the parent enzyme of SEQ ID NO:1, i.e. the remaining sequence is identical to SEQ ID NO:1.

In some aspects, the detergent composition of the present invention, as defined herein, comprises a xanthan lyase variant having at least 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NO:2 and comprising a set of alterations selected from the group consisting of A190Q + E229S + S635E + T649K + I656V + N672D + I703L + G753E + S754E + S757D + A769D + L775A + D801G + K875T + N892Y + N1008D, E229S + S635E + T649K + I656V + N672D + I703L + G753E + S754E + A769D + L775A + D801G + K875T + N892Y + N1008D, E229S + V3521 + S635E + T649K + I656V + N672D + G753E + S754E + A769D + L775A + V800P + D801G + K875T + N892Y, E229S + K360G + D458S + S582K + N672D + G753E + S754E + A769D + L775A + D801G + K875T + N892Y + N1008D, S100D + E229S + K360G + D458S + S582K + T664K + N672D + G753E + S754E + S757D + A769D + L775A + D801G + A843P + K875T + N892Y + A911V + N1008D + K1016T, E229S + I234V + S582K + N672D + G753E + S754E + A769D + L775A + V800P + D801G + K875T + N892Y, Q89Y + E229S + N440K + S582K + A624E + N672D + G753E + S754E + A769D + L775A + D801G + K875T + N892Y, E229S + S635E + T649K + I656V + N672D + P752K + G753E + A769D + L775A + D801G + A843P + K875T + N892Y, E229S + S635E + T649K + I656V + N672D + G753E + S754E + S757D + A769D + L775A + D801G + K875T + N892Y, E229S + N440K + S582K + N672D + G753E + S754E + A769D + L775A + D801G + A843P + K875T + N892Y + N1008D, E229S + N440K + S582K + A624E + N672D + G753E + S754E + A769D + L775A + V800P + D801G + K875T + N892Y, A190Q + E229S + S635E + T649K + I656V + N672D + P752K + G753E + A769D + L775A + D801G + A843P + K875T + N892Y, A190Q + E229S + S582K + N672D + G753E + S754E + A769D + L775A + D801G + K875T + N892Y, E229S + N440K + S582K + N672D + P752R + G753E + S754E + S757D + A769D + L775A + D801G + K875T + N892Y + N1008D, E229S + S582K + S635E + N672D + P752R + G753E + S754E + A769D + L775A + D801G + K875T + N892Y + N1008D, A190Q + E229S + N440K + S582K + A624E + S635E + N672D + G753E + S754E + A769D + L775A + D801G + K875T + N892Y, E229S + I234V + A492L + S582K + N672D + G753E + S754E + A769D + L775A + D801G + K875T + N892Y, A190Q + E229S + K360G + D458S + S582K + T664K + N672D + G753E + S754E + A769D + L775A + D801G + K875T + N892Y + N1008D, S100D + E229S + K360G + D458S + S582K + N672D + G753E + S754E + S757D + A769D + L775A + D801G + A843P + K875T + N892Y + T915A + N1008D, E229S + N440K + S582K + A624E+ S635E + N672D + G738L + G753E + S754E + S757D + A769D + L775A + D801G + K875T + N892Y, S100D + E229S + K360G + D458S + S582K + N672D + G753E + S754E + A769D + L775A + D801G + K875T + N892Y + N1008D, A190Q + E229S + D458S + T631N + N672D + G753E + S754E + A769D + L775A + D801G + A843P + K875T + N892Y, A190Q + E229S + K360G + D458S + S582K + N672D + G753E + S754E + A769D + L775A + D801G + K875T + N892Y + N1008D, E229S + S635E + T649K + I656V + N672D + G753E + S754R + S757D + A769D + L775A + D801G + A843P + K875T + N892Y, E229S + D458S + S582K + T631N + S635E + N672D + M728V + G753E + S754E + S757D + A769D + L775A + D801G + K875T + N892Y, A190Q + E229S + K360G + D458S + S582K + N672D + G753E + S754E + S757D + A769D + L775A + D801G + K875T + N892Y + N1008D, E229S + A492L + S635E + T649K + I656V + N672D + G753E + S757D + A769D + L775A + D801G + K875T + N892Y, S100D + A190Q + E229S + K360G + D458S + S582K + N672D + G753E + S754E + A769D + L775A + D801G + K875T + N892Y + N1008D, A190Q + E229S + I234V + S582K + N672D + G753E + S754E + S757D + A769D + L775A + D801G + K875T + N892Y, E229S + N399K + D458S + A492H + K567R + S582K + S635E + T649K + N672D + G753E + S754E + A769D + L775A + D777R + D801G + K875T + N892Y, E229S + D458S + A492L + T631N + N672D + G753E + S754E + S757D + A769D + L775A + D801G + K875T + N892Y, E229S + D458S + A492H + K567R + S582K + S635E + N672D + G753E + S754E + A769D + L775A + D777R + D801G + K875T + N892Y, S100D + E229S + K360G + D458S + S582K + N672D + G753E + S754E + S757D + A769D + L775A + D801G + A843P + K875T + N892Y + N1008D, E229S + N399K + D458S + K567R + S582K + S635E + N672D + G753E + S754E + A769D + L775A + D777R + D801G + K875T + N892Y, preferably selected from the group consisting of E229N + N672D + P752K + G753E + A769D + L775A + D801G + K875T + N892Y, E229S + N672D + G753E + S754E + A769D + L775A + D801G + K875T + N892Y, E229S + N672D + P752R + G753E + S754E + A769D + L775A + D801G + K875T + N892Y, A190Q + E229S + I234V + A624E + N672D + G753E + S754E + A769D + L775A + D801G + K875T, A190Q + E229S + T631N + N672D + I703L + P752K + G753E + A769D + L775A + D801G + K875T, A190Q + E229S + I234V + S582K + N672D + G753E + S754E + S757D + A769D + L775A + D801G + K875T + N892Y, E229S + N440K + S582K + A624E + S635E + N672D + G738L + G753E + S754E + S757D + A769D + L775A + D801G + K875T + N892Y, and S100D + E229S + K360G + D458S + S582K + N672D + G753E + S754E + S757D + A769D + L775A + D801G + A843P + K875T + N892Y + N1008D, wherein said positions correspond to amino acid positions of SEQ ID NO:2.

In a particular aspect, the xanthan lyase variant has besides the aforementioned alterations no further alterations relative to the parent enzyme of SEQ ID NO:2, i.e. the remaining sequence is identical to SEQ ID NO:2.

In one aspect, the detergent composition of the present invention comprises preferred endoglucanase variants in combination with preferred xanthan lyase variants.

In some aspects, the detergent composition of the present invention thus comprises
(A) an endoglucanase variant having at least 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NO:1 and comprising a set of alterations selected from the group consisting of (A1) A559N + Y579W + T697G, (A2) K512P + A559N + Y579W + T697G, (A3) N18G + A71E + A186P + E408D + Y579W + I602T + A651P + A688G + V756Y, (A4) N18G + N189K + E408D + A559N + Y579W + A688G + T697G + V756Y + K921R + Y934G, (A5) S313D + E408D, (A6) R880K + N905D + K921R + Y934G, (A7) I302D + S313D + E408D + Y579W + I602T + A651P + T697G + R880K + K921R + Y934G, and (A8) N216Q + S313D + E408D + D476R + Y579W + I602T + F638N + A651P + T697G + W719R + R880K + T887K + K921R + Y934G, wherein said positions correspond to amino acid positions of SEQ ID NO:1; and/or
(B) a xanthan lyase variant having at least 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NO:2 and comprising a set of alterations selected from the group consisting of (B1) E229N + N672D + P752K + G753E + A769D + L775A + D801G + K875T + N892Y, (B2) E229S + N672D + G753E + S754E + A769D + L775A + D801G + K875T + N892Y, (B3) E229S + N672D + P752R + G753E + S754E + A769D + L775A + D801G + K875T + N892Y, (B4) A190Q + E229S + I234V + A624E + N672D + G753E + S754E + A769D + L775A + D801G + K875T, (B5) A190Q + E229S + T631N + N672D + I703L + P752K + G753E + A769D + L775A + D801G + K875T, (B6) A190Q + E229S + I234V + S582K + N672D + G753E + S754E + S757D + A769D + L775A + D801G + K875T + N892Y, (B7) E229S + N440K + S582K + A624E + S635E + N672D + G738L + G753E + S754E + S757D + A769D + L775A + D801G + K875T + N892Y, and (B8) S100D + E229S + K360G + D458S + S582K + N672D + G753E + S754E + S757D + A769D + L775A + D801G + A843P + K875T + N892Y + N1008D, wherein said positions correspond to amino acid positions of SEQ ID NO:2; and
(C) an enzyme stabilizing component.

In a particular aspect, detergent composition of the present invention comprises
(A) an endoglucanase variant having at least 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NO:1 and comprising a set of alterations selected from the group consisting of (A1) A559N + Y579W + T697G, (A2) K512P + A559N + Y579W + T697G, (A3) N18G + A71E + A186P + E408D + Y579W + I602T + A651P + A688G + V756Y, (A4) N18G + N189K + E408D + A559N + Y579W + A688G + T697G + V756Y + K921R + Y934G, (A5) S313D + E408D, (A6) R880K + N905D + K921R + Y934G, (A7) I302D + S313D + E408D + Y579W + I602T + A651P + T697G + R880K + K921R + Y934G, and (A8) N216Q + S313D + E408D + D476R + Y579W + I602T + F638N + A651P + T697G + W719R + R880K + T887K + K921R + Y934G, wherein said positions correspond to amino acid positions of SEQ ID NO:1; and
(B) a xanthan lyase variant having at least 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NO:2 and comprising a set of alterations selected from the group consisting of (B1) E229N + N672D + P752K + G753E + A769D + L775A + D801G + K875T + N892Y, (B2) E229S + N672D + G753E + S754E + A769D + L775A + D801G + K875T + N892Y, (B3) E229S + N672D + P752R + G753E + S754E + A769D + L775A + D801G + K875T + N892Y, (B4) A190Q + E229S + I234V + A624E + N672D + G753E + S754E + A769D + L775A + D801G + K875T, (B5) A190Q + E229S + T631N + N672D + I703L + P752K + G753E + A769D + L775A + D801G + K875T, (B6) A190Q + E229S + I234V + S582K + N672D + G753E + S754E + S757D + A769D + L775A + D801G + K875T + N892Y, (B7) E229S + N440K + S582K + A624E + S635E + N672D + G738L + G753E + S754E + S757D + A769D + L775A + D801G + K875T + N892Y, and (B8) S100D + E229S + K360G + D458S + S582K + N672D + G753E + S754E + S757D + A769D + L775A + D801G + A843P + K875T + N892Y + N1008D, wherein said positions correspond to amino acid positions of SEQ ID NO:2; and
(C) an enzyme stabilizing component, wherein the enzyme stabilizing component is selected from the group consisting of polyols such as glycerol or 1,2-propylene glycol, alditols such as non-cyclic polyols having the formula HOCH₂[CH(OH)]ₙCH₂OH, mannitol, isomalt, lactit, sorbitol, xylitol, threitol, erythritol or arabitol, alkanol amines such as ethanol amine, or formates such as sodium formate, potassium formate, calcium formate or magnesium formate, preferably said enzyme stabilizing component is selected from glycerol and sodium formate, more preferably being sodium formate.

In some aspects, the endoglucanase and/or the xanthan lyase variant do not comprise any further substitution besides those explicitly mentioned above, i.e. the remainder of the sequence is identical to that of the parent enzyme as set forth in SEQ ID NO:1 and SEQ ID NO:2, respectively.

In some aspects, the detergent composition of the present invention comprises any one of the endoglucanase variants (A1), (A2), (A3), (A4), (A5), (A6), (A7), and (A8), as defined above, in combination with any one of the xanthan lyase variants (B1), (B2), (B3), (B4), (B5), (B6), (B7) and (B8), as defined above, wherein the respective enzymes preferably have at least 90%, 91%, 92%, 93%, 94%, 95%, 96%, 97%, 98% or 99% sequence identity to SEQ ID NO:1 and SEQ ID NO:2, respectively, preferably are identical to their respective parent sequence with the exception of the substitutions explicitly mentioned herein.

In some aspects, the detergent composition of the present invention additionally comprises one or more further detergent components, preferably a surfactant.

In some aspects, the detergent composition of the present invention comprises 0.000001 to 10 wt.-%, such as 0.00001 to 5 wt.-%, preferably 0.0001 to 1 wt.-%, more preferably 0.0005 to 0.5 wt.-%, even more preferably 0.001 to 0.1 wt.-%, of said endoglucanase variant (active enzyme per ml detergent composition) and/or 0.000001 to 10 wt.-%, such as 0.00001 to 5 wt.-%, preferably 0.0001 to 1 wt.-%, more preferably 0.0005 to 0.5 wt.-%, even more preferably 0.001 to 0.1 wt.-%, of said xanthan lyase variant (active enzyme per ml detergent composition) and/or 0.001 to 20 wt.-%, such as 0.005 to 15 wt.-%, preferably 0.01 to 10 wt.-%, more preferably 0.1 to 5.5 wt.-%, even more preferably 0.25 to 1.5 wt.-%, of said enzyme stabilizing agent (by weight of the detergent composition).

In some aspects, the detergent composition of the present invention comprises 0.1 to 5.5 wt.-%, preferably 0.25 to 1.5 wt.-%, of sodium formate by weight of the detergent composition.

In a further aspect, the detergent composition of the present invention comprises said endoglucanase variant and/or said xanthan lyase variant and said enzyme stabilizing agent in a weight ratio (enzyme:stabilizer) of 0.00001:1 to 0.1:2, preferably 0.0001:1 to 0.01:1, more preferably 0.0005:1 to 0.001:1.

In one aspect, the detergent composition of the present invention comprises said endoglucanase variant and/or said xanthan lyase variant and sodium formate in a weight ratio (enzyme:sodium formate) of 0.0005:1 to 0.001:1, preferably 0.001:1 to 0.02:1, more preferably 0.005:1 to 0.01:1.

In a particular aspect, detergent composition of the present invention thus comprises
(A) 0.0005 to 0.5 wt.-%, preferably 0.001 to 0.1 wt.-%, of an endoglucanase variant having at least 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NO:1 and comprising a set of alterations selected from the group consisting of (A1) A559N + Y579W + T697G, (A2) K512P + A559N + Y579W + T697G, (A3) N18G + A71E + A186P + E408D + Y579W + I602T + A651P + A688G + V756Y, (A4) N18G + N189K + E408D + A559N + Y579W + A688G + T697G + V756Y + K921R + Y934G, (A5) S313D + E408D, (A6) R880K + N905D + K921R + Y934G, (A7) I302D + S313D + E408D + Y579W + I602T + A651P + T697G + R880K + K921R + Y934G, and (A8) N216Q + S313D + E408D + D476R + Y579W+ I602T + F638N + A651P + T697G + W719R + R880K + T887K + K921R + Y934G, wherein said positions correspond to amino acid positions of SEQ ID NO:1;
(B) 0.0005 to 0.5 wt.-%, preferably 0.001 to 0.1 wt.-%, of a xanthan lyase variant having at least 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NO:2 and comprising a set of alterations selected from the group consisting of (B1) E229N + N672D + P752K + G753E + A769D + L775A + D801G + K875T + N892Y, (B2) E229S + N672D + G753E + S754E + A769D + L775A + D801G + K875T + N892Y, (B3) E229S + N672D + P752R + G753E + S754E + A769D + L775A + D801G + K875T + N892Y, (B4) A190Q + E229S + I234V + A624E + N672D + G753E + S754E + A769D + L775A + D801G + K875T, (B5) A190Q + E229S + T631N + N672D + I703L + P752K + G753E + A769D + L775A + D801G + K875T, (B6) A190Q + E229S + I234V + S582K + N672D + G753E + S754E + S757D + A769D + L775A + D801G + K875T + N892Y, (B7) E229S + N440K + S582K + A624E + S635E + N672D + G738L + G753E + S754E + S757D + A769D + L775A + D801G + K875T + N892Y, and (B8) S100D + E229S + K360G + D458S + S582K + N672D + G753E + S754E + S757D + A769D + L775A + D801G + A843P + K875T + N892Y + N1008D, wherein said positions correspond to amino acid positions of SEQ ID NO:2;
(C) 0.1 to 5.5 wt.-%, preferably 0.25 to 1.5 wt.-%, of an enzyme stabilizing component, wherein the enzyme stabilizing component is selected from the group consisting of glycerol and formates, preferably being sodium formate; and
(D) 0 to 99 wt.% of at least one further detergent component, preferably a surfactant.

In some aspects, the present invention relates to use of a composition of the present invention, wherein said use is selected from the group consisting of: use for degrading xanthan gum and use in a cleaning process, such as laundry or hard surface cleaning such as dish wash.

In some aspects, the present invention further relates to the use of a detergent composition of the present invention for degrading xanthan gum, for washing or cleaning textiles and/or hard surfaces, such as dish wash, wherein the composition has an enzyme detergency benefit.

In some aspects, the present invention also relates to methods of degrading xanthan gum using detergent compositions of the present invention, wherein xanthan gum is on the surface of a hard surface or textile.

In some aspects, the present invention relates to the use of an enzyme stabilizing component for improving stability of an endoglucanase variant and/or a xanthan lyase variant and/or a mixture thereof in a detergent composition, in particular in a liquid laundry detergent composition, wherein the endoglucanase variant and/or the xanthan lyase variant is/are as defined as above, and wherein the enzyme stabilizing component is selected from the group consisting of polyols such as glycerol or 1,2-propylene glycol, alditols such as non-cyclic polyols having the formula HOCH₂[CH(OH)]ₙCH₂OH, mannitol, isomalt, lactit, sorbitol, xylitol, threitol, erythritol or arabitol, alkanol amines such as ethanol amine, or formates such as sodium formate, potassium formate, calcium formate or magnesium formate, preferably said enzyme stabilizing component is selected from glycerol and sodium formate, more preferably being sodium formate.

In a particular aspect, the present invention relates to the use of an enzyme stabilizing component for improving stability of an endoglucanase variant and/or a xanthan lyase variant and/or a mixture thereof in a detergent composition, wherein the endoglucanase variant has at least 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NO:1 and comprises a set of alterations selected from the group consisting of A559N + Y579W + T697G, K512P + A559N + Y579W + T697G, N18G + A71E + A186P + E408D + Y579W + I602T + A651P + A688G + V756Y, N18G + N189K + E408D + A559N + Y579W + A688G + T697G + V756Y + K921R + Y934G, S313D + E408D, R880K + N905D + K921R + Y934G, I302D + S313D + E408D + Y579W + I602T + A651P + T697G + R880K + K921R + Y934G, and N216Q + S313D + E408D + D476R + Y579W + I602T + F638N + A651P + T697G + W719R + R880K + T887K + K921R + Y934G, wherein said positions correspond to amino acid positions of SEQ ID NO:1; wherein the xanthan lyase variant has at least 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NO:2 and comprising a set of alterations selected from the group consisting of E229N + N672D + P752K + G753E + A769D + L775A + D801G + K875T + N892Y, E229S + N672D + G753E + S754E + A769D + L775A + D801G + K875T + N892Y, E229S + N672D + P752R + G753E + S754E + A769D + L775A + D801G + K875T + N892Y, A190Q + E229S + I234V + A624E + N672D + G753E + S754E + A769D + L775A + D801G + K875T, A190Q + E229S + T631N + N672D + I703L + P752K + G753E + A769D + L775A + D801G + K875T, A190Q + E229S + I234V + S582K + N672D + G753E + S754E + S757D + A769D + L775A + D801G + K875T + N892Y, E229S + N440K + S582K + A624E + S635E + N672D + G738L + G753E + S754E + S757D + A769D + L775A + D801G + K875T + N892Y, and S100D + E229S + K360G + D458S + S582K + N672D + G753E + S754E + S757D + A769D + L775A + D801G + A843P + K875T + N892Y + N1008D, wherein said positions correspond to amino acid positions of SEQ ID NO:2; and wherein the enzyme stabilizing component is selected from the group of glycerol and sodium formate.

### OVERVIEW OF SEQUENCE LISTING

SEQ ID NO:1 is the amino acid sequence of the parent mature endoglucanase from a strain of *Paenibacillus sp.*
SEQ ID NO:2 is the amino acid sequence of the parent mature xanthan lyase from a strain of *Paenibacillus sp.*

### DEFINITIONS

**Cleaning or detergent application:** the term "cleaning or detergent application" means applying the endoglucanase variant and/or the xanthan lyase variant of the invention in any composition for the purpose of cleaning or washing, by hand, machine or automated, on a hard surface or a textile.

**Color clarification:** During washing and wearing loose or broken fibers can accumulate on the surface of the fabrics. One consequence can be that the colors of the fabric appear less bright or less intense because of the surface contaminations. Removal of the loose or broken fibers from the textile will partly restore the original colors and looks of the textile. By the term "color clarification", as used herein, is meant the partial restoration of the initial colors of textile.

**Corresponding to:** The term "corresponding to" as used herein, refers to a way of determining the specific amino acid of a sequence wherein reference is made to a specific amino acid sequence. For example, for the purposes of the present invention, when references are made to specific amino acid positions, the skilled person would be able to align another amino acid sequence to said amino acid sequence that reference has been made to, in order to determine which specific amino acid may be of interest in said another amino acid sequence. Alignment of another amino acid sequence with e.g. the sequence as set forth in SEQ ID NO:1 or 2 has been described elsewhere herein. Alternative alignment methods may be used and are well-known for the skilled person.

**Degrading xanthan gum and xanthan gum degrading activity:** The terms "degrading xanthan gum" and "xanthan gum degrading activity" are used interchangeably and are defined as the depolymerization, degradation or breaking down of xanthan gum into smaller components. The degradation of xanthan gum can either be the removal of one or more side chain saccharides, the cutting of the backbone of xanthan gum into smaller components or the removal of one or more side chain saccharides and the cutting of the backbone of xanthan gum into smaller components. A preferred assay for measuring degradation of xanthan gum is the reducing sugar assay as described in examples 2 and 3 herein. Non-limiting examples of the xanthan gum degrading activity include endoglucanase EC 3.2.1.4 activity and/or xanthan lyase EC 4.2.2.12 activity.

**Detergent component:** the term "detergent component" is defined herein to mean the types of chemicals which can be used in detergent compositions. Examples of detergent components are surfactants, hydrotropes, builders, cobuilders, chelators or chelating agents, bleaching system or bleach components, polymers, fabric hueing agents, fabric conditioners, foam boosters, suds suppressors, dispersants, dye transfer inhibitors, fluorescent whitening agents, perfume, optical brighteners, bactericides, fungicides, soil suspending agents, soil release polymers, anti-redeposition agents, enzyme inhibitors or stabilizers, enzyme activators, antioxidants, and solubilizers. The detergent composition of the present invention may comprise of one or more of any type of detergent component.

**Detergent composition:** the term "detergent composition" (or "cleaning composition") refers to compositions that find use in the removal of undesired compounds from items to be cleaned, such as textiles, dishes, and hard surfaces. The detergent composition may be used to e.g. clean textiles, dishes and hard surfaces for both household cleaning and industrial cleaning. The terms encompass any materials/compounds selected for the particular type of cleaning composition desired and the form of the product (e.g. liquid, gel, powder, granulate, paste, or spray compositions) and includes, but is not limited to, detergent compositions (e.g. liquid and/or solid laundry detergents and fine fabric detergents; hard surface cleaning formulations, such as for glass, wood, ceramic and metal counter tops and windows; carpet cleaners; oven cleaners; fabric fresheners; fabric softeners; and textile and laundry pre-spotters, as well as dish wash detergents). In addition to containing an endoglucanase and/or xanthan lyase, as described herein, the detergent formulation may contain one or more additional enzymes (such as proteases, amylases, lipases, cutinases, cellulases, endoglucanases, xyloglucanases, pectinases, pectin lyases, xanthanases, peroxidases, haloperoxygenases, oxidoreductases, catalases and mannanases, or any mixture thereof), and/or further detergent components.

**Dish wash:** The term "dish wash" refers to all forms of washing dishes, e.g. by hand or automatic dish wash. Washing dishes includes, but is not limited to, the cleaning of all forms of crockery such as plates, cups, glasses, bowls, all forms of cutlery such as spoons, knives, forks and serving utensils as well as ceramics, plastics, metals, china, glass and acrylics.

**Dish washing composition:** The term "dish washing composition" refers to all forms of compositions for cleaning hard surfaces. The present invention is not restricted to any particular type of dish wash composition or any particular detergent.

**Endoglucanase:** The term "endoglucanase" or "EG" means an endo-1,4-(1,3;1,4)-β-D-glucan 4-glucanohydrolase (EC 3.2.1.4) that catalyzes endohydrolysis of 1,4-β-D-glycosidic linkages in cellulose, cellulose derivatives (such as carboxymethyl cellulose and hydroxyethyl cellulose), lichenin, β-1,4 bonds in mixed β-1,3 glucans such as cereal β-D-glucans, xyloglucans, xanthans and other plant material containing cellulosic components. A preferred assay for measuring endoglucanase activity is the reducing sugar assay as described in example 2 herein. Non-limiting examples of endoglucanases include the mature parent endoglucanase having SEQ ID NO:1.

**Endoglucanase variant having activity on xanthan gum pre-treated with xanthan lyase:** The term "endoglucanase variant having activity on xanthan gum pre-treated with xanthan lyase" or an "endoglucanase having activity on xanthan gum pre-treated with xanthan lyase and belonging to the GH9 class of glycosyl hydrolases" is defined as a polypeptide comprising a domain belonging to the GH9 class of glycosyl hydrolases, and having activity (e.g. enzymatic activity, xanthan degrading activity, endoglucanase EC 3.2.1.4 activity) on xanthan gum pre-treated with xanthan lyase. A preferred assay for measuring activity on xanthan gum pre-treated with xanthan lyase is disclosed in example 2 herein.

**Enzyme detergency benefit:** The term "enzyme detergency benefit" is defined herein as the advantageous effect an enzyme may add to a detergent compared to the same detergent without the enzyme. Important detergency benefits which can be provided by enzymes are stain removal with no or very little visible soils after washing and or cleaning, prevention or reduction of redeposition of soils released in the washing process (an effect that also is termed anti-redeposition), restoring fully or partly the whiteness of textiles, which originally were white but after repeated use and wash have obtained a greyish or yellowish appearance (an effect that also is termed whitening). Textile care benefits, which are not directly related to catalytic stain removal or prevention of redeposition of soils are also important for enzyme detergency benefits. Examples of such textile care benefits are prevention or reduction of dye transfer from one fabric to another fabric or another part of the same fabric (an effect that is also termed dye transfer inhibition or anti-backstaining), removal of protruding or broken fibers from a fabric surface to decrease pilling tendencies or remove already existing pills or fuzz (an effect that also is termed anti-pilling), improvement of the fabric softness, color clarification of the fabric and removal of particulate soils which are trapped in the fibers of the fabric or garment. Enzymatic bleaching is a further enzyme detergency benefit where the catalytic activity generally is used to catalyze the formation of bleaching component such as hydrogen peroxide or other peroxides.

**Hard surface cleaning:** The term "hard surface cleaning" is defined herein as cleaning of hard surfaces wherein hard surfaces may include floors, tables, walls, roofs etc. as well as surfaces of hard objects such as cars (car wash) and dishes (dish wash).

**Improved property:** The term "improved property" means a characteristic associated with a variant that is improved compared to the parent. Such improved properties include, but are not limited to, catalytic efficiency, catalytic rate, chemical stability, oxidation stability, pH activity, pH stability, specific activity, stability under storage conditions, chelator stability, substrate binding, substrate cleavage, substrate specificity, substrate stability, surface properties, thermal activity, thermostability, wash performance, or stain removal.

**Laundering:** The term "laundering" relates to both household laundering and industrial laundering and means the process of treating textiles with a solution containing a cleaning or detergent composition of the present invention. The laundering process can be carried out using e.g. a household or an industrial washing machine or can be carried out by hand.

**Mature polypeptide:** The term "mature polypeptide" means a polypeptide in its final form following translation and any post-translational modifications, such as N-terminal processing, C-terminal truncation, glycosylation, phosphorylation, etc. In one aspect, the mature polypeptide is amino acids 1 to 1055 of SEQ ID NO:1 or amino acids 1 to 1037 of SEQ ID NO:2.

**Parent:** The term "parent" or "parent endoglucanase" or "parent xanthan lyase" means any polypeptide with endoglucanase resp. xanthan lyase activity to which an alteration is made to produce the enzyme variants of the present invention. In one aspect, the parent is an endoglucanase (resp. xanthan lyase) having the identical amino acid sequence of the variant, but not having the alterations at one or more of the specified positions. It will be understood that the expression "having identical amino acid sequence" relates to 100% sequence identity. Non-limiting examples of parent endoglucanases include the mature parent endoglucanase having SEQ ID NO:1. Non-limiting examples of parent xanthan lyases include the mature parent xanthan lyase having SEQ ID NO:2.

**Sequence identity:** The relatedness between two amino acid sequences is described by the parameter "sequence identity". For purposes of the present invention, the sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the - nobrief option) is used as the percent identity and is calculated as follows: (Identical Residues x 100)/(Length of Alignment - Total Number of Gaps in Alignment).

**Stability:** The term "stability" means resistance or the degree of resistance to change, unfolding, disintegration, denaturation or activity loss. Non-limiting examples of stability include conformational stability, storage stability and stability during use, e.g. during a wash process and reflects the stability of a polypeptide (e.g. an endoglucanase variant or xanthan lyase variant according to the invention) as a function of time, e.g. how much activity is retained when said polypeptide (e.g. said endoglucanase variant or xanthan lyase variant) is kept in solution, in particular in a detergent solution. The stability is influenced by many factors, e.g. presence of chelators, pH, temperature, detergent composition, e.g. amount of builders, surfactants, chelators etc. The endoglucanase or xanthan lyase stability may be measured as described in examples 2 and 3 herein.

**Textile:** The term "textile" means any textile material including yarns, yarn intermediates, fibers, non-woven materials, natural materials, synthetic materials, and any other textile material, fabrics made of these materials and products made from fabrics (e.g. garments and other articles). The textile or fabric may be in the form of knits, wovens, denims, non-wovens, felts, yarns, and toweling. The textile may be cellulose based such as natural cellulosic, including cotton, flax/linen, jute, ramie, sisal or coir or manmade cellulosic (e.g. originating from wood pulp) including viscose/rayon, ramie, cellulose acetate fibers (tricell), lyocell or blends thereof. The textile or fabric may also be non-cellulose based such as natural polyamides including wool, camel, cashmere, mohair, rabbit and silk or synthetic polymer such as nylon, aramid, polyester, acrylic, polypropylene and spandex/elastane, or blends thereof as well as blend of cellulose based and non-cellulose based fibers. Examples of blends are blends of cotton and/or rayon/viscose with one or more companion material such as wool, synthetic fibers (e.g. polyamide fibers, acrylic fibers, polyester fibers, polyvinyl alcohol fibers, polyvinyl chloride fibers, polyurethane fibers, polyurea fibers, aramid fibers), and cellulose-containing fibers (e.g. rayon/viscose, ramie, flax/linen, jute, cellulose acetate fibers, lyocell). Fabric may be conventional washable laundry, e.g. stained household laundry. When the term fabric or garment is used it is intended to include the broader term textiles as well.

**Variant:** The term "variant" means a polypeptide (e.g. an endoglucanase polypeptide or a xanthan lyase polypeptide) comprising an alteration, i.e. a substitution, insertion, and/or deletion, at one or more (e.g. several) positions. A substitution means replacement of the amino acid occupying a position with a different amino acid; a deletion means removal of the amino acid occupying a position; and an insertion means adding one or more (e.g. several) amino acids, e.g. 1-5 amino acids, adjacent to and immediately following the amino acid occupying a position. Non-limiting examples of endoglucanase variants and/or xanthan lyase variants, as described herein, include endoglucanase variants and/or xanthan lyase variants having an activity on xanthan gum (for xanthan lyase) and xanthan gum pre-treated with xanthan lyase (for endoglucanase). Non-limiting examples of variants described herein further include variants having at least 20%, e.g. at least 40%, 50%, 60%, 70%, 80%, 90%, 95%, or 100% endoglucanase activity of the mature parent having SEQ ID NO:1 or SEQ ID NO:2. A preferred assay for measuring activity on xanthan gum (optionally pre-treated with xanthan lyase) is disclosed in examples 2 and 3 herein.

**Conventions for designation of variants:** For purposes of the present invention, the mature polypeptide disclosed in SEQ ID NO:1 is used to determine the corresponding amino acid residue in another endoglucanase and the mature polypeptide disclosed in SEQ ID NO:2 is used to determine the corresponding amino acid residue in another xanthan lyase. The amino acid sequence of another endoglucanase/xanthan lyase is aligned with the mature polypeptide disclosed in SEQ ID NO:1 or, and based on the alignment, the amino acid position number corresponding to any amino acid residue in the mature polypeptide disclosed in SEQ ID NO:1 or 2 is determined using the Needleman-Wunsch algorithm (as described above). Identification of the corresponding amino acid residue in another endoglucanase or xanthan lyase can be determined by an alignment of multiple polypeptide sequences using several computer programs, which are known to the skilled person. In describing the variants of the present invention, the nomenclature described below is adapted for ease of reference. The accepted IUPAC single letter or three letter amino acid abbreviation is employed.

Substitutions: For an amino acid substitution, the following nomenclature is used: Original amino acid, position, substituted amino acid. Accordingly, the substitution of threonine at position 226 with alanine is designated as "Thr226Ala" or "T226A". Multiple mutations are separated by addition marks ("+"), e.g. "Gly205Arg + Ser411Phe" or "G205R + S411F", representing substitutions at positions 205 and 411 of glycine (G) with arginine (R) and serine (S) with phenylalanine (F), respectively.

Deletions: For an amino acid deletion, the following nomenclature is used: Original amino acid, position, *. Accordingly, the deletion of glycine at position 195 is designated as "Gly195*" or "G195*". Multiple deletions are separated by addition marks ("+"), e.g. "Gly195* + Ser411*" or "G195* + S411*".

Insertions: For an amino acid insertion, the following nomenclature is used: Original amino acid, position, original amino acid, inserted amino acid. Accordingly, the insertion of lysine after glycine at position 195 is designated "Gly195GlyLys" or "G195GK". An insertion of multiple amino acids is designated [Original amino acid, position, original amino acid, inserted amino acid #1, inserted amino acid #2; etc.]. For example, the insertion of lysine and alanine after glycine at position 195 is indicated as "Gly195GlyLysAla" or "G195GKA". An indication of an insertion at a particular position is understood as being an insertion after the original amino acid residue. For example, an "insertion at position 195" is understood to be an insertion after the original residue in position 195.

In such cases the inserted amino acid residue(s) are numbered by the addition of lower-case letters to the position number of the amino acid residue preceding the inserted amino acid residue(s). In the above example, the sequence would thus be:

| Parent: | Variant: |
|---|---|
| 195 | 195 195a 195b |
| G | G - K - A |

Multiple alterations: Variants comprising multiple alterations are separated by addition marks ("+"), e.g. "Arg170Tyr + Gly195Glu" or "R170Y + G195E" representing a substitution of arginine and glycine at positions 170 and 195 with tyrosine and glutamic acid, respectively.

Different alterations: Where different alterations can be introduced at a position, the different alterations are separated by a comma, e.g. "Arg170Tyr,Glu" represents a substitution of arginine at position 170 with tyrosine or glutamic acid. Thus, "Tyr167Gly,Ala + Arg170Gly,Ala" designates the following variants: "Tyr167Gly + Arg170Gly", "Tyr167Gly + Arg170Ala", "Tyr167Ala + Arg170Gly", and "Tyr167Ala + Arg170Ala". Alternatively, different alterations or may be indicated using brackets, e.g. Arg170[Tyr, Gly] or in one-letter code R170[Y,G].

**Wash performance:** The term "wash performance" is used as an enzyme's ability to remove stains present on the object to be cleaned during e.g. wash or hard surface cleaning. The improvement in the wash performance may be quantified by calculating the so-called intensity value (Int) in 'Automatic Mechanical Stress Assay (AMSA) for laundry' or the remission value (Rem).

**Xanthan lyase variant having activity on xanthan gum:** The term "xanthan lyase variant having activity on xanthan gum" is defined as a polypeptide that cleaves the β-D-mannosyl-β-D-1,4-glucuronosyl bond of xanthan (e.g. xanthan lyase EC 4.2.2.12 activity). A preferred assay for measuring activity on xanthan gum is disclosed in example 3 herein. Examples of the xanthan lyase variants having activity on xanthan gum, are xanthan lyase polypeptides as such. Thus, polypeptides that that cleaves the β-D-mannosyl-β-D-1,4-glucuronosyl bond of xanthan.

### DETAILED DESCRIPTION OF THE INVENTION

The known xanthan endoglucanase having SEQ ID NO:1 and the xanthan lyase having SEQ ID NO:2 are both large enzymes (>1000 amino acid residues). It is therefore extremely laborious and expensive to target its properties for improvement of, e.g. stability in a detergent composition, e.g. in the presence of a surfactant or a chelator.

The inventors of the present invention have surprisingly found that the stability of specific endoglucanase variants and/or xanthan lyase variants in detergent compositions can be improved by addition of certain enzyme stabilizing components, like e.g. being selected from the group consisting of polyols such as glycerol or 1,2-propylene glycol, alditols such as non-cyclic polyols having the formula HOCH₂[CH(OH)]ₙCH₂OH, mannitol, isomalt, lactit, sorbitol, xylitol, threitol, erythritol or arabitol, alkanol amines such as ethanol amine, or formates such as sodium formate, potassium formate, calcium formate or magnesium formate, preferably said enzyme stabilizing component is selected from glycerol and formates, in particular by addition of sodium formate.

In one embodiment, the detergent composition of the present invention comprises an endoglucanase variant comprising an alteration, preferably a substitution, at one or more positions in one or more regions selected from the group consisting of region 1 corresponding to amino acids 95 to 105 of SEQ ID NO:1, region 2 corresponding to amino acids 115 to 138 of SEQ ID NO:1, region 3 corresponding to amino acids 210 to 251 of SEQ ID NO:1, region 4 corresponding to amino acids 267 to 301 of SEQ ID NO:1, region 5 corresponding to amino acids 339 to 361 of SEQ ID NO:1, region 6 corresponding to amino acids 547 to 595 of SEQ ID NO:1, region 7 corresponding to amino acids 612 to 660 of SEQ ID NO:1, region 8 corresponding to amino acids 806 to 828 of SEQ ID NO:1, region 9 corresponding to amino acids 839 to 1042 of SEQ ID NO:1, region 10 corresponding to amino acids 1 to 94 of SEQ ID NO:1, region 11 corresponding to amino acids 106 to 114 of SEQ ID NO:1, region 12 corresponding to amino acids 139 to 209 of SEQ ID NO:1, region 13 corresponding to amino acids 252 to 266 of SEQ ID NO:1, region 14 corresponding to amino acids 302 to 338 of SEQ ID NO:1, region 15 corresponding to amino acids 362 to 546 of SEQ ID NO:1, region 16 corresponding to amino acids 596 to 611 of SEQ ID NO:1, region 17 corresponding to amino acids 661 to 805 of SEQ ID NO:1, region 18 corresponding to amino acids 829 to 838 of SEQ ID NO:1, and region 19 corresponding to amino acids 1043 to 1055 of SEQ ID NO:1.

In one embodiment, the detergent composition of the present invention comprises a xanthan lyase variant comprising an alteration, preferably a substitution, at one or more positions in one or more regions selected from the group consisting of region 1 corresponding to amino acids 154 to 176 of SEQ ID NO:2, region 2 corresponding to amino acids 614 to 658 of SEQ ID NO:2, region 3 corresponding to amino acids 731 to 803 of SEQ ID NO:2, region 4 corresponding to amino acids 807 to 846 of SEQ ID NO:2, region 5 corresponding to amino acids 872 to 885 of SEQ ID NO:2, region 6 corresponding to amino acids 903 to 1004 of SEQ ID NO:2, region 7 corresponding to amino acids 1 to 153 of SEQ ID NO:2, region 8 corresponding to amino acids 177 to 613 of SEQ ID NO:2, region 9 corresponding to amino acids 659 to 730 of SEQ ID NO:2, region 10 corresponding to amino acids 804 to 806 of SEQ ID NO:2, region 11 corresponding to amino acids 847 to 871 of SEQ ID NO:2, region 12 corresponding to amino acids 886 to 902 of SEQ ID NO:2, and region 13 corresponding to amino acids 1005 to 1037 of SEQ ID NO:2.

In one embodiment, the present invention provides detergent compositions comprising variants of an endoglucanase and of a xanthan lyase, as described herein, both of which have significantly improved stability in said detergent composition due to addition of an enzyme stabilizing component.

In one embodiment, the enzyme stabilizing component is selected from the group consisting of polyols such as glycerol or 1,2-propylene glycol, alditols such as non-cyclic polyols having the formula HOCH₂[CH(OH)]ₙCH₂OH, mannitol, isomalt, lactit, sorbitol, xylitol, threitol, erythritol or arabitol, alkanol amines such as ethanol amine, or formates such as sodium formate, potassium formate, calcium formate or magnesium formate, preferably said enzyme stabilizing component is selected from glycerol and formates.

In a preferred embodiment, the enzyme stabilizing component is glycerol and/or sodium formate.

### Endoglucanase variants

In one embodiment, the detergent composition of the present invention comprises an endoglucanase variant comprising an alteration, preferably a substitution, at one or more positions in one or more regions selected from the group consisting of region 1 corresponding to amino acids 95 to 105 of SEQ ID NO:1, region 2 corresponding to amino acids 115 to 138 of SEQ ID NO:1, region 3 corresponding to amino acids 210 to 251 of SEQ ID NO:1, region 4 corresponding to amino acids 267 to 301 of SEQ ID NO:1, region 5 corresponding to amino acids 339 to 361 of SEQ ID NO:1, region 6 corresponding to amino acids 547 to 595 of SEQ ID NO:1, region 7 corresponding to amino acids 612 to 660 of SEQ ID NO:1, region 8 corresponding to amino acids 806 to 828 of SEQ ID NO:1, region 9 corresponding to amino acids 839 to 1042 of SEQ ID NO:1, region 10 corresponding to amino acids 1 to 94 of SEQ ID NO:1, region 11 corresponding to amino acids 106 to 114 of SEQ ID NO:1, region 12 corresponding to amino acids 139 to 209 of SEQ ID NO:1, region 13 corresponding to amino acids 252 to 266 of SEQ ID NO:1, region 14 corresponding to amino acids 302 to 338 of SEQ ID NO:1, region 15 corresponding to amino acids 362 to 546 of SEQ ID NO:1, region 16 corresponding to amino acids 596 to 611 of SEQ ID NO:1, region 17 corresponding to amino acids 661 to 805 of SEQ ID NO:1, region 18 corresponding to amino acids 829 to 838 of SEQ ID NO:1, and region 19 corresponding to amino acids 1043 to 1055 of SEQ ID NO:1.

In one embodiment, such variant has at least 60% and less than 100% sequence identity to SEQ ID NO:1, preferably said endoglucanase variant has activity on xanthan gum pre-treated with xanthan lyase, further preferably said activity is a xanthan gum degrading activity.

In one embodiment, the detergent composition of the present invention comprises an endoglucanase variant, comprising an alteration, preferably a substitution, at one or more positions in one or more regions or multiple alterations (such as 2, 3, 4, 5, 6, 7, 8, 9 or 10) in one region or multiple alterations (e.g. 2, 3, 4, 5, 6, 7, 8, 9 or 10) in multiple regions (e.g. 2, 3, 4, 5, 6, 7, 8, or 9) selected from the group consisting of region 1 corresponding to amino acids 95 to 105 of SEQ ID NO:1, region 2 corresponding to amino acids 115 to 138 of SEQ ID NO:1, region 3 corresponding to amino acids 210 to 251 of SEQ ID NO:1, region 4 corresponding to amino acids 267 to 301 of SEQ ID NO:1, region 5 corresponding to amino acids 339 to 361 of SEQ ID NO:1, region 6 corresponding to amino acids 547 to 595 of SEQ ID NO:1, region 7 corresponding to amino acids 612 to 660 of SEQ ID NO:1, region 8 corresponding to amino acids 806 to 828 of SEQ ID NO:1, region 9 corresponding to amino acids 839 to 1042 of SEQ ID NO:1, region 10 corresponding to amino acids 1 to 94 of SEQ ID NO:1, region 11 corresponding to amino acids 106 to 114 of SEQ ID NO:1, region 12 corresponding to amino acids 139 to 209 of SEQ ID NO:1, region 13 corresponding to amino acids 252 to 266 of SEQ ID NO:1, region 14 corresponding to amino acids 302 to 338 of SEQ ID NO:1, region 15 corresponding to amino acids 362 to 546 of SEQ ID NO:1, region 16 corresponding to amino acids 596 to 611 of SEQ ID NO:1, region 17 corresponding to amino acids 661 to 805 of SEQ ID NO:1, region 18 corresponding to amino acids 829 to 838 of SEQ ID NO:1, and region 19 corresponding to amino acids 1043 to 1055 of SEQ ID NO:1, wherein said variant has at least 60% and less than 100% sequence identity to SEQ ID NO:1, preferably said endoglucanase variant has activity on xanthan gum pre-treated with xanthan lyase, further preferably said activity is a xanthan gum degrading activity.

In one embodiment, the detergent composition of the present invention comprises an endoglucanase variant comprising an alteration, preferably a substitution, at one or more positions in: (i) regions 6 and 17; (ii) regions 6, 15 and 17; (iii) regions 10, 12 and 15; (iv) regions 6, 7, 16, and 17; (v) region 6, 9, 10, 12, 15, and 17; (vi) region 14 and 15; (vii) region 9; (viii) 6, 7, 9, 14, 15, 16, and 17; or (ix) 3, 6, 7, 9, 14, 15, 16, and 17; wherein said variant preferably has no alternation in the other regions besides those mentioned.

In one embodiment, the detergent composition of the present invention comprises an endoglucanase variant having at least 60%, e.g. at least 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% and less than 100% sequence identity to the amino acid sequence as set forth in SEQ ID NO:1.

In one embodiment, the detergent composition of the present invention comprises an endoglucanase variant comprising an alteration, preferably a substitution, at one or more positions selected from the group consisting of 4, 17, 18, 20, 51, 53, 55, 56, 60, 63, 71, 79, 87, 92, 99, 120, 125, 126, 130, 137, 182, 186, 189, 192, 213, 216, 221, 226, 228, 230, 231, 232, 233, 235, 240, 243, 247, 249, 278, 279, 281, 283, 285, 289, 292, 294, 298, 302, 311, 313, 333, 346, 353, 358, 386, 387, 388, 390, 403, 408, 410, 416, 441, 448, 451, 471, 472, 476, 489, 507, 512, 515, 538, 555, 556, 557, 558, 559, 560, 561, 562, 563, 564, 567, 568, 570, 575, 578, 579, 580, 581, 583, 589, 590, 591, 592, 593, 595, 598, 599, 602, 603, 605, 607, 609, 616, 627, 630, 631, 635, 636, 638, 639, 640, 641, 642, 643, 644, 651, 676, 683, 688, 690, 694, 698, 699, 706, 711, 713, 719, 720, 744, 749, 754, 756, 760, 781, 786, 797, 810, 811, 812, 815, 823, 824, 825, 827, 828, 833, 834, 835, 837, 843, 848, 868, 869, 870, 871, 872, 873, 874, 880, 881, 883, 884, 885, 887, 888, 890, 892, 894, 898, 905, 906, 912, 920, 921, 924, 926, 927, 928, 932, 933, 934, 935, 937, 938, 939, 940, 941, 942, 943, 946, 948, 950, 952, 953, 954, 956, 957, 960, 966, 971, 972, 980, 989, 991, 994, 995, 998, 999, 1006, 1009, 1010, 1011, 1029, 1030, 1031, 1032, 1035, 1037, 1038, 1040, 1041, 1042, 1044, 1045 and 1048, wherein said positions correspond to amino acid positions of SEQ ID NO:1.

In one embodiment, the detergent composition of the present invention comprises an endoglucanase variant comprising an alteration, preferably a substitution, at 2, 3, 4, 5, 6, 7, 8, 9 or more positions selected from the group consisting of 4, 17, 18, 20, 51, 53, 55, 56, 60, 63, 71, 79, 87, 92, 99, 120, 125, 126, 130, 137, 182, 186, 189, 192,213,216,221,226,228,230,231,232,233,235,240, 243, 247, 249, 278, 279, 281, 283, 285, 289, 292, 294, 298, 302, 311, 313, 333, 346, 353, 358, 386, 387, 388, 390, 403, 408, 410, 416, 441, 448, 451, 471, 472, 476, 489, 507, 512, 515, 538, 555, 556, 557, 558, 559, 560, 561, 562, 563, 564, 567, 568, 570, 575, 578, 579, 580, 581, 583, 589, 590, 591, 592, 593, 595, 598, 599, 602, 603, 605, 607, 609, 616, 627, 630, 631, 635, 636, 638, 639, 640, 641, 642, 643, 644, 651, 676, 683, 688, 690, 694, 698, 699, 706, 711, 713, 719, 720, 744, 749, 754, 756, 760, 781, 786, 797, 810, 811, 812, 815, 823, 824, 825, 827, 828, 833, 834, 835, 837, 843, 848, 868, 869, 870, 871, 872, 873, 874, 880, 881, 883, 884, 885, 887, 888, 890, 892, 894, 898, 905, 906, 912, 920, 921, 924, 926, 927, 928, 932, 933, 934, 935, 937, 938, 939, 940, 941, 942, 943, 946, 948, 950, 952, 953, 954, 956, 957, 960, 966, 971, 972, 980, 989, 991, 994, 995, 998, 999, 1006, 1009, 1010, 1011, 1029, 1030, 1031, 1032, 1035, 1037, 1038, 1040, 1041, 1042, 1044, 1045 and 1048, wherein said positions correspond to amino acid positions of SEQ ID NO:1.

In a preferred embodiment, the detergent composition of the present invention comprises an endoglucanase variant comprising an alteration, preferably a substitution, at one or more positions selected from the group consisting of 17, 20, 216, 283, 302, 311, 313, 408, 476, 579, 602, 627, 636, 638, 651, 688, 697, 719, 756, 880, 881, 883, 887, 906, 921, 934, 937, and 956, wherein said positions correspond to amino acid positions of SEQ ID NO:1.

In a more preferred embodiment, the detergent composition of the present invention comprises an endoglucanase variant comprising an alteration, preferably a substitution, at each position (or at least four positions) corresponding to positions 17, 20, 216, 283, 302, 311, 313, 408, 476, 579, 602, 627, 636, 638, 651, 688, 697, 719, 756, 880, 881, 883, 887, 906, 921, 934, 937, and 956, wherein said positions correspond to amino acid positions of SEQ ID NO:1.

In one embodiment, the detergent composition of the present invention comprises an endoglucanase variant comprising one or more amino acid substitutions selected from the group consisting of V4T, S17A, N18G, F20P, F20N, F20G, F20Y, K51Q, K51H, E53Y, E53P, E53G, Y55M, Y55D, V56M, Y60F, S63F, A71E, T87R, T92S, K99R, A120P, I125V, A126R, S128X, N129D, K130R, F137L, H182Y, A186P, N189K, K192N, K213R, N216D, N216Q, N216R, A221R, L226K, K228E, K228I, G230F, G230L, G230A, G230H, G230N, G230W, G230T, F231Y, F231N, V232R, V232G, L233H, H235D, N240Q, G243K, G243R, D247N, A249N, A278S, G279E, K281F, K281V, K281Y, K281H, K281Q, K281N, K281W, K281T, K281R, A283D, N285G, N285L, N285M, N285S, N285P, N285T, N285Y, N285H, N285K, N285D, N285W, N285R, Q289E, T292A, T292F, T292L, T292I, T292V, T292S, T292P, T292Y, T292Q, T292N, T292K, T292D, T292G, A294V, F297L, Q298E, I302D, I302H, I302V, I302M, H311N, S313D, W333L, A346H, A346D, T353D, A386P, I387T, K388R, K390Q, I403Y, E408D, E408N, E408S, E408P, E408A, E408G, P410G, Q416S, Q416D, N441G, A448E, A448W, A448S, K451Q, K451S, G471S, S472Y, D476R, Q489P, K507R, K512P, S515V, S538C, L555Q, G556S, G557R, N558P, N558F, N558K, N558D, N558M, N558Q, N558I, N558Y, N558H, N558E, A559N, A559F, A559M, A559P, A559Y, A559H, A559Q, A559D, A559R, A559G, A559I, A559S, A559K, S560F, S560P, S560K, S560G, S560D, T561E, T561Q, T561S, T561D, T561P, G562W, G562P, G563E, A564I, A564Y, A564H, A564Q, A564K, A564E, A564D, E565M, V567F, V567P, K568R, L569F, L569Y, L569D, L569E, P570F, P570L, P570I, P570M, P570V, P570S, P570T, P570A, P570Y, P570H, P570Q, P570N, P570K, P570E, P570W, P570R, P570G, I575V, I575M, I575A, I575D, I575E, I576F, I576M, I576P, D578R, Y579F, Y579W, V580L, T581M, D583M, Q589G, P590S, P590T, P590E, E591L, G592D, S593P, S593H, S593Q, S593N, S593K, S593D, S593E, S593R, S595L, S598Q, A599S, I602T, I602D, V603P, S605T, S607C, G609E, S616G, S616D, K627L, K627M, K627V, K627S, K627T, K627Q, K627R, I630F, I630V, I630Y, K631R, K631A, T633V, D635L, D635M, D635T, D635A, D635P, D635N, D635K, D635E, D635G, D635W, S636L, S636M, S636A, S636H, S636Q, S636N, S636K, S636R, F638N, F638Y, T639D, F638I, F638V, F638T, F638L, F638H, F638M, T639V, T639S, T639L, T639I, T639M, T639A, T639E, T639W, T639G, T639Y, T639P, T640S, Y641E, S642T, S642N, N643D, N643H, N643T, T644F, A651P, A651S, D676H, Q683E, A688G, Y690F, T694A, T697G, R698W, T699A, T706Q, T711S, T711V, T711Y, K713R, W719R, K720H, K744H, K744Q, A749T, K754R, V756Y, V756H, S760G, T781M, N786K, T797S, S810Q, S810R, A811S, V812F, V812I, V812M, V812W, V812R, N815V, N815Y, N815E, N815W, N815R, S823Q, A824T, A824D, T825G, T825N, T825W, T825A, T825D, V827I, V827M, V827S, N828D, N833D, Q834E, S835A, S835D, V837I, T843V, N848D, A868E, A869V, D870F, D870L, D870I, D870M, D870V, D870S, D870T, D870Y, D870H, D870Q, D870N, D870K, D870E, D870W, D870R, D870G, P871F, P871L, P871I, P871M, P871V, P871S, P871T, P871A, P871Y, P871H, P871Q, T872S, T872F, T872A, T872Y, T872H, T872Q, T872N, T872K, T872D, T872E, T872W, T872R, T872G, D873K, D873E, T874V, T874S, T874P, T874A, T874H, T874Q, T874N, T874K, R880K, V881Q, V881T, T883R, T883V, T883C, T883K, Y884H, A885F, A885Q, A885N, T887L, T887I, T887S, T887H, T887R, T887K, L888M, V890R, T892P, T892V, K894E, R898Q, N905D, F906A, Q912V, N920P, N920D, K921R, K921E, K921D, A924D, V926F, V926P, K927R, S928D, T932A, N933V, N933S, Y934G, Y934M, Y934S, Y934A, Y934Q, Y934N, Y934E, Y934W, Y934R, T935W, A937F, A937V, A937S, A937T, A937Q, A937D, A937E, V938I, K939I, K939V, D940E, N941S, N941H, N941D, A942P, A942E, D943Y, D943H, G946R, K948R, K948E, R950V, R950H, R950N, F952S, F952W, N953Y, G954L, Q956Y, Q956S, A957L, A957P, Y960F, A964N, A964C, N966P, N966C, G971A, T972K, Q974K, Q974C, M980I, Q989I, Q991L, Q991I, Q991M, Q991V, Q991T, Q991K, Q991C, G994D, G994N, S995I, S995V, S995Q, S995R, S995C, G998V, G998A, T999R, S1006T, S1006A, S1006K, S1006R, K1009E, Y1010W, L1011M, L1011S, L1011A, L1011Q, L1011N, L1011D, L1011E, R1029N, F1030M, K1031I, K1031S, K1031T, K1031H, V1032G, K1035A, A1037E, A1037W, S1038L, S1038I, S1038G, L1040N, L1040E, G1041F, G1041R, Y1042N, L1044F, L1044S, L1044N, L1044W, P1045Q, P1045W, and F1048W, wherein said positions correspond to amino acid positions of SEQ ID NO:1.

In a preferred embodiment, the detergent composition of the present invention comprises an endoglucanase variant comprising one or more amino acid substitutions selected from the group consisting of S17A, F20P, N216D, N216Q, A283D, I302D, H311N, S313D, E408D, D476R, Y579W, I602T, K627R, S636N, S636K F638I, F638N, A651P, A688G, T697G, W719R, V756Y, R880K, V881Q, T883R, T887K, F906A, K921R, Y934G, A937E, and Q956Y, wherein said positions correspond to amino acid positions of SEQ ID NO:1.

In a further preferred embodiment, the detergent composition of the present invention comprises an endoglucanase variant comprising a set of alterations selected from the group consisting of A559K + N558K + S560F + T561P + G562W, A559N + P570Q, A559N + P570T, A559N + Y579F, A559N + Y579F + K627R, A559N + Y579W, A559N + Y579W + K99R, A559N + Y579W + S128X + K627R, A559N + Y579W + S128X + S636N, A559N + Y579W + K281R, A559N + Y579W + Q416D + S636N, A559N + Y579W + S560P, A559N + Y579W + A5641, A559N + Y579W + P570N, A559N + Y579W + P570K, A559N + Y579W + P570R, A559N + Y579W + P570A, A559N + Y579W + P570T, A559N + Y579W + P570S, A559N + Y579W + P570Q, A559N + Y579W + P570H, A559N + Y579W + S616D, A559N + Y579W + S616D + K627R, A559N + Y579W + S616D + S636K, A559N + Y579W + S616D + A651P, A559N + Y579W + S616D + K921R, A559N + Y579W + S616D + K921R + Y934G, A559N + Y579W + K627R, A559N + Y579W + K627R + S636N + A651P, A559N + Y579W + K627R + S636N + K921R, A559N + Y579W + K627R + K921R, A559N + Y579W + K627M, A559N + Y579W + K627M + A651P, A559N + Y579W + S636N, A559N + Y579W + S636N + A651P, A559N + Y579W + S636N + K921R, A559N + Y579W + S636K + A651P, A559N + Y579W + S636K + K921R, A559N + Y579W + S636L, A559N + Y579W + F638I, A559N + Y579W + A651P, A559N + Y579W + A651P + K921R + Y934G, A559N + Y579W + A651P + Y934G, A559N + Y579W + D870M, A559N + Y579W + K921R, A559N + Y579W + K921R + A651P, A559N + Y579W + K921R + Y934G, A559N + Y579W + Y934G, A559N + Y579W + K948E, A559N + Y579W + K1009E, A559N + K627R, Y579F + A564E, Y579W + K99R, Y579W + G562P, Y579W + A564D 579W + P570K, Y579W + P570Q, Y579W + K627R, Y579W + S636N, Y579W + F638I, Y579W + A651P, Y579W + K921R, Y579W + Y934G, Y579W + K948E, Y579W + K1009E, K627R + K51Q, K627R + K451S, K627R + P570Q, K627R + P570T, K627R + S636N, K627R + F638I, K627R + A885F, K627R + Y934G, S636N + P570T, S636N + K921R, S636N + Y934G, F638I + P570K, F638I + P570Q, F638I + P570T, F638I + A651P, F638I + Y934G, F638I + K921R, A651P + P570Q, A651P + P570T, A885F + P570T, A885F + Y934G, K921R + P570Q, K921R + P570T, Y934G + P570T, wherein said positions correspond to amino acid positions of SEQ ID NO:1.

In an even more preferred embodiment, the detergent composition of the present invention comprises an endoglucanase variant comprising a set of alterations selected from the group consisting of 559N + Y579W + T697G, K512P + A559N + Y579W + T697G, N18G + A71E + A186P + E408D + Y579W + I602T + A651P + A688G + V756Y, N18G + N189K + E408D + A559N + Y579W + A688G + T697G + V756Y + K921R + Y934G, S313D + E408D, R880K + N905D + K921R + Y934G, 1302D + S313D + E408D + Y579W + I602T + A651P + T697G + R880K + K921R + Y934G, and N216Q + S313D + E408D + D476R + Y579W + I602T + F638N + A651P + T697G + W719R + R880K + T887K + K921R + Y934G, wherein said positions correspond to amino acid positions of SEQ ID NO:1.

In a particular aspect, the endoglucanase variant has besides the aforementioned alterations no further alterations relative to the parent enzyme of SEQ ID NO:1, i.e. the remaining sequence is identical to SEQ ID NO:1.

### Xanthan lyase variants

In one embodiment, the detergent composition of the present invention comprises a xanthan lyase variant comprising an alteration, preferably a substitution, at one or more positions in one or more regions selected from the group consisting of region 1 corresponding to amino acids 154 to 176 of SEQ ID NO:2, region 2 corresponding to amino acids 614 to 658 of SEQ ID NO:2, region 3 corresponding to amino acids 731 to 803 of SEQ ID NO:2, region 4 corresponding to amino acids 807 to 846 of SEQ ID NO:2, region 5 corresponding to amino acids 872 to 885 of SEQ ID NO:2, region 6 corresponding to amino acids 903 to 1004 of SEQ ID NO:2, region 7 corresponding to amino acids 1 to 153 of SEQ ID NO:2, region 8 corresponding to amino acids 177 to 613 of SEQ ID NO:2, region 9 corresponding to amino acids 659 to 730 of SEQ ID NO:2, region 10 corresponding to amino acids 804 to 806 of SEQ ID NO:2, region 11 corresponding to amino acids 847 to 871 of SEQ ID NO:2, region 12 corresponding to amino acids 886 to 902 of SEQ ID NO:2, and region 13 corresponding to amino acids 1005 to 1037 of SEQ ID NO:2.

In one embodiment, such variant has at least 60% and less than 100% sequence identity to SEQ ID NO:2, preferably said xanthan lyase variant has activity on xanthan gum, further preferably said activity is a xanthan gum degrading activity.

In one embodiment, the detergent composition of the present invention comprises a xanthan lyase variant, comprising an alteration, preferably a substitution, at one or more positions in one or more regions or multiple alterations (such as 2, 3, 4, 5, 6, 7, 8, 9 or 10) in one region or multiple alterations (e.g. 2, 3, 4, 5, 6, 7, 8, 9 or 10) in multiple regions (e.g. 2, 3, 4, 5, 6, 7, 8, or 9) selected from the group consisting of: region 1 corresponding to amino acids 154 to 176 of SEQ ID NO:2, region 2 corresponding to amino acids 614 to 658 of SEQ ID NO:2, region 3 corresponding to amino acids 731 to 803 of SEQ ID NO:2, region 4 corresponding to amino acids 807 to 846 of SEQ ID NO:2, region 5 corresponding to amino acids 872 to 885 of SEQ ID NO:2, region 6 corresponding to amino acids 903 to 1004 of SEQ ID NO:2, region 7 corresponding to amino acids 1 to 153 of SEQ ID NO:2, region 8 corresponding to amino acids 177 to 613 of SEQ ID NO:2, region 9 corresponding to amino acids 659 to 730 of SEQ ID NO:2, region 10 corresponding to amino acids 804 to 806 of SEQ ID NO:2, region 11 corresponding to amino acids 847 to 871 of SEQ ID NO:2, region 12 corresponding to amino acids 886 to 902 of SEQ ID NO:2, and region 13 corresponding to amino acids 1005 to 1037 of SEQ ID NO:2, wherein said variant has at least 60% and less than 100% sequence identity to SEQ ID NO:2, preferably said xanthan lyase variant has activity on xanthan gum, further preferably said activity is a xanthan gum degrading activity.

In one embodiment, the xanthan lyase variant, as described herein, is one that comprises an alteration, preferably a substitution, at one or more positions in: (i) regions 3 and 5; (ii) regions 3, 5 and 12; (iii) regions 8, and 9; (iv) regions 2, 3, and 5; (v) regions 2, 3, 5, and 12; (vi) regions 3, 5, 8, 9, and 12; (vii) regions 2, 3, 5, 8, and 9; (viii) 3, 5, 8, 9, and 12; (ix) 2, 3, 5, 8, 9, and 12; (x) region 3; (xi) regions 3, 4 and 5; (xii) regions 7, 8 and 9; (xiii) regions 12 and 13; (xiv) regions 3, 4, 5, 8, 9, and 12; (xv) regions 8, 9, 12, and 13; (xvi) regions 7, 8, 9, 12, and 13; (xvii) regions 3, 4, 5, 7, 8, 9, and 12; and (xviii) regions 3, 4, 5, 7, 8, 9, 12, and 13, wherein said variant preferably has no alteration in the other regions besides those mentioned.

In one embodiment, the detergent composition of the present invention comprises a xanthan lyase variant having at least 60%, e.g. at least 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% and less than 100% sequence identity to the amino acid sequence as set forth in SEQ ID NO:2.

In one embodiment, the detergent composition of the present invention comprises a xanthan lyase variant comprising an alteration, preferably a substitution, at one or more positions selected from the group consisting of 9, 15, 18, 46, 58, 66, 89, 95, 100, 106, 109, 155, 159, 183, 188, 190, 203, 204, 221, 229, 234, 238, 240, 242, 243, 257, 258, 284, 291, 293, 316, 317, 320, 324, 329, 333, 339, 341, 352, 354, 360, 372, 377, 399, 400, 419, 440, 450, 451, 454, 458, 481, 492, 505, 533, 567, 568, 576, 578, 579, 582, 620, 624, 626, 631, 635, 649, 650, 656, 664, 672, 703, 722, 726, 727, 728, 738, 745, 752, 753, 754, 757, 769, 775, 777, 779, 782, 786, 789, 792, 796, 799, 801, 819, 824, 843, 851, 855, 856, 867, 875, 887, 892, 899, 900, 901, 902, 903, 911, 912, 915, 919, 921, 923, 925, 927, 928, 930, 933, 941, 966, 991, 1008 and 1016, wherein said positions correspond to amino acid positions of SEQ ID NO:2.

In one embodiment, the detergent composition of the present invention comprises a xanthan lyase variant comprising an alteration, preferably a substitution, at 2, 3, 4, 5, 6, 7, 8, 9 or more positions selected from the group consisting of 9, 15, 18, 46, 58, 66, 89, 95, 100, 106, 109, 155, 159, 183, 188, 190, 203, 204, 221, 229, 234, 238, 240, 242, 243, 257, 258, 284, 291, 293, 316, 317, 320, 324, 329, 333, 339, 341, 352, 354, 360, 372, 377, 399, 400, 419, 440, 450, 451, 454, 458, 481, 492, 505, 533, 567, 568, 576, 578, 579, 582, 620, 624, 626, 631, 635, 649, 650, 656, 664, 672, 703, 722, 726, 727, 728, 738, 745, 752, 753, 754, 757, 769, 775, 777, 779, 782, 786, 789, 792, 796, 799, 801, 819, 824, 843, 851, 855, 856, 867, 875, 887, 892, 899, 900, 901, 902, 903, 911, 912, 915, 919, 921, 923, 925, 927, 928, 930, 933, 941, 966, 991, 1008 and 1016, wherein said positions correspond to amino acid positions of SEQ ID NO:2.

In a preferred embodiment, the detergent composition of the present invention comprises a xanthan lyase variant comprising an alteration at one or more positions selected from the group consisting of 624, 631, 635, 649, 656, 738, 752, 753, 754, 757, 769, 775, 777, 800, 801, 843, 875, 911 and 915, and/or an alteration at one or more positions selected from the group consisting of 89, 100, 190, 229, 234, 352, 360, 399, 440, 458, 492, 567, 582, 664, 672, 703, 728, 892, 1008 and 1016, wherein said positions correspond to amino acid positions of SEQ ID NO:2.

In one embodiment, the detergent composition of the present invention comprises a xanthan lyase variant comprising one or more amino acid substitutions selected from the group consisting of K9R, N15T, T18D, L46D, A58L, S66H, Q89Y, K95E, S100D, N106Y, Q109R, Q109D, Q109F, Q109K, Q109A, Y155E, A159P, K183Q, K183R, V188I, A190Q, A203P, K204R, A221P, E229N, E229S, E229V, I234V, I238W, I238L, I238M, I240W, N242S, G243V, Y257W, R258E, R284G, K291R, A293G, A293P, K316R, R317K, K320R, L324Q, K329R, K333R, L339M, I341P, V352I, S354P, K360G, K360R, Q372H, F377Y, N399K, K400R, F419Y, N440K, D450P, K451E, K451R, A454V, D458S, K481R, A492H, A492L, T505I, L533I, K567R, G568A, S578K, S578N, S578R, S579R, S579K, S582K, K620R, A624E, A626Q, T631N, S635E, S635T, T649V, T649K, Q650G, I656V, T664K, N672D, I703L, I722F, P726Q, T727P, M728V, G738L, K745R, P752R, P752K, G753E, G753Q, G753S, S754E, S754L, S754Q, S754R, S757D, S757P, S757E, A769D, A769T, A769R, L775A, L775F, L775I, L775M, L775Q, L775S, L775Y, D777R, P779V, Y782I, N786K, G789R, K792W, K792Y, K792V, K792A, N796Q, A799H, D801G, K819R, K824R, A843P, S851F, K855R, E856D, P867S, K875T, K875E, K887R, N892Y, N892W, N892F, G899S, I900G, D901A, T902F, T903A, T903Q, A911M, A911V, A911S, A912T, A912I, A912Y, T915S, T915V, T915Q, T919D, T919F, T919G, T921R, T921S, T923D, T923H, T925D, T925Q, T925R, T927K, D928W, Y930F, Y930H, Y930L, D933M, G941D, G941E, A966P, N991D, V998K, N1008D and K1016T, wherein said positions correspond to amino acid positions of SEQ ID NO:2.

In a preferred embodiment, the detergent composition of the present invention comprises a xanthan lyase variant comprising one or more substitutions selected from the group consisting of Q89Y, S100D, A190Q, E229S, I234V, V352I, K360G, N399K, N440K, D458S, A492H, A492L, K567R, S582K, T664K, N672D, I703L, M728V, N892Y N1008D and K1016T, and/or one or more substitutions selected from the group consisting of A624E, T631N, S635E, T649K, I656V, G738L, P752K, P752R, G753E, S754E, S754R, S757D, A769D, L775A, D777R, V800P, D801G, A843P, K875T, A911V and T915A, wherein said positions correspond to amino acid positions of SEQ ID NO:2.

In one embodiment, the detergent composition of the present invention comprises a xanthan lyase variant comprising a set of alterations selected from the group consisting of A190Q + E229S + S635E + T649K + 1656V + N672D + I703L + G753E + S754E + S757D + A769D + L775A + D801G + K875T + N892Y + N1008D, E229S + S635E + T649K + 1656V + N672D + I703L + G753E + S754E + A769D + L775A + D801G + K875T + N892Y + N1008D, E229S + V3521 + S635E + T649K + 1656V + N672D + G753E + S754E + A769D + L775A + V800P + D801G + K875T + N892Y, E229S + K360G + D458S + S582K + N672D + G753E + S754E + A769D + L775A + D801G + K875T + N892Y + N1008D, S1 OOD + E229S + K360G + D458S + S582K + T664K + N672D + G753E + S754E + S757D + A769D + L775A + D801G + A843P + K875T + N892Y + A911V + N1008D + K1016T, E229S + I234V + S582K + N672D + G753E + S754E + A769D + L775A + V800P + D801G + K875T + N892Y, Q89Y + E229S + N440K + S582K + A624E + N672D + G753E + S754E + A769D + L775A + D801G + K875T + N892Y, E229S + S635E + T649K + 1656V + N672D + P752K + G753E + A769D + L775A + D801G + A843P + K875T + N892Y, E229S + S635E + T649K + 1656V + N672D + G753E + S754E + S757D + A769D + L775A + D801G + K875T + N892Y, E229S + N440K + S582K + N672D + G753E + S754E + A769D + L775A + D801G + A843P + K875T + N892Y + N1008D, E229S + N440K + S582K + A624E + N672D + G753E + S754E + A769D + L775A + V800P + D801G + K875T + N892Y, A190Q + E229S + S635E + T649K + 1656V + N672D + P752K + G753E + A769D + L775A + D801G + A843P + K875T + N892Y, A190Q + E229S + S582K + N672D + G753E + S754E + A769D + L775A + D801G + K875T + N892Y, E229S + N440K + S582K + N672D + P752R + G753E + S754E + S757D + A769D + L775A + D801G + K875T + N892Y + N1008D, E229S + S582K + S635E + N672D + P752R + G753E + S754E + A769D + L775A + D801G + K875T + N892Y + N1008D, A190Q + E229S + N440K + S582K + A624E + S635E + N672D + G753E + S754E + A769D + L775A + D801G + K875T + N892Y, E229S + I234V + A492L + S582K + N672D + G753E + S754E + A769D + L775A + D801G + K875T + N892Y, A190Q + E229S + K360G + D458S + S582K + T664K + N672D + G753E + S754E + A769D + L775A + D801G + K875T + N892Y + N1008D, S100D + E229S + K360G + D458S + S582K + N672D + G753E + S754E + S757D + A769D + L775A + D801G + A843P + K875T + N892Y + T915A + N1008D, E229S + N440K + S582K + A624E+ S635E + N672D + G738L + G753E + S754E + S757D + A769D + L775A + D801G + K875T + N892Y, S100D + E229S + K360G + D458S + S582K + N672D + G753E + S754E + A769D + L775A + D801G + K875T + N892Y + N1008D, A190Q + E229S + D458S + T631N + N672D + G753E + S754E + A769D + L775A + D801G + A843P + K875T + N892Y, A190Q + E229S + K360G + D458S + S582K + N672D + G753E + S754E + A769D + L775A + D801G + K875T + N892Y + N1008D, E229S + S635E + T649K + 1656V + N672D + G753E + S754R + S757D + A769D + L775A + D801G + A843P + K875T + N892Y, E229S + D458S + S582K + T631N + S635E + N672D + M728V + G753E + S754E + S757D + A769D + L775A + D801G + K875T + N892Y, A190Q + E229S + K360G + D458S + S582K + N672D + G753E + S754E + S757D + A769D + L775A + D801G + K875T + N892Y + N1008D, E229S + A492L + S635E + T649K + I656V + N672D + G753E + S757D + A769D + L775A + D801G + K875T + N892Y, S100D + A190Q + E229S + K360G + D458S + S582K + N672D + G753E + S754E + A769D + L775A + D801G + K875T + N892Y + N1008D, A190Q + E229S + I234V + S582K + N672D + G753E + S754E + S757D + A769D + L775A + D801G + K875T + N892Y, E229S + N399K + D458S + A492H + K567R + S582K + S635E + T649K + N672D + G753E + S754E + A769D + L775A + D777R + D801G + K875T + N892Y, E229S + D458S + A492L + T631N + N672D + G753E + S754E + S757D + A769D + L775A + D801G + K875T + N892Y, E229S + D458S + A492H + K567R + S582K + S635E + N672D + G753E + S754E + A769D + L775A + D777R + D801G + K875T + N892Y, S100D + E229S + K360G + D458S + S582K + N672D + G753E + S754E + S757D + A769D + L775A + D801G + A843P + K875T + N892Y + N1008D, E229S + N399K + D458S + K567R + S582K + S635E + N672D + G753E + S754E + A769D + L775A + D777R + D801G + K875T + N892Y.

In a preferred embodiment, the detergent composition of the present invention comprises a xanthan lyase variant comprising a set of alterations selected from the group consisting of E229N + N672D + P752K + G753E + A769D + L775A + D801G + K875T + N892Y, E229S + N672D + G753E + S754E + A769D + L775A + D801G + K875T + N892Y, E229S + N672D + P752R + G753E + S754E + A769D + L775A + D801G + K875T + N892Y, A190Q + E229S + I234V + A624E + N672D + G753E + S754E + A769D + L775A + D801G + K875T, A190Q + E229S + T631N + N672D + I703L + P752K + G753E + A769D + L775A + D801G + K875T, A190Q + E229S + I234V + S582K + N672D + G753E + S754E + S757D + A769D + L775A + D801G + K875T + N892Y, E229S + N440K + S582K + A624E + S635E + N672D + G738L + G753E + S754E + S757D + A769D + L775A + D801G + K875T + N892Y, and S100D + E229S + K360G + D458S + S582K + N672D + G753E + S754E + S757D + A769D + L775A + D801G + A843P + K875T + N892Y + N1008D, wherein said positions correspond to amino acid positions of SEQ ID NO:2.

In a particular aspect, the xanthan lyase variant has besides the aforementioned alterations no further alterations relative to the parent enzyme of SEQ ID NO:2, i.e. the remaining sequence is identical to SEQ ID NO:2.

### Further preferred embodiments

In various embodiments, the detergent composition of the present invention comprises preferred endoglucanase variants in combination with the preferred xanthan lyase variants.

In a preferred embodiment, the detergent composition of the present invention thus comprises
(A) an endoglucanase variant having at least 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NO:1 and comprising a set of alterations selected from the group consisting of (A1) A559N + Y579W+ T697G, (A2) K512P + A559N + Y579W+ T697G, (A3) N18G + A71E + A186P + E408D + Y579W+ I602T + A651P + A688G + V756Y, (A4) N18G + N189K + E408D + A559N + Y579W+ A688G + T697G + V756Y + K921R + Y934G, (A5) S313D + E408D, (A6) R880K + N905D + K921R + Y934G, (A7) I302D + S313D + E408D + Y579W + I602T + A651P + T697G + R880K + K921R + Y934G, and (A8) N216Q + S313D + E408D + D476R + Y579W + I602T + F638N + A651P + T697G + W719R + R880K + T887K + K921R + Y934G, wherein said positions correspond to amino acid positions of SEQ ID NO:1; and/or
(B) a xanthan lyase variant having at least 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NO:2 and comprising a set of alterations selected from the group consisting of (B1) E229N + N672D + P752K + G753E + A769D + L775A + D801G + K875T + N892Y, (B2) E229S + N672D + G753E + S754E + A769D + L775A + D801G + K875T + N892Y, (B3) E229S + N672D + P752R + G753E + S754E + A769D + L775A + D801G + K875T + N892Y, (B4) A190Q + E229S + I234V + A624E + N672D + G753E + S754E + A769D + L775A + D801G + K875T, (B5) A190Q + E229S + T631N + N672D + I703L + P752K + G753E + A769D + L775A + D801G + K875T, (B6) A190Q + E229S + I234V + S582K + N672D + G753E + S754E + S757D + A769D + L775A + D801G + K875T + N892Y, (B7) E229S + N440K + S582K + A624E + S635E + N672D + G738L + G753E + S754E + S757D + A769D + L775A + D801G + K875T + N892Y, and (B8) S100D + E229S + K360G + D458S + S582K + N672D + G753E + S754E + S757D + A769D + L775A + D801G + A843P + K875T + N892Y + N1008D, wherein said positions correspond to amino acid positions of SEQ ID NO:2; and
(C) an enzyme stabilizing component.

In a further preferred embodiment, the detergent composition of the present invention comprises
(A) an endoglucanase variant having at least 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NO:1 and comprising a set of alterations selected from the group consisting of (A1) A559N + Y579W+ T697G, (A2) K512P + A559N + Y579W+ T697G, (A3) N18G + A71E + A186P + E408D + Y579W+ I602T + A651P + A688G + V756Y, (A4) N18G + N189K + E408D + A559N + Y579W+ A688G + T697G + V756Y + K921R + Y934G, (A5) S313D + E408D, (A6) R880K + N905D + K921R + Y934G, (A7) I302D + S313D + E408D + Y579W + I602T + A651P + T697G + R880K + K921R + Y934G, and (A8) N216Q + S313D + E408D + D476R + Y579W + I602T + F638N + A651P + T697G + W719R + R880K + T887K + K921R + Y934G, wherein said positions correspond to amino acid positions of SEQ ID NO:1; and
(B) a xanthan lyase variant having at least 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NO:2 and comprising a set of alterations selected from the group consisting of (B1) E229N + N672D + P752K + G753E + A769D + L775A + D801G + K875T + N892Y, (B2) E229S + N672D + G753E + S754E + A769D + L775A + D801G + K875T + N892Y, (B3) E229S + N672D + P752R + G753E + S754E + A769D + L775A + D801G + K875T + N892Y, (B4) A190Q + E229S + I234V + A624E + N672D + G753E + S754E + A769D + L775A + D801G + K875T, (B5) A190Q + E229S + T631N + N672D + I703L + P752K + G753E + A769D + L775A + D801G + K875T, (B6) A190Q + E229S + I234V + S582K + N672D + G753E + S754E + S757D + A769D + L775A + D801G + K875T + N892Y, (B7) E229S + N440K + S582K + A624E + S635E + N672D + G738L + G753E + S754E + S757D + A769D + L775A + D801G + K875T + N892Y, and (B8) S100D + E229S + K360G + D458S + S582K + N672D + G753E + S754E + S757D + A769D + L775A + D801G + A843P + K875T + N892Y + N1008D, wherein said positions correspond to amino acid positions of SEQ ID NO:2; and
(C) an enzyme stabilizing component, wherein the enzyme stabilizing component is selected from the group consisting of polyols such as glycerol or 1,2-propylene glycol, alditols such as non-cyclic polyols having the formula HOCH₂[CH(OH)]ₙCH₂OH, mannitol, isomalt, lactit, sorbitol, xylitol, threitol, erythritol or arabitol, alkanol amines such as ethanol amine, or formates such as sodium formate, potassium formate, calcium formate or magnesium formate, preferably said enzyme stabilizing component is selected from glycerol and formates, more preferably being sodium formate.

In a particular preferred embodiment, the detergent composition of the present invention comprises
(A) an endoglucanase variant having at least 90%, 91%, 92%, 93%, 94%, 95%, 96%, 97%, 98% or 99% sequence identity to SEQ ID NO:1 and comprising a set of alterations selected from the group consisting of (A1) A559N + Y579W+ T697G, (A2) K512P + A559N + Y579W+ T697G, (A3) N18G + A71E + A186P + E408D + Y579W + I602T + A651P + A688G + V756Y, (A4) N18G + N189K + E408D + A559N + Y579W + A688G + T697G + V756Y + K921R + Y934G, (A5) S313D + E408D, (A6) R880K + N905D + K921R + Y934G, (A7) I302D + S313D + E408D + Y579W + I602T + A651P + T697G + R880K + K921R + Y934G, and (A8) N216Q + S313D + E408D + D476R + Y579W+ I602T + F638N + A651P + T697G + W719R + R880K + T887K + K921R + Y934G, wherein said positions correspond to amino acid positions of SEQ ID NO:1; and
(B) a xanthan lyase variant having at least 90%, 91%, 92%, 93%, 94%, 95%, 96%, 97%, 98% or 99% sequence identity to SEQ ID NO:2 and comprising a set of alterations selected from the group consisting of (B1) E229N + N672D + P752K + G753E + A769D + L775A + D801G + K875T + N892Y, (B2) E229S + N672D + G753E + S754E + A769D + L775A + D801G + K875T + N892Y, (B3) E229S + N672D + P752R + G753E + S754E + A769D + L775A + D801G + K875T + N892Y, (B4) A190Q + E229S + I234V + A624E + N672D + G753E + S754E + A769D + L775A + D801G + K875T, (B5) A190Q + E229S + T631N + N672D + I703L + P752K + G753E + A769D + L775A + D801G + K875T, (B6) A190Q + E229S + I234V + S582K + N672D + G753E + S754E + S757D + A769D + L775A + D801G + K875T + N892Y, (B7) E229S + N440K + S582K + A624E + S635E + N672D + G738L + G753E + S754E + S757D + A769D + L775A + D801G + K875T + N892Y, and (B8) S100D + E229S + K360G + D458S + S582K + N672D + G753E + S754E + S757D + A769D + L775A + D801G + A843P + K875T + N892Y + N1008D, wherein said positions correspond to amino acid positions of SEQ ID NO:2; and
(C) an enzyme stabilizing component, wherein the enzyme stabilizing component is selected from glycerol and sodium formate, preferably being sodium formate.

In one embodiment, the endoglucanase and/or the xanthan lyase variant do not comprise any further substitution besides those explicitly mentioned above, i.e. the remainder of the sequence is identical to that of the parent enzyme as set forth in SEQ ID NO:1 and SEQ ID NO:2, respectively.

In one embodiment, the detergent composition of the present invention comprises the endoglucanase variant (A1), as defined above, in combination with any one of the xanthan lyase variants (B1), (B2), (B3), (B4), (B5), (B6), (B7) and (B8), as defined above, wherein the respective enzymes preferably have at least 90%, 91%, 92%, 93%, 94%, 95%, 96%, 97%, 98% or 99% sequence identity to SEQ ID NO:1 and SEQ ID NO:2, respectively, preferably are identical to their respective parent sequence with the exception of the substitutions explicitly mentioned herein.

In one embodiment, the detergent composition of the present invention comprises the endoglucanase variant (A2), as defined above, in combination with any one of the xanthan lyase variants (B1), (B2), (B3), (B4), (B5), (B6), (B7) and (B8), as defined above, wherein the respective enzymes preferably have at least 90%, 91%, 92%, 93%, 94%, 95%, 96%, 97%, 98% or 99% sequence identity to SEQ ID NO:1 and SEQ ID NO:2, respectively, preferably are identical to their respective parent sequence with the exception of the substitutions explicitly mentioned herein.

In one embodiment, the detergent composition of the present invention comprises the endoglucanase variant (A3), as defined above, in combination with any one of the xanthan lyase variants (B1), (B2), (B3), (B4), (B5), (B6), (B7) and (B8), as defined above, wherein the respective enzymes preferably have at least 90%, 91%, 92%, 93%, 94%, 95%, 96%, 97%, 98% or 99% sequence identity to SEQ ID NO:1 and SEQ ID NO:2, respectively, preferably are identical to their respective parent sequence with the exception of the substitutions explicitly mentioned herein.

In one embodiment, the detergent composition of the present invention comprises the endoglucanase variant (A4), as defined above, in combination with any one of the xanthan lyase variants (B1), (B2), (B3), (B4), (B5), (B6), (B7) and (B8), as defined above, wherein the respective enzymes preferably have at least 90%, 91%, 92%, 93%, 94%, 95%, 96%, 97%, 98% or 99% sequence identity to SEQ ID NO:1 and SEQ ID NO:2, respectively, preferably are identical to their respective parent sequence with the exception of the substitutions explicitly mentioned herein.

In one embodiment, the detergent composition of the present invention comprises the endoglucanase variant (A5), as defined above, in combination with any one of the xanthan lyase variants (B1), (B2), (B3), (B4), (B5), (B6), (B7) and (B8), as defined above, wherein the respective enzymes preferably have at least 90%, 91%, 92%, 93%, 94%, 95%, 96%, 97%, 98% or 99% sequence identity to SEQ ID NO:1 and SEQ ID NO:2, respectively, preferably are identical to their respective parent sequence with the exception of the substitutions explicitly mentioned herein.

In one embodiment, the detergent composition of the present invention comprises the endoglucanase variant (A6), as defined above, in combination with any one of the xanthan lyase variants (B1), (B2), (B3), (B4), (B5), (B6), (B7) and (B8), as defined above, wherein the respective enzymes preferably have at least 90%, 91%, 92%, 93%, 94%, 95%, 96%, 97%, 98% or 99% sequence identity to SEQ ID NO:1 and SEQ ID NO:2, respectively, preferably are identical to their respective parent sequence with the exception of the substitutions explicitly mentioned herein.

In one embodiment, the detergent composition of the present invention comprises the endoglucanase variant (A7), as defined above, in combination with any one of the xanthan lyase variants (B1), (B2), (B3), (B4), (B5), (B6), (B7) and (B8), as defined above, wherein the respective enzymes preferably have at least 90%, 91%, 92%, 93%, 94%, 95%, 96%, 97%, 98% or 99% sequence identity to SEQ ID NO:1 and SEQ ID NO:2, respectively, preferably are identical to their respective parent sequence with the exception of the substitutions explicitly mentioned herein.

In one embodiment, the detergent composition of the present invention comprises the endoglucanase variant (A8), as defined above, in combination with any one of the xanthan lyase variants (B1), (B2), (B3), (B4), (B5), (B6), (B7) and (B8), as defined above, wherein the respective enzymes preferably have at least 90%, 91%, 92%, 93%, 94%, 95%, 96%, 97%, 98% or 99% sequence identity to SEQ ID NO:1 and SEQ ID NO:2, respectively, preferably are identical to their respective parent sequence with the exception of the substitutions explicitly mentioned herein.

In one embodiment, the detergent composition of the present invention comprises the xanthan lyase variant (B1), as defined above, in combination with any one of the endoglucanase variants (A1), (A2), (A3), (A4), (A5), (A6), (A7) and (A8), as defined above, wherein the respective enzymes preferably have at least 90%, 91%, 92%, 93%, 94%, 95%, 96%, 97%, 98% or 99% sequence identity to SEQ ID NO:1 and SEQ ID NO:2, respectively, preferably are identical to their respective parent sequence with the exception of the substitutions explicitly mentioned herein.

In one embodiment, the detergent composition of the present invention comprises the xanthan lyase variant (B2), as defined above, in combination with any one of the endoglucanase variants (A1), (A2), (A3), (A4), (A5), (A6), (A7) and (A8), as defined above, wherein the respective enzymes preferably have at least 90%, 91%, 92%, 93%, 94%, 95%, 96%, 97%, 98% or 99% sequence identity to SEQ ID NO:1 and SEQ ID NO:2, respectively, preferably are identical to their respective parent sequence with the exception of the substitutions explicitly mentioned herein.

In one embodiment, the detergent composition of the present invention comprises the xanthan lyase variant (B3), as defined above, in combination with any one of the endoglucanase variants (A1), (A2), (A3), (A4), (A5), (A6), (A7) and (A8), as defined above, wherein the respective enzymes preferably have at least 90%, 91%, 92%, 93%, 94%, 95%, 96%, 97%, 98% or 99% sequence identity to SEQ ID NO:1 and SEQ ID NO:2, respectively, preferably are identical to their respective parent sequence with the exception of the substitutions explicitly mentioned herein.

In one embodiment, the detergent composition of the present invention comprises the xanthan lyase variant (B4), as defined above, in combination with any one of the endoglucanase variants (A1), (A2), (A3), (A4), (A5), (A6), (A7) and (A8), as defined above, wherein the respective enzymes preferably have at least 90%, 91%, 92%, 93%, 94%, 95%, 96%, 97%, 98% or 99% sequence identity to SEQ ID NO:1 and SEQ ID NO:2, respectively, preferably are identical to their respective parent sequence with the exception of the substitutions explicitly mentioned herein.

In one embodiment, the detergent composition of the present invention comprises the xanthan lyase variant (B5), as defined above, in combination with any one of the endoglucanase variants (A1), (A2), (A3), (A4), (A5), (A6), (A7) and (A8), as defined above, wherein the respective enzymes preferably have at least 90%, 91%, 92%, 93%, 94%, 95%, 96%, 97%, 98% or 99% sequence identity to SEQ ID NO:1 and SEQ ID NO:2, respectively, preferably are identical to their respective parent sequence with the exception of the substitutions explicitly mentioned herein.

In one embodiment, the detergent composition of the present invention comprises the xanthan lyase variant (B6), as defined above, in combination with any one of the endoglucanase variants (A1), (A2), (A3), (A4), (A5), (A6), (A7) and (A8), as defined above, wherein the respective enzymes preferably have at least 90%, 91%, 92%, 93%, 94%, 95%, 96%, 97%, 98% or 99% sequence identity to SEQ ID NO:1 and SEQ ID NO:2, respectively, preferably are identical to their respective parent sequence with the exception of the substitutions explicitly mentioned herein.

In one embodiment, the detergent composition of the present invention comprises the xanthan lyase variant (B7), as defined above, in combination with any one of the endoglucanase variants (A1), (A2), (A3), (A4), (A5), (A6), (A7) and (A8), as defined above, wherein the respective enzymes preferably have at least 90%, 91%, 92%, 93%, 94%, 95%, 96%, 97%, 98% or 99% sequence identity to SEQ ID NO:1 and SEQ ID NO:2, respectively, preferably are identical to their respective parent sequence with the exception of the substitutions explicitly mentioned herein.

In one embodiment, the detergent composition of the present invention comprises the xanthan lyase variant (B8), as defined above, in combination with any one of the endoglucanase variants (A1), (A2), (A3), (A4), (A5), (A6), (A7) and (A8), as defined above, wherein the respective enzymes preferably have at least 90%, 91%, 92%, 93%, 94%, 95%, 96%, 97%, 98% or 99% sequence identity to SEQ ID NO:1 and SEQ ID NO:2, respectively, preferably are identical to their respective parent sequence with the exception of the substitutions explicitly mentioned herein.

If not explicitly indicated otherwise, all the endoglucanase and/or xanthan lyase variants, as described above, may further comprise one or more additional alterations at one or more (e.g. several) other positions in any of the regions described herein.

The amino acid changes may be of a minor nature, that is conservative amino acid substitutions or insertions that do not significantly affect the folding and/or activity of the protein, small deletions, typically of 1-30 amino acids, small amino- or carboxyl-terminal extensions, such as an amino-terminal methionine residue, a small linker peptide of up to 20-25 residues or a small extension that facilitates purification by changing net charge or another function, such as a poly-histidine tract, an antigenic epitope or a binding domain.

Examples of conservative substitutions are within the groups of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions that do not generally alter specific activity are known in the art. Common substitutions are Ala/Ser, VaI/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly.

Alternatively, the amino acid changes are of such a nature that the physico-chemical properties of the polypeptides are altered. For example, amino acid changes may improve the thermal stability of the polypeptide, alter the substrate specificity, change the pH optimum, and the like.

Essential amino acids in a polypeptide can be identified according to procedures known in the art, such as site-directed mutagenesis or alanine-scanning mutagenesis. The active site of the enzyme or other biological interaction can also be determined by physical analysis of structure, as determined by such techniques as nuclear magnetic resonance, crystallography, electron diffraction, or photoaffinity labeling, in conjunction with mutation of putative contact site amino acids. The identity of essential amino acids can also be inferred from an alignment with a related polypeptide.

In one embodiment, the detergent composition of the present invention additionally comprises one or more further detergent components, preferably a surfactant.

In one embodiment, the detergent composition of the present invention comprises 0.000001 to 10 wt.-%, such as 0.00001 to 5 wt.-%, preferably 0.0001 to 1 wt.-%, more preferably 0.0005 to 0.5 wt.-%, even more preferably 0.001 to 0.1 wt.-%, of said endoglucanase variant (active enzyme per ml detergent composition) and/or 0.000001 to 10 wt.-%, such as 0.00001 to 5 wt.-%, preferably 0.0001 to 1 wt.-%, more preferably 0.0005 to 0.5 wt.-%, even more preferably 0.001 to 0.1 wt.-%, of said xanthan lyase variant (active enzyme per ml detergent composition) and/or 0.001 to 20 wt.-%, such as 0.005 to 15 wt.-%, preferably 0.01 to 10 wt.-%, more preferably 0.1 to 5.5 wt.-%, even more preferably 0.25 to 1.5 wt.-%, of said enzyme stabilizing agent (by weight of the detergent composition).

In one embodiment, the detergent composition of the present invention comprises said endoglucanase variant and/or said xanthan lyase variant and said enzyme stabilizing agent in a weight ratio (enzyme:stabilizer) of 0.00001:1 to 0.1:2, preferably 0.0001:1 to 0.01:1, more preferably 0.0005:1 to 0.001:1.

Without being bound to the theory, it is conceivable that the addition of the enzyme stabilizing component directly interacts with the natively folded enzymes and/or influences the interaction of the enzymes and other detergent ingredients like surfactants, chelators, water, and others.

In one embodiment, the preferred endoglucanase and/or xanthan lyase variants have an improved stability in a detergent composition containing such enzyme stabilizing component(s) compared to a detergent composition not containing such enzyme stabilizing component(s).

In this respect, in a preferred embodiment, the detergent composition of the present invention comprises
(A) 0.0005 to 0.5 wt.-%, preferably 0.001 to 0.1 wt.-%, of an endoglucanase variant having at least 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NO:1 and comprising a set of alterations selected from the group consisting of (A1) A559N + Y579W + T697G, (A2) K512P + A559N + Y579W+ T697G, (A3) N18G + A71E + A186P + E408D + Y579W + I602T + A651P + A688G + V756Y, (A4) N18G + N189K + E408D + A559N + Y579W + A688G + T697G + V756Y + K921R + Y934G, (A5) S313D + E408D, (A6) R880K + N905D + K921R + Y934G, (A7) I302D + S313D + E408D + Y579W + I602T + A651P + T697G + R880K + K921R + Y934G, and (A8) N216Q + S313D + E408D + D476R + Y579W + I602T + F638N + A651P + T697G + W719R + R880K + T887K + K921R + Y934G, wherein said positions correspond to amino acid positions of SEQ ID NO:1;
(B) 0.0005 to 0.5 wt.-%, preferably 0.001 to 0.1 wt.-%, of a xanthan lyase variant having at least 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NO:2 and comprising a set of alterations selected from the group consisting of (B1) E229N + N672D + P752K + G753E + A769D + L775A + D801G + K875T + N892Y, (B2) E229S + N672D + G753E + S754E + A769D + L775A + D801G + K875T + N892Y, (B3) E229S + N672D + P752R + G753E + S754E + A769D + L775A + D801G + K875T + N892Y, (B4) A190Q + E229S + I234V + A624E + N672D + G753E + S754E + A769D + L775A + D801G + K875T, (B5) A190Q + E229S + T631N + N672D + I703L + P752K + G753E + A769D + L775A + D801G + K875T, (B6) A190Q + E229S + I234V + S582K + N672D + G753E + S754E + S757D + A769D + L775A + D801G + K875T + N892Y, (B7) E229S + N440K + S582K + A624E + S635E + N672D + G738L + G753E + S754E + S757D + A769D + L775A + D801G + K875T + N892Y, and (B8) S100D + E229S + K360G + D458S + S582K + N672D + G753E + S754E + S757D + A769D + L775A + D801G + A843P + K875T + N892Y + N1008D, wherein said positions correspond to amino acid positions of SEQ ID NO:2;
(C) 0.1 to 5.5 wt.-%, preferably 0.25 to 1.5 wt.-%, of an enzyme stabilizing component, wherein the enzyme stabilizing component is selected from the group consisting of glycerol and formates, preferably being sodium formate; and
(D) 0 to 99 wt.% of at least one further detergent component, preferably a surfactant.

In a particular preferred embodiment, the detergent composition of the present invention comprises
(A) 0.0005 to 0.5 wt.-%, preferably 0.001 to 0.1 wt.-%, of an endoglucanase variant having at least 90%, 91%, 92%, 93%, 94%, 95%, 96%, 97%, 98% or 99% sequence identity to SEQ ID NO:1 and comprising a set of alterations selected from the group consisting of (A1) A559N + Y579W + T697G, (A2) K512P + A559N + Y579W + T697G, (A3) N18G + A71E + A186P + E408D + Y579W + I602T + A651P + A688G + V756Y, (A4) N18G + N189K + E408D + A559N + Y579W + A688G + T697G + V756Y + K921R + Y934G, (A5) S313D + E408D, (A6) R880K + N905D + K921R + Y934G, (A7) I302D + S313D + E408D + Y579W + I602T + A651P + T697G + R880K + K921R + Y934G, and (A8) N216Q + S313D + E408D + D476R + Y579W + I602T + F638N + A651P + T697G + W719R + R880K + T887K + K921R + Y934G, wherein said positions correspond to amino acid positions of SEQ ID NO:1;
(B) 0.0005 to 0.5 wt.-%, preferably 0.001 to 0.1 wt.-%, of a xanthan lyase variant having at least 90%, 91%, 92%, 93%, 94%, 95%, 96%, 97%, 98% or 99% sequence identity to SEQ ID NO:2 and comprising a set of alterations selected from the group consisting of (B1) E229N + N672D + P752K + G753E + A769D + L775A + D801G + K875T + N892Y, (B2) E229S + N672D + G753E + S754E + A769D + L775A + D801G + K875T + N892Y, (B3) E229S + N672D + P752R + G753E + S754E + A769D + L775A + D801G + K875T + N892Y, (B4) A190Q + E229S + I234V + A624E + N672D + G753E + S754E + A769D + L775A + D801G + K875T, (B5) A190Q + E229S + T631N + N672D + I703L + P752K + G753E + A769D + L775A + D801G + K875T, (B6) A190Q + E229S + I234V + S582K + N672D + G753E + S754E + S757D + A769D + L775A + D801G + K875T + N892Y, (B7) E229S + N440K + S582K + A624E + S635E + N672D + G738L + G753E + S754E + S757D + A769D + L775A + D801G + K875T + N892Y, and (B8) S100D + E229S + K360G + D458S + S582K + N672D + G753E + S754E + S757D + A769D + L775A + D801G + A843P + K875T + N892Y + N1008D, wherein said positions correspond to amino acid positions of SEQ ID NO:2;
(C) 0.1 to 5.5 wt.-%, preferably 0.25 to 1.5 wt.-% of an enzyme stabilizing component, wherein the enzyme stabilizing component is sodium formate; and
(D) 0 to 99 wt.% of at least one further detergent component, preferably a surfactant.

### Detergent compositions

In one certain aspect, the preferred endoglucanase variants and/or xanthan lyase variants have an improved stability in a detergent composition containing enzyme stabilizing component selected from the group consisting of polyols such as glycerol or 1,2-propylene glycol, alditols such as non-cyclic polyols having the formula HOCH₂[CH(OH)]ₙCH₂OH, mannitol, isomalt, lactit, sorbitol, xylitol, threitol, erythritol or arabitol, alkanol amines such as ethanol amine, or formates such as sodium formate, potassium formate, calcium formate or magnesium formate, preferably said enzyme stabilizing component is selected from glycerol and formates, more preferably being sodium formate, compared to a detergent composition not containing such enzyme stabilizing component(s), wherein activity and/or stability in detergent is measured as disclosed in examples 2 and 3 herein.

Besides said endoglucanase variants and/or xanthan lyase variants, the detergent composition of the present invention may comprise additional components. The choice of additional components is within the skill of the artisan and includes further enzymes and conventional ingredients, including the exemplary non-limiting components set forth below. The choice of components may include, for fabric care, the consideration of the type of fabric to be cleaned, the type and/or degree of soiling, the temperature at which cleaning is to take place, and the formulation of the detergent product. Although components mentioned below are categorized by general header according to a particular functionality, this is not to be construed as a limitation, as a component may comprise additional functionalities as will be appreciated by the skilled artisan.

The detergent composition of the present invention may be suitable for the laundering of textiles such as e.g. fabrics, cloths or linen, or for cleaning hard surfaces such as e.g. floors, tables, or dish wash.

In one embodiment, an endoglucanase variant, as described herein, may be added to a detergent composition in an amount corresponding to 0.0001 to 200 mg, such as 0.0005 to 100 mg, preferably 0.001 to 30 mg, more preferably 0.005 to 8 mg, even more preferably 0.01 to 2 mg, of enzyme protein per liter of wash liquor.

In one embodiment, an xanthan lyase variant, as described herein, may be added to a detergent composition of the present invention in an amount corresponding to 0.0001 to 200 mg, such as 0.0005 to 100 mg, preferably 0.001 to 30 mg, more preferably 0.005 to 8 mg, even more preferably 0.01 to 2 mg, of enzyme protein per liter of wash liquor.

In some embodiments, each an endoglucanase variant, as described herein, and a xanthan lyase variant, as described herein, may be added to a detergent composition of the present invention each in an amount corresponding to 0.0001 to 200 mg of enzyme protein, such as 0.0005 to 100 mg, preferably 0.001 to 30 mg, more preferably 0.005 to 8 mg, even more preferably 0.01 to 2 mg, of enzyme protein per liter of wash liquor.

A composition for use in automatic dishwash (ADW) may include 0.0001 to 50 wt.-%, such as 0.001 to 20 wt.-%, preferably 0.01 to 10 wt.-%, more preferably 0.05 to 5 wt.-%, even more preferably 0.001 to 0.1 wt.-%, of each of the enzyme proteins by weight of the composition.

A composition for use in laundry granulation or a solid/granular laundry composition in general may include 0.0001 to 50 wt.-%, such as 0.001 to 20 wt.-%, preferably 0.01 to 10 wt.-%, more preferably 0.05 to 5 wt.-%, even more preferably 0.001 to 0.1 wt.-%, of each of the enzyme proteins by weight of the composition.

A composition for use in laundry liquid may include 0.0001 to 10 wt.-%, such as 0.001 to 7 wt.-%, preferably 0.1 to 5 wt.-%, more preferably 0.001 to 0.1 wt.-%, of each of the enzyme proteins by weight of the composition.

A composition for use in automatic dishwash (ADW) may include 0.01 to 20 wt.-%, such as 0.1 to 10 wt.-%, preferably 0.2 to 8 wt.-%, more preferably 0.5 to 5 wt.-%, even more preferably 0.75 to 2.5 wt.-%, of said enzyme stabilizing component by weight of the composition.

A composition for use in laundry granulation or a solid/granular laundry composition in general may include 0.01 to 20 wt.-%, such as 0.1 to 10 wt.-%, preferably 0.2 to 8 wt.-%, more preferably 0.5 to 5 wt.-%, even more preferably 0.75 to 2.5 wt.-%, of said enzyme stabilizing component by weight of the composition.

A composition for use in laundry liquid may include 0.01 to 20 wt.-%, such as 0.1 to 10 wt.-%, preferably 0.2 to 8 wt.-%, more preferably 0.5 to 5 wt.-%, even more preferably 0.75 to 2.5 wt.-%, of said enzyme stabilizing component by weight of the composition.

A composition for use in automatic dishwash (ADW) may include said endoglucanase variant and/or said xanthan lyase variant and said enzyme stabilizing agent in a weight ratio (enzyme:stabilizer) of 0.00001:1 to 0.1:2, preferably 0.0001:1 to 0.01:1, more preferably 0.0005:1 to 0.001:1.

A composition for use in laundry granulation or a solid/granular laundry composition in general may include said endoglucanase variant and/or said xanthan lyase variant and said enzyme stabilizing agent in a weight ratio (enzyme:stabilizer) of 0.00001:1 to 0.1:2, preferably 0.0001:1 to 0.01:1, more preferably 0.0005:1 to 0.001:1.

A composition for use in laundry liquid may include said endoglucanase variant and/or said xanthan lyase variant and said enzyme stabilizing agent in a weight ratio (enzyme:stabilizer) of 0.00001:1 to 0.1:2, preferably 0.0001:1 to 0.01:1, more preferably 0.0005:1 to 0.001:1.

Further to the specific enzyme stabilizing component, as described herein, the enzyme(s) of the detergent composition of the present invention may be stabilized using conventional stabilizing agents, e.g. a polyol such as propylene glycol or glycerol, a sugar or sugar alcohol, lactic acid, boric acid, or a boric acid derivative, e.g. an aromatic borate ester, or a phenyl boronic acid derivative such as 4-formyl phenyl boronic acid (4-FPBA), and the composition may be formulated as described in e.g. WO 92/19709 and WO 92/19708.

In certain markets different wash conditions and, as such, different types of detergents are used. This is disclosed in e.g. EP 1025240. In Asia (Japan) a low detergent concentration system is used, while the United States uses a medium detergent concentration system, and Europe uses a high detergent concentration system. A low detergent concentration system includes detergents where less than about 800 ppm of detergent components are present in the wash water. Japanese detergents are typically considered low detergent concentration system as they have approximately 667 ppm of detergent components present in the wash water. A medium detergent concentration includes detergents where between about 800 ppm and about 2000 ppm of detergent components are present in the wash water. North American detergents are generally considered to be medium detergent concentration systems as they have approximately 975 ppm of detergent components present in the wash water. A high detergent concentration system includes detergents where greater than about 2000 ppm of detergent components are present in the wash water. European detergents are generally considered to be high detergent concentration systems as they have approximately 4500 to 5000 ppm of detergent components in the wash water. Latin American detergents are generally high suds phosphate builder detergents and the range of detergents used in Latin America can fall in both the medium and high detergent concentrations as they range from 1500 to 6000 ppm of detergent components in the wash water. Such detergent compositions are all embodiments of the invention. A polypeptide of the present invention may also be incorporated in the detergent formulations disclosed in WO 97/07202.

### Surfactants

The detergent composition of the present invention may comprise one or more surfactants, which may be anionic and/or cationic and/or non-ionic and/or semi-polar and/or zwitterionic, or a mixture thereof. In preferred embodiments, the detergent compositions of the invention comprise at least one surfactant. In a particular embodiment, the detergent composition of the present invention includes a mixture of one or more nonionic surfactants and one or more anionic surfactants. The surfactant(s) is/are typically present at a level of from about 0.1 to 60 wt.-%, such as 1 to 40 wt.-%, 3 to 20 wt.-% or 3 to 10 wt.-%. The surfactant(s) is/are chosen based on the desired cleaning application, and includes any conventional surfactant(s) known in the art. Any surfactant known in the art for use in detergents may be utilized.

When included therein, the detergent will usually comprise from about 1 to 40 wt.-%, such as 5 to 30 wt.-%, 5 to 15 wt.-% or 20 to 25 wt.-%, of an anionic surfactant. Non-limiting examples of anionic surfactants include sulfates and sulfonates, in particular linear alkyl benzene sulfonates (LAS), isomers of LAS, branched alkyl benzene sulfonates (BABS), phenyl alkane sulfonates, alpha-olefin sulfonates (AOS), olefin sulfonates, alkene sulfonates, alkane-2,3-diylbis(sulfates), hydroxy alkane sulfonates and disulfonates, alkyl sulfates (AS) such as sodium dodecyl sulfate (SDS), fatty alcohol sulfates (FAS), primary alcohol sulfates (PAS), alcohol ether sulfates (AES or AEOS or FES, also known as alcohol ethoxy sulfates or fatty alcohol ether sulfates), secondary alkane sulfonates (SAS), paraffin sulfonates (PS), ester sulfonates, sulfonated fatty acid glycerol esters, alpha-sulfo fatty acid methyl esters (alpha-SFMe or SES) including methyl ester sulfonate (MES), alkyl- or alkenyl succinic acid, dodecenyl/tetradecenyl succinic acid (DTSA), fatty acid derivatives of amino acids, diesters and monoesters of sulfo succinic acid or soap, and combinations thereof.

When included therein, the detergent will usually comprise from about 0 to 10 wt.-% of a cationic surfactant. Non-limiting examples of cationic surfactants include alkyl dimethyl ethanolamine quat (ADMEAQ), cetyl trimethyl ammonium bromide (CTAB), dimethyl distearyl ammonium chloride (DSDMAC), and alkyl benzyl dimethyl ammonium, alkyl quaternary ammonium compounds, alkoxylated quaternary ammonium (AQA) compounds, and combinations thereof.

When included therein, the detergent will usually comprise from about 0.2 to 40 wt.-% of a non-ionic surfactant, e.g. 0.5 to 30 wt.-%, in particular 1 to 20 wt.-%, 3 to 10 wt.-%, 3 to 5 wt.-% or 8 to 12 wt.-%. Non-limiting examples of non-ionic surfactants include alcohol ethoxylates (AE or AEO), alcohol propoxylates, propoxylated fatty alcohols (PFA), alkoxylated fatty acid alkyl esters, such as ethoxylated and/or propoxylated fatty acid alkyl esters, alkyl phenol ethoxylates (APE), nonyl phenol ethoxylates (NPE), alkyl polyglycosides (APG), alkoxylated amines, fatty acid monoethanolamides (FAM), fatty acid diethanol amides (FADA), ethoxylated fatty acid monoethanolamides (EFAM), propoxylated fatty acid monoethanolamides (PFAM), polyhydroxy alkyl fatty acid amides, or N-acyl N-alkyl derivatives of glucosamine (glucamides, GA, or fatty acid glucamide, FAGA), as well as products available under the trade names SPAN and TWEEN, and combinations thereof.

When included therein, the detergent will usually comprise from about 0 to 10 wt.-% of a semi-polar surfactant. Non-limiting examples of semipolar surfactants include amine oxides (AO) such as alkyl dimethyl amine oxide, N-(coco alkyl)-N,N-dimethyl amine oxide and N-(tallow-alkyl)-N,N-bis-(2-hydroxy ethyl) amine oxide, fatty acid alkanol amides and ethoxylated fatty acid alkanol amides, and combinations thereof.

When included therein, the detergent will usually comprise from about 0 to 10 wt.-% of a zwitterionic surfactant. Non-limiting examples of zwitterionic surfactants include betaine, alkyl dimethyl betaine, sulfo betaine, and combinations thereof.

### Hydrotropes

A hydrotrope is a compound that solubilizes hydrophobic compounds in aqueous solutions (or oppositely, polar substances in a non-polar environment). Typically, hydrotropes have both hydrophilic and a hydrophobic character (so-called amphiphilic properties as known from surfactants); however, the molecular structure of hydrotropes generally do not favor spontaneous self-aggregation. Hydrotropes do not display a critical concentration above which self-aggregation occurs as found for surfactants and lipids forming micellar, lamellar or other well defined meso-phases. Instead, many hydrotropes show a continuous-type aggregation process where the sizes of aggregates grow as concentration increases. However, many hydrotropes alter the phase behavior, stability, and colloidal properties of systems containing substances of polar and non-polar character, including mixtures of water, oil, surfactants, and polymers. Hydrotropes are classically used across industries from pharma, personal care, food, to technical applications. Use of hydrotropes in detergent compositions of the present invention allow more concentrated formulations of surfactants (as in the process of compacting liquid detergents by removing water) without inducing undesired phenomena such as phase separation or high viscosity.

The detergent may comprise 0 to 5 wt.-%, such as 0.5 to 5 wt.-% or 3 to 5 wt.-%, of a hydrotrope. Any hydrotrope known in the art for use in detergents may be utilized. Non-limiting examples of hydrotropes include sodium benzene sulfonate, sodium p-toluene sulfonate (STS), sodium xylene sulfonate (SXS), sodium cumene sulfonate (SCS), sodium cymene sulfonate, amine oxides, alcohols and polyglycol ethers, sodium hydroxy naphthoate, sodium hydroxy naphthalene sulfonate, sodium ethyl hexyl sulfate, and combinations thereof.

### Builders and Cobuilders

The detergent composition of the present invention may comprise about 0 to 65 wt.-%, such as 5 to 45 wt.-%, of a detergent builder or cobuilder, or a mixture thereof. In a dish wash detergent, the level of builder is typically 40 to 65 wt.-%, particularly 50 to 65 wt.-%. The builder and/or cobuilder may particularly be a chelating agent that forms water-soluble complexes with Ca²⁺ and Mg²⁺. Any builder and/or cobuilder known in the art for use in laundry detergents may be utilized. Non-limiting examples of builders include zeolites, diphosphates (pyrophosphates), triphosphates such as sodium triphosphate (STP or STPP), carbonates such as sodium carbonate, soluble silicates such as sodium metasilicate, layered silicates (e.g. SKS-6 from Hoechst), ethanol amines such as 2-amino ethan-1-ol (MEA), diethanolamine (DEA, also known as imino diethanol), triethanolamine (TEA, also known as 2,2',2"-nitrilo triethanol), and carboxy methyl inulin (CMI), and combinations thereof.

The detergent composition of the present invention may also comprise 0 to 20 wt.-%, such as 5 to 10 wt.-%, of a detergent cobuilder, or a mixture thereof. The detergent composition of the present invention may include a cobuilder alone, or in combination with a builder, e.g. a zeolite builder. Non-limiting examples of cobuilders include homopolymers of polyacrylates or copolymers thereof, such as poly (acrylic acid) (PAA) or copoly (acrylic acid/maleic acid) (PAA/PMA). Further non-limiting examples include citrate, chelators such as amino carboxylates, amino polycarboxylates and phosphonates, and alkyl- or alkenyl succinic acid. Additional specific examples include 2,2',2"-nitrilo triacetic acid (NTA), ethylene diamine tetraacetic acid (EDTA), diethylene triamine pentaacetic acid (DTPA), imino disuccinic acid (IDS), ethylene diamine-N,N'-disuccinic acid (EDDS), methyl glycine diacetic acid (MGDA), glutamic acid-N,N-diacetic acid (GLDA), 1-hydroxy ethane-1,1-diphosphonic acid (HEDP), ethylene diamine tetra-(methylene phosphonic acid) (EDTMPA), diethylene triamine pentakis (methylene phosphonic acid) (DTPMPA or DTMPA), N-(2-hydroxy ethyl) imino diacetic acid (EDG), aspartic acid-N-monoacetic acid (ASMA), aspartic acid-N,N-diacetic acid (ASDA), aspartic acid-N-monopropionic acid (ASMP), imino disuccinic acid (IDA), N-(2-sulfo methyl) aspartic acid (SMAS), N-(2-sulfo ethyl) aspartic acid (SEAS), N-(2-sulfo methyl) glutamic acid (SMGL), N-(2-sulfo ethyl) glutamic acid (SEGL), N-methyl imino diacetic acid (MIDA), α-alanine-N,N-diacetic acid (a-ALDA), serine-N,N-diacetic acid (SEDA), isoserine-N,N-diacetic acid (ISDA), phenyl alanine-N,N-diacetic acid (PHDA), anthranilic acid-N,N-diacetic acid (ANDA), sulfanilic acid-N,N-diacetic acid (SLDA), taurine-N,N-diacetic acid (TUDA) and sulfo methyl-N,N-diacetic acid (SMDA), N-(2-hydroxy ethyl) ethylidene diamine-N,N',N'-triacetate (HEDTA), diethanol glycine (DEG), diethylene triamine penta (methylene phosphonic acid) (DTPMP), amino tris (methylene phosphonic acid) (ATMP), and combinations and salts thereof. Further exemplary builders and/or cobuilders are described in e.g. WO 2009/102854, US 5977053.

### Bleaching systems

The detergent may comprise 0 to 50 wt.-%, such as 0.1 to 25 wt.-%, of a bleaching system. Any bleaching system known in the art for use in laundry detergents may be utilized. Suitable bleaching system components include bleaching catalysts, photobleaches, bleach activators, sources of hydrogen peroxide such as sodium percarbonate and sodium perborates, preformed peracids and mixtures thereof.

Suitable preformed peracids include, but are not limited to, peroxy carboxylic acids and salts, percarbonic acids and salts, perimidic acids and salts, peroxy monosulfuric acids and salts, e.g. Oxone^{®}, and mixtures thereof.

Non-limiting examples of bleaching systems include peroxide based bleaching systems, which may comprise, e.g., an inorganic salt, including alkali metal salts such as sodium salts of perborate (usually mono- or tetra-hydrate), percarbonate, persulfate, perphosphate, persilicate salts, in combination with a peracid-forming bleach activator.

The term bleach activator is meant herein as a compound which reacts with peroxygen bleach like hydrogen peroxide to form a peracid. The peracid thus formed constitutes the activated bleach. Suitable bleach activators to be used herein include those belonging to the class of esters amides, imides or anhydrides. Suitable examples are tetra cetyl ethylene diamine (TAED), sodium 4-[(3,5,5-trimethyl hexanoyl) oxy] benzene sulfonate (ISONOBS), diperoxy dodecanoic acid, 4-(dodecanoyl oxy) benzene sulfonate (LOBS), 4-(decanoyl oxy) benzene sulfonate, 4-(decanoyl oxy) benzoate (DOBS), 4-(nonanoyl oxy) benzene sulfonate (NOBS), and/or those disclosed in WO 98/17767. A particular family of bleach activators of interest was disclosed in EP 0624154 and particularly preferred in that family is acetyl triethyl citrate (ATC). ATC or a short chain triglyceride like triacetin has the advantage that it is environmentally friendly as it eventually degrades into citric acid and alcohol. Furthermore, acetyl triethyl citrate and triacetin has a good hydrolytic stability in the product upon storage and it is an efficient bleach activator. Finally, ATC provides a good building capacity to the laundry additive.

Alternatively, the bleaching system may comprise peroxy acids of, e.g., the amide, imide, or sulfone type. The bleaching system may also comprise peracids such as 6-(phthalo imido) peroxy hexanoic acid (PAP).

The bleaching system may also include a bleach catalyst. In some embodiments the bleach component may be an organic catalyst selected from the group consisting of organic catalysts having the following formulae: 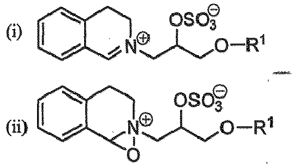 (iii) and mixtures thereof;
wherein each R¹ is independently a branched alkyl group containing from 9 to 24 carbons or linear alkyl group containing from 11 to 24 carbons, preferably each R¹ is independently a branched alkyl group containing from 9 to 18 carbons or linear alkyl group containing from 11 to 18 carbons, more preferably each R¹ is independently selected from the group consisting of 2-propyl heptyl, 2-butyl octyl, 2-pentyl nonyl, 2-hexyl decyl, n-dodecyl, n-tetradecyl, n-hexadecyl, n-octadecyl, isononyl, isodecyl, isotridecyl and isopentadecyl.

Other exemplary bleaching systems are described in e.g. WO 2007/087258, WO 2007/087244, WO 2007/087259 and WO 2007/087242.

Suitable photobleaches may be e.g. sulfonated zinc phthalo cyanine.

### Polymers

The detergent may comprise 0 to 10 wt.-%, such as 0.5 to 5 wt.-%, 2 to 5 wt.-%, 0.5 to 2 wt.-% or 0.2 or 1 wt.-%, of a polymer. Any polymer known in the art for use in detergents may be utilized. The polymer may function as a cobuilder as mentioned above, or may provide anti-redeposition, fiber protection, soil release, dye transfer inhibition, grease cleaning and/or anti-foaming properties. Some polymers may have more than one of the above-mentioned properties and/or more than one of the below-mentioned motifs. Exemplary polymers include carboxy methyl cellulose (CMC), poly (vinyl alcohol) (PVA), poly (vinyl pyrrolidone) (PVP), poly (ethylene glycol) or poly (ethylene oxide) (PEG), ethoxylated poly (ethylene imine), carboxy methyl inulin (CMI), and polycarboxylates such as PAA, PAA/PMA, polyaspartic acid, and lauryl methacrylate/acrylic acid copolymers, hydrophobically modified CMC (HM-CMC) and silicones, copolymers of terephthalic acid and oligomeric glycols, copolymers of poly (ethylene terephthalate) and poly (oxyethene terephthalate) (PET-POET), PVP, poly (vinyl imidazole) (PVI), poly (vinyl pyridine-N-oxide) (PVPO or PVPNO) and polyvinyl pyrrolidone vinyl imidazole (PVPVI). Further exemplary polymers include sulfonated polycarboxylates, polyethylene oxide and polypropylene oxide (PEO-PPO) and diquaternium ethoxy sulfate. Other exemplary polymers are disclosed in, e.g. WO 2006/130575. Salts of the abovementioned polymers are also contemplated.

### Fabric hueing agent

The detergent compositions of the present invention may also comprise fabric hueing agents such as dyes or pigments, which when formulated in detergent compositions of the present invention can deposit onto a fabric when said fabric is contacted with a wash liquor comprising said detergent compositions and thus altering the tint of said fabric through absorption/reflection of visible light. Fluorescent whitening agents emit at least some visible light. In contrast, fabric hueing agents alter the tint of a surface as they absorb at least a portion of the visible light spectrum. Suitable fabric hueing agents include dyes and dye-clay conjugates, and may also include pigments. Suitable dyes include small molecule dyes and polymeric dyes. Suitable small molecule dyes include small molecule dyes selected from the group consisting of dyes falling into the Color Index (C.I.) classifications of Direct Blue, Direct Red, Direct Violet, Acid Blue, Acid Red, Acid Violet, Basic Blue, Basic Violet and Basic Red, or mixtures thereof, as described in e.g. WO 2005/003274, WO 2005/003275, WO 2005/003276 and EP 1876226.

The detergent composition of the present invention preferably comprises from about 0.00003 to 0.2 wt.-%, such as 0.00008 to 0.05 wt.-% or 0.0001 to 0.04 wt.-%, of a fabric hueing agent. The composition may comprise from 0.0001 to 0.2 wt.-% fabric hueing agent, this may be especially preferred when the composition is in the form of a unit dose pouch. Suitable hueing agents are also disclosed in e.g. WO 2007/087257 and WO 2007/087243.

### Additional enzymes

The detergent composition of the present invention may comprise one or more additional enzymes such as proteases, amylases, lipases, cutinases, cellulases, endoglucanases, xyloglucanases, pectinases, pectin lyases, xanthanases, peroxidases, haloperoxygenases, oxidoreductases, catalases and mannanases, or any mixture thereof.

In general, the properties of the selected enzyme(s) should be compatible with the selected detergent, (i.e. pH-optimum, compatibility with other enzymatic and non-enzymatic ingredients, etc.), and the enzyme(s) should be present in effective amounts.

### Cellulases

Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium,* or the fungal cellulases produced from *Humicola insolens, Myceliophthora thermophila* and *Fusarium oxysporum,* disclosed in US 4435307, US 5648263, US 5691178, US 5776757 and WO 89/09259.

Especially suitable cellulases are the alkaline or neutral cellulases having color care benefits. Examples of such cellulases are described in EP 0495257, EP 0531372, WO 96/11262, WO 96/29397, WO 98/08940. Other examples are cellulase variants are described in WO 94/07998, EP 0531315, US 5457046, US 5686593, US 5763254, WO 95/24471, WO 98/12307 and WO 99/01544.

Examples of cellulases exhibiting endo-β-1,4-glucanase activity (EC 3.2.1.4) are described in WO 2002/099091.

Other examples of cellulases include the GH45 cellulases described in WO 96/29397, and especially variants thereof having substitution, insertion and/or deletion at one or more of the positions corresponding to the following positions in SEQ ID NO:8 of WO 2002/099091: 2, 4, 7, 8, 10, 13, 15, 19, 20, 21, 25, 26, 29, 32, 33, 34, 35, 37, 40, 42, 42a, 43, 44, 48, 53, 54, 55, 58, 59, 63, 64, 65, 66, 67, 70, 72, 76, 79, 80, 82, 84, 86, 88, 90, 91, 93, 95, 95d, 95h, 95j, 97, 100, 101, 102, 103, 113, 114, 117, 119, 121, 133, 136, 137, 138, 139, 140a, 141, 143a, 145, 146, 147, 150e, 150j, 151, 152, 153, 154, 155, 156, 157, 158, 159, 160c, 160e, 160k, 161, 162, 164, 165, 168, 170, 171, 172, 173, 175, 176, 178, 181, 183, 184, 185, 186, 188, 191, 192, 195, 196, 200, and/or 20, preferably selected among P19A, G20K, Q44K, N48E, Q119H or Q146R.

Commercially available cellulases include Celluzyme^{™}, and Carezyme^{™} (Novozymes A/S), Clazinase^{™}, and Puradax HA^{™} (Genencor International Inc. / DuPont), and KAC-500(B)^{™} (Kao Corporation).

### Proteases

The protease may be of animal, vegetable or microbial origin, including chemically or genetically modified mutants. Microbial origin is preferred. It may be an alkaline protease, such as a serine protease or a metalloprotease. A serine protease may be of the S1 family, such as trypsin, or the S8 family such as subtilisin. A metalloproteases protease may be a thermolysin from e.g. family M4, M5, M7 or M8.

The term "subtilases" refers to a subgroup of serine protease. Serine proteases are a subgroup of proteases characterized by having a serine in the active site, which forms a covalent adduct with the substrate. The subtilases may be divided into 6 subdivisions, i.e. the Subtilisin family, the Thermitase family, the Proteinase K family, the Lantibiotic peptidase family, the Kexin family and the Pyrolysin family. In one aspect, the protease may be a subtilase, such as a subtilisin or a variant hereof. Further the subtilases (and the serine proteases) are characterized by having two active site amino acid residues apart from the serine, namely a histidine and an aspartic acid residue.

Examples of subtilisins are those derived from *Bacillus* such as subtilisin lentus, *Bacillus lentus,* subtilisin Novo, subtilisin Carlsberg, *Bacillus licheniformis,* subtilisin BPN', subtilisin 309, subtilisin 147 and subtilisin 168, described in WO 89/06279, and protease PD138 (WO 93/18140). Further serine protease examples are described in WO 98/20115, WO 2001/044452, WO 2001/058275, WO 2001/058276, WO 2003/006602 and WO 2004/099401.

An example of a subtilase variants may be those having mutations in any of the positions: 3, 4, 9, 15, 27, 36, 68, 76, 87, 95, 96, 97, 98, 99, 100, 101, 102, 103, 104, 106, 118, 120, 123, 128, 129, 130, 160, 167, 170, 194, 195, 199, 205, 217, 218, 222, 232, 235, 236, 245, 248, 252 and 274 (using the BPN' numbering). More preferred the subtilase variants may comprise one of the mutations selected from the group consisting of S3T, V4I, S9R, A15T, K27R, *36D, V68A, N76D, N87S,R, *97E, A98S, S99G,D,A, S99AD, S101G,M,R, S103A, V104I,Y,N, S106A, G118V,R, H120D,N, N123S, S128L, P129Q, S130A, G160D, Y167A, R170S, A194P, G195E, V199M, V205I, L217D, N218D, M222S, A232V, K235L, Q236H, Q245R, N252K and T274A (using BPN' numbering).

A further preferred protease is the alkaline protease from *Bacillus lentus* DSM 5483, as described in e.g. WO 95/23221, and variants thereof which are described in e.g. WO 92/21760, WO 95/23221, EP 1921147 and EP 1921148.

Examples of trypsin-like proteases are trypsin (e.g. of porcine or bovine origin) and the *Fusarium* protease, described in WO 89/06270 and WO 94/25583. Examples of useful proteases are the variants described in WO 92/19729, WO 98/20115, WO 98/20116 and WO 98/34946, especially the variants with substitutions in one or more of the following positions: 27, 36, 57, 76, 87, 97, 101, 104, 120, 123, 167, 170, 194, 206, 218, 222, 224, 235, and 274.

Examples of metalloproteases are the neutral metalloprotease as described in WO 2007/044993.

Preferred commercially available protease enzymes include Alcalase^{™}, Blaze Evity^{™}, Coronase^{™}, Duralase^{™}, Durazym^{™}, Esperase^{™}, Everlase^{™}, Kannase^{™}, Liquanase^{™}, Liquanase Ultra^{™}, Ovozyme^{™}, Polarzyme^{™}, Primase^{™}, Relase^{™}, Savinase^{™} and Savinase Ultra^{™} (Novozymes A/S), Axapem^{™} (Gist-Brocases N.V.), BLAP and variants thereof (Henkel AG & Co. KGaA), Excellase^{™}, FN2^{™}, FN3^{™}, FN4^{™}, Maxaca^{™}, Maxapem^{™}, Maxatase^{™}, Properase^{™}, Purafast^{™}, Purafect^{™}, Purafect OxP^{™}, Purafect Prime^{™} and Puramax^{™} (Genencor int. / DuPont).

### Lipases and Cutinases

Suitable lipases and cutinases include those of bacterial or fungal origin. Chemically modified or protein engineered mutant enzymes are included. Examples include lipase from *Thermomyces,* e.g. from *T. lanuginosus* (previously named *Humicola lanuginosa*) as described in EP 0258068 and EP 0305216, cutinase from *Humicola,* e.g. *H. insolens* (WO 96/13580), lipase from strains of *Pseudomonas* (some of these now renamed to *Burkholderia*)*,* e.g. *P*. *alcaligenes* or *P*. *pseudoalcaligenes* (EP 0218272), *P*. *cepacia* (EP 0331376), *P*. *sp.* strain SD705 (WO 95/06720 & WO 96/27002), *P. wisconsinensis* (WO 96/12012), GDSL-type *Streptomyces* lipases (WO 2010/065455), cutinase from *Magnaporthe grisea* (WO 2010/107560), cutinase from *Pseudomonas mendocina* (US 5389536), lipase from *Thermobifida fusca* (WO 2011/084412), *Geobacillus stearothermophilus* lipase (WO 2011/084417), lipase from *Bacillus subtilis* (WO 2011/084599), and lipase from *Streptomyces griseus* (WO 2011/150157) and *S. pristinaespiralis* (WO 2012/137147).

Further examples are lipases sometimes referred to as acyltransferases or perhydrolases, e.g. acyltransferases with homology to *Candida antarctica* lipase A (WO 2010/111143), acyltransferase from *Mycobacterium smegmatis* (WO 2005/056782), perhydrolases from the CE 7 family (WO 2009/067279), and variants of the *M. smegmatis* perhydrolase, in particular the S54V variant used in the commercial product Gentle Power Bleach from Huntsman Textile Effects Pte Ltd (WO 2010/100028). Other examples are lipase variants such as those described in EP 0407225, WO 92/05249, WO 94/01541, WO 94/25578, WO 95/14783, WO 95/30744, WO 95/35381, WO 95/22615, WO 96/00292, WO 97/04079, WO 97/07202, WO 2000/034450, WO 2000/060063, WO 2001/092502, WO 2007/087508 and WO 2009/109500.

Preferred commercial lipase products include Lipolase^{™}, Lipex^{™}, Lipolex^{™} and Lipoclean^{™} (Novozymes A/S), Lumafast (Genencor / DuPont) and Lipomax (Gist-Brocades).

### Amylases

The amylase may be an α-amylase, a β-amylase or a glucoamylase and may be of bacterial or fungal origin. Chemically modified or protein engineered mutants are included. Amylases include, e.g., α-amylases obtained from *Bacillus,* e.g. *Bacillus licheniformis* (GB 1296839).

Examples of amylases are those having SEQ ID NO:3 in WO 95/10603 or variants having 90% sequence identity to SEQ ID NO:3 thereof, preferred variants being described in WO 94/02597, WO 94/18314, WO 97/43424; and SEQ ID NO:4 of WO 99/019467, as well as variants with substitutions in one or more of the following positions: 15, 23, 105, 106, 124, 128, 133, 154, 156, 178, 179, 181, 188, 190, 197, 201, 202, 207, 208, 209, 211, 243, 264, 304, 305, 391, 408, and 444 of SEQ ID NO:3 in WO 95/10603.

Further amylases which can be used are amylases having SEQ ID NO:6 in WO 2002/010355 or variants thereof having 90% sequence identity to SEQ ID NO:6. Preferred variants of SEQ ID NO:6 are those having a deletion in positions 181 and 182 and a substitution in position 193.

Other amylase examples are hybrid α-amylases comprising residues 1 to 33 of the α-amylase derived from *B. amyloliquefaciens* shown in SEQ ID NO:6 of WO 2006/066594 and residues 36 to 483 of the *B. licheniformis* α-amylaseshown in SEQ ID NO:4 of WO 2006/066594 or variants having 90% sequence identity thereof. Preferred variants of this hybrid α-amylaseare those having a substitution, a deletion or an insertion in one of more of the following positions: G48, T49, G107, H156, A181, N190, M197, 1201, A209 and Q264. Most preferred variants of the hybrid α-amylase comprising residues 1 to 33 of SEQ ID NO:6 of WO 2006/066594 and residues 36 to 483 of SEQ ID NO:4 of WO 2006/066594 of WO 2006/066594 are those having the substitutions: M197T, H156Y + A181T + N190F + A209V + Q264S or G48 + T49 + G107 + H156 + A181 + N190 + 1201 + A209 + Q264.

Further amylase examples are those having SEQ ID NO:6 in WO 99/19467 or variants thereof having 90% sequence identity to SEQ ID NO:6. Preferred variants of SEQ ID NO:6 are those having a substitution, a deletion or an insertion in one or more of the following positions: R181, G182, H183, G184, N195, 1206, E212, E216 and K269. Particularly preferred amylases are those having deletion in positions G182 + H183 or H183 + G184.

Additional amylases are those having SEQ ID NO:1, SEQ ID NO:2 or SEQ ID NO:7 of WO 96/23873 or variants thereof having 90% sequence identity to SEQ ID NO:1,2 or 7. Preferred variants of SEQ ID NO:1, SEQ ID NO:2 or SEQ ID NO:7 are those having a substitution, a deletion or an insertion in one or more of the following positions: 140, 181, 182, 183, 184, 195, 206, 212, 243, 260, 269, 304 and 476. More preferred variants are those having a deletion in positions 182 + 183 or 183 + 184. Most preferred amylase variants of SEQ ID NO:1, SEQ ID NO:2 or SEQ ID NO:7 are those having a deletion in positions 183 + 184 and a substitution in positions 140, 195, 206, 243, 260, 304 and 476.

Other amylases which can be used are those having SEQ ID NO:2 of WO 2008/153815, SEQ ID NO: 10 in WO 2001/066712 or variants thereof having 90% sequence identity to SEQ ID NO:2 of WO 2008/153815 or 90% sequence identity to SEQ ID NO:10 in WO 2001/066712. Preferred variants of SEQ ID NO:10 in WO 2001/066712 are those having a substitution, a deletion or an insertion in one of more of the following positions: 176, 177, 178, 179, 190, 201, 207, 211 and 264.

Further amylases which can be used are amylases having SEQ ID NO:2 of WO 2009/061380 or variants thereof having 90% sequence identity to SEQ ID NO:2. Preferred variants of SEQ ID NO:2 are those having a substitution, a deletion or an insertion in one of more of the following positions: Q87, Q98, S125, N128, T131, T165, K178, R180, S181, T182, G183, M201, F202, N225, S243, N272, N282, Y305, R309, D319, Q320, Q359, K444 and G475. More preferred variants of SEQ ID NO:2 are those having the substitution in one of more of the following positions: Q87E,R, Q98R, S125A, N128C, T131I, T165I, K178L, T182G, M201L, F202Y, N225E,R, N272E,R, S243Q,A,E,D, Y305R, R309A, Q320R, Q359E, K444E and G475K and/or deletion in position R180 and/or S181. Most preferred amylase variants of SEQ ID NO:2 are those having the substitutions: N128C + K178L + T182G + Y305R + G475K, N128C + K178L + T182G + F202Y + Y305R + D319T + G475K, S125A + N128C + K178L + T182G + Y305R + G475K or S125A + N128C + T131I + T165I + K178L + T182G + Y305R + G475K, wherein the variant optionally further comprises a substitution at position 243 and/or a deletion at position 180 and/or 181.

Other amylases are variants of SEQ ID NO:1 of WO 2016/203064 having at least 75% sequence identity to SEQ ID NO:1 thereof. Preferred variants are variants comprising a modification in one or more positions corresponding to positions 1, 54, 56, 72, 109, 113, 116, 134, 140, 159, 167, 169, 172, 173, 174, 181, 182, 183, 184, 189, 194, 195,206,255,260,262,265,284,289,304,305, 347, 391, 395, 439, 469, 444, 473, 476, or 477 of SEQ ID NO:1, wherein said α-amylase variant has a sequence identity of at least 75% but less than 100% to SEQ ID NO:1.

Other examples of amylases are the α-amylase having SEQ ID NO:12 in WO 2001/066712 or a variant having at least 90% sequence identity to SEQ ID NO:12. Preferred amylase variants are those having a substitution, a deletion or an insertion in one of more of the following positions of SEQ ID NO:12 in WO 2001/066712: R28, R118, N174; R181, G182, D183, G184, G186, W189, N195, M202, Y298, N299, K302, S303, N306, R310, N314; R320, H324, E345, Y396, R400, W439, R444, N445, K446, Q449, R458, N471, N484. Particular preferred amylases include variants having a deletion of D183 and G184 and having the substitutions R118K, N195F, R320K and R458K, and a variant additionally having substitutions in one or more position selected from the group: M9, G149, G182, G186, M202, T257, Y295, N299, M323, E345 and A339, most preferred a variant that additionally has substitutions in all these positions.

Commercially available amylases are Amplify 12L^{™}, Amplify Prime 100L^{™}, Duramyl^{™}, Termamyl^{™}, Fungamyl^{™}, Stainzyme^{™}, Stainzyme Plus^{™}, Natalase^{™} and BAN^{™} (Novozymes A/S), Preferenz S210^{™}, Excellenz S3300^{™}, Rapidase^{™} and Purastar^{™} (Genencor / DuPont).

### Peroxidases/Oxidases

Suitable peroxidases/oxidases include those of plant, bacterial or fungal origin. Chemically modified or protein engineered mutants are included. Examples of useful peroxidases include peroxidases from *Coprinus,* e.g. *C*. *cinereus,* and variants thereof as those described in WO 93/24618, WO 95/10602 and WO 98/15257. Commercially available peroxidases include Guardzyme^{™} (Novozymes A/S).

The detergent enzyme(s) may be included in a detergent composition of the present invention by adding separate additives containing one or more enzymes, or by adding a combined additive comprising all of these enzymes. A detergent additive of the invention, i.e. a separate additive or a combined additive, can be formulated, e.g., as a granulate, liquid, slurry, etc. Preferred detergent additive formulations are granulates, in particular non-dusting granulates, liquids, in particular stabilized liquids, or slurries.

Non-dusting granulates may be produced, e.g. as disclosed in US 4106991 and US 4661452, and may optionally be coated by methods known in the art. Examples of waxy coating materials are poly-(ethylene oxide) products (polyethylene glycol, PEG) with mean molar weights of 1000 to 20000; ethoxylated nonyl phenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of filmforming coating materials suitable for application by fluid bed techniques are given in GB 1483591.

Liquid enzyme preparations may, e.g., be stabilized by adding a polyol such as propylene glycol, a sugar or sugar alcohol, lactic acid or boric acid according to established methods. Protected enzymes may be prepared according to the method disclosed in EP 0238216.

### Adjunct materials

Any detergent components known in the art for use in laundry detergents may also be utilized. Other optional detergent components include anti-corrosion agents, anti-shrink agents, anti-soil redeposition agents, anti-wrinkling agents, bactericides, binders, corrosion inhibitors, disintegrants/disintegration agents, dyes, enzyme stabilizers (including boric acid, borates, CMC and/or polyols such as propylene glycol), fabric conditioners including clays, fillers/processing aids, fluorescent whitening agents/optical brighteners, foam boosters, foam (suds) regulators, perfumes, soil-suspending agents, softeners, suds suppressors, tarnish inhibitors, and wicking agents, either alone or in combination. Any ingredient known in the art for use in laundry detergents may be utilized. The choice of such ingredients is well within the skill of the artisan.

Dispersants: The detergent compositions of the present invention can also contain dispersants. In particular, powdered detergents may comprise dispersants. Suitable water-soluble organic materials include the homo- or copolymeric acids or their salts, in which the polycarboxylic acid comprises at least two carboxyl radicals separated from each other by not more than two carbon atoms. Suitable dispersants are described in e.g. Powdered Detergents, Surfactant science series volume 71, Marcel Dekker, Inc.

Dye Transfer Inhibiting Agents: The detergent compositions of the present invention may also include one or more dye transfer inhibiting agents. Suitable polymeric dye transfer inhibiting agents include, but are not limited to, polyvinyl pyrrolidone polymers, polyamine-N-oxide polymers, copolymers of N-vinyl pyrrolidone and N-vinyl imidazole, polyvinyl oxazolidones and polyvinyl imidazoles or mixtures thereof. When present in a detergent composition, the dye transfer inhibiting agents may be present at levels from about 0.0001 to 10 wt.-%, such as 0.01 to 5 wt.-% or 0.1 to 3 wt.-%.

Fluorescent whitening agent: The detergent compositions of the present invention may also contain additional components that may tint articles being cleaned, such as fluorescent whitening agent or optical brighteners. Where present the brightener is preferably at a level of about 0.01 to 0.5 wt.-%. Any fluorescent whitening agent suitable for use in a laundry detergent composition may be used in the detergent composition. The most commonly used fluorescent whitening agents are those belonging to the classes of diamino stilbene sulphonic acid derivatives, diaryl pyrazoline derivatives and bisphenyl distyryl derivatives. Examples of diamino stilbene sulphonic acid derivative type of fluorescent whitening agents include sodium salts of 4,4'-bis-(2-diethanol amino-4-anilino-s-triazin-6-yl amino) stilbene-2,2'-disulphonate, 4,4'-bis-(2,4-dianilino-s-triazin-6-yl amino) stilbene-2,2'-disulphonate, 4,4'-bis-(2-anilino-4-(N-methyl-N-2-hydroxy ethyl amino)-s-triazin-6-yl amino) stilbene-2,2'-disulphonate, 4,4'-bis-(4-phenyl-2,1,3-triazol-2-yl) stilbene-2,2'-disulphonate, 4,4'-bis-(2-anilino-4-(1-methyl-2-hydroxy ethyl amino)-s-triazin-6-yl amino) stilbene-2,2'-disulphonate and 2-(stilbyl-4"naptho-1,2',4,5)-1,2,3-trizole-2"-sulphonate. Preferred fluorescent whitening agents are Tinopal DMS and Tinopal CBS (Ciba-Geigy AG). Tinopal DMS is the disodium salt of 4,4'-bis-(2-morpholino-4 anilino-s-triazin-6-yl amino) stilbene disulphonate. Tinopal CBS is the disodium salt of 2,2'-bis-(phenyl styryl) disulphonate. Also preferred are fluorescent whitening agents is the commercially available Parawhite KX (Paramount Minerals and Chemicals). Other fluorescers suitable for use in the invention include the 1-3-diaryl pyrazolines and the 7-alkyl amino coumarins. Suitable fluorescent brightener levels include lower levels of from about 0.01 wt.-%, such as 0.05 wt.-%, 0.1 wt.-% or 0.2 wt.-%, to upper levels of 0.5 wt.-% or 0.75 wt.-%.

Soil release polymers: The detergent compositions of the present invention may also include one or more soil release polymers which aid the removal of soils from fabrics such as cotton and polyester based fabrics, in particular the removal of hydrophobic soils from polyester based fabrics. The soil release polymers may be nonionic or anionic terephthalate-based polymers, polyvinyl caprolactam and related copolymers, vinyl graft copolymers, polyester polyamides (see e.g. Chapter 7 in Powdered Detergents, Surfactant science series volume 71, Marcel Dekker, Inc). Another type of soil release polymer is amphiphilic alkoxylated grease cleaning polymers comprising a core structure and a plurality of alkoxylate groups attached to that core structure. The core structure may comprise a polyalkylene imine structure or a polyalkanol amine structure as described in detail in WO 2009/087523. Furthermore, random graft copolymers are suitable soil release polymers Suitable graft copolymers are described in more detail in WO 2007/138054, WO 2006/108856 and WO 2006/113314. Other soil release polymers are substituted polysaccharide structures especially substituted cellulosic structures such as modified cellulose derivatives such as those described in EP 1867808 or WO 2003/040279. Suitable cellulosic polymers include cellulose, cellulose ethers, cellulose esters, cellulose amides and mixtures thereof. Suitable cellulosic polymers include anionically modified cellulose, non-ionically modified cellulose, cationically modified cellulose, zwitterionically modified cellulose, and mixtures thereof. Suitable cellulosic polymers include methyl cellulose, carboxy methyl cellulose, ethyl cellulose, hydroxyl ethyl cellulose, hydroxyl propyl methyl cellulose, ester carboxy methyl cellulose, and mixtures thereof.

Anti-redeposition agents: The detergent compositions of the present invention may also include one or more anti-redeposition agents such as carboxy methyl cellulose (CMC), polyvinyl alcohol (PVA), polyvinyl pyrrolidone (PVP), polyoxyethylene and/or polyethylene glycol (PEG), homopolymers of acrylic acid, copolymers of acrylic acid and maleic acid, and ethoxylated polyethylene imines. The cellulose based polymers described under soil release polymers above may also function as anti-redeposition agents.

Other suitable adjunct materials include, but are not limited to, anti-shrink agents, anti-wrinkling agents, bactericides, binders, carriers, dyes, enzyme stabilizers, fabric softeners, fillers, foam regulators, hydrotropes, perfumes, pigments, suds suppressors, solvents and structurants for liquid detergents and/or structure elasticizing agents.

### Formulation of detergent products

The detergent composition may be in any convenient form, e.g. a bar, a homogenous tablet, a tablet having two or more layers, a pouch having one or more compartments, a regular or compact powder, a granule, a paste, a gel, or a regular, compact or concentrated liquid. There are a number of detergent formulation forms such as layers (same or different phases), pouches, as well as forms for machine dosing unit.

Pouches can be configured as single or multicompartment. It can be of any form, shape and material which is suitable for hold the composition, e.g. without allowing the release of the composition from the pouch prior to water contact. The pouch is made from water soluble film which encloses an inner volume. Said inner volume can be divided into compartments of the pouch. Preferred films are polymeric materials preferably polymers which are formed into a film or sheet. Preferred polymers, copolymers or derivates thereof are selected from polyacrylates, water soluble acrylate copolymers, methyl cellulose, carboxy methyl cellulose, sodium dextrin, ethyl cellulose, hydroxy ethyl cellulose, hydroxy propyl methyl cellulose, malto dextrin, polymethacrylates, most preferably polyvinyl alcohol copolymers and, hydroxy propyl methyl cellulose (HPMC). Preferably the level of polymer in the film is at least about 60%. Preferred average molecular weight will typically be about 20000 to about 150000. Films can also be of blend compositions comprising hydrolytically degradable and water-soluble polymer blends such as polylactide and polyvinyl alcohol (known under the Trade reference M8630 as sold by Chris Craft In. Prod. Of Gary, Ind., US) plus plasticizers like glycerol, ethylene glycerol, propylene glycol, sorbitol and mixtures thereof. The pouches can comprise a solid laundry cleaning composition or part components and/or a liquid cleaning composition or part components separated by the water-soluble film. The compartment for liquid components can be different in composition than compartments containing solids (US 2009/011970).

Detergent ingredients can be separated physically from each other by compartments in water dissolvable pouches or in different layers of tablets. Thereby negative storage interaction between components can be avoided. Different dissolution profiles of each of the compartments can also give rise to delayed dissolution of selected components in the wash solution.

A liquid or gel detergent, which is not unit dosed, may be aqueous, typically containing at least 20 wt.-% and up to 95 wt.-%, such as up to 70 wt.-%, 65 wt.-%, 55 wt.-%, 45 wt.-% or 35 wt.-%, water. Other types of liquids, including without limitation, alkanols, amines, diols, ethers and polyols may be included in an aqueous liquid or gel. An aqueous liquid or gel detergent may contain from 0 to 30 wt.-% organic solvent. A liquid or gel detergent may be non-aqueous.

The enzymes of the invention may be added to laundry soap bars and used for hand washing laundry, fabrics and/or textiles. The term laundry soap bar includes laundry bars, soap bars, combo bars, syndet bars and detergent bars. The types of bar usually differ in the type of surfactant they contain, and the term laundry soap bar includes those containing soaps from fatty acids and/or synthetic soaps. The laundry soap bar has a physical form which is solid and not a liquid, gel or a powder at room temperature. The term solid is defined as a physical form which does not significantly change over time, i.e. if a solid object (e.g. laundry soap bar) is placed inside a container, the solid object does not change to fill the container it is placed in. The bar is a solid typically in bar form but can be in other solid shapes such as round or oval.

### Method of producing the composition

The present invention also relates to methods of producing the composition. The method may be relevant for the (storage) stability of the detergent composition, e.g. soap bar premix method (WO 2009/155557).

### Uses

The present invention is also directed to methods for using the detergent compositions thereof. The present invention may be used for example in any detergent application which requires the degradation of xanthan gum.

### Use to degrade xanthan gum

Xanthan gum has been used as an ingredient in many consumer products including foods and cosmetics and has found use in the oil industry. Therefore, the degradation of xanthan gum can result in improved cleaning processes, such as the easier removal of stains containing gums, such as xanthan gum. Thus, the detergent composition of the present invention comprising endoglucanases and/or xanthan lyases, as described herein, can be used to degrade xanthan gum. Degradation of xanthan gum can preferably be measured using the viscosity reduction assay (e.g. ViPr assay) or alternatively as described in examples 2 and 3 herein.

Endoglucanase activity may alternatively be measured by assessment of reducing ends on xanthan gum pre-treated with xanthan lyase using the colorimetric assay developed by Lever (Anal. Biochem. 47: 273-279, 1972). A preferred embodiment is the use of 0.1% xanthan gum pre-treated with xanthan lyase. Degradation of xanthan gum pre-treated with xanthan lyase may be determined by calculating difference between blank and sample, wherein a difference of more than 0.5 mAU, preferably more than 0.6 mAU, more preferably more than 0.7 mAU or even more preferably more than 0.8 mAU, shows degradation of xanthan gum pre-treated with xanthan lyase.

Xanthan lyase activity may alternatively be measured by assessment of reducing ends liberated from xanthan gum using the colorimetric assay developed by Lever (Anal. Biochem. 47: 273-279, 1972). A preferred embodiment is the use of 0.1% xanthan gum. Degradation of xanthan gum may be determined by calculating difference between blank and sample wherein a difference of more than 0.1 mAU, preferably more than 0.15 mAU, more preferably more than 0.2 mAU or even more preferably more than 0.25 mAU shows degradation of xanthan gum.

Endoglucanase and xanthan lyase activity may alternatively be measured by assessment of reducing ends liberated from xanthan gum using the colorimetric assay developed by Lever (Anal. Biochem. 47: 273-279, 1972). A preferred embodiment is the use of 0.1% xanthan gum. Degradation of xanthan gum may be determined by calculating difference between blank and sample wherein a difference of more than 0.4 mAU, preferably more than 0.5 mAU, more preferably more than 0.6 mAU or even more preferably more than 0.8 mAU shows degradation of xanthan gum.

The invention also relates to methods for degrading xanthan gum comprising applying a detergent composition comprising one or more endoglucanase variants, as described herein, and/or one or more xanthan lyase variants, as described herein.

### Use in detergents

The present invention inter alia relates to the use of detergent compositions comprising endoglucanase variants and/or xanthan lyase variants, as described herein, in cleaning processes such as the laundering of textiles and fabrics (e.g. household laundry washing and industrial laundry washing), as well as household and industrial hard surface cleaning, such as dish wash. For this, the endoglucanase variants and/or xanthan lyase variants may be added to a detergent composition comprising of one or more detergent components.

The endoglucanase variants and/or xanthan lyase variants, as described herein, may be added to and thus become a component of a detergent composition. The detergent composition may be formulated, e.g., as a hand or machine laundry detergent composition for both household and industrial laundry cleaning, including a laundry additive composition suitable for pre-treatment of stained fabrics and a rinse added fabric softener composition, or be formulated as a detergent composition for use in general household or industrial hard surface cleaning operations, or be formulated for hand or machine (both household and industrial) dishwashing operations. In a specific aspect, the present invention relates to a detergent additive comprising endoglucanase variants and/or xanthan lyase variants, as described herein.

The invention also relates to methods for degrading xanthan gum on the surface of a textile or hard surface, such as dish wash, comprising applying a detergent composition, as described herein, to xanthan gum.

It has been contemplated that the use of an endoglucanase variant and/or xanthan lyase variant, as described herein, alone gives an enzyme detergency benefit, preferably an enzyme detergency benefit on xanthan gum.

In some aspects, the invention relates to the use of a detergent composition comprising one or more detergent components and an endoglucanase variant, as described herein, together with a xanthan lyase variant, as described herein.

### Use of enzyme stabilizing component

In a particular aspect, the present invention relates to the use of an enzyme stabilizing component for improving stability of an endoglucanase variant, as described herein, and/or a xanthan lyase variant, as described herein, and/or a mixture thereof in a detergent composition, in particular in a liquid laundry detergent composition, and wherein the enzyme stabilizing component is selected from the group consisting of polyols such as glycerol or 1,2-propylene glycol, alditols such as non-cyclic polyols having the formula HOCH₂[CH(OH)]ₙCH₂OH, mannitol, isomalt, lactit, sorbitol, xylitol, threitol, erythritol or arabitol, alkanol amines such as ethanol amine, or formates such as sodium formate, potassium formate, calcium formate or magnesium formate, preferably said enzyme stabilizing component is selected from glycerol and sodium formate, more preferably being sodium formate.

In a preferred embodiment, the present invention relates to the use of glycerol and/or sodium formate for improving stability of an endoglucanase variant, as described herein, and/or a xanthan lyase variant, as described herein, and/or a mixture thereof in a detergent composition.

In a particular preferred embodiment, the present invention relates to the use of sodium formate for improving stability of an endoglucanase variant, as described herein, and/or a xanthan lyase variant, as described herein, and/or a mixture thereof in a detergent composition.

In a further preferred embodiment, the present invention relates to the use of sodium formate for improving stability of an endoglucanase variant, as described herein, and a xanthan lyase variant, as described herein, in a detergent composition.

In an even preferred embodiment, the present invention relates to the use of sodium formate for improving stability of an endoglucanase variant and/or a xanthan lyase variant in a detergent composition, as described herein,
wherein the endoglucanase has at least 60% and less than 100% sequence identity to SEQ ID NO:1 and having a set of alteration selected from the group consisting of A559N + Y579W + T697G, K512P + A559N + Y579W + T697G, N18G + A71E + A186P + E408D + Y579W + I602T + A651P + A688G + V756Y, N18G + N189K + E408D + A559N + Y579W + A688G + T697G + V756Y + K921R + Y934G, S313D + E408D, R880K + N905D + K921R + Y934G, 1302D + S313D + E408D + Y579W + I602T + A651P + T697G + R880K + K921R + Y934G, and N216Q + S313D + E408D + D476R + Y579W + I602T + F638N + A651P + T697G + W719R + R880K + T887K + K921R + Y934G, wherein said positions correspond to amino acid positions of SEQ ID NO:1;
wherein the xanthan lyase variant has at least 60% and less than 100% sequence identity to SEQ ID NO:2 and having a set of alteration selected from the group consisting of E229N + N672D + P752K + G753E + A769D + L775A + D801G + K875T + N892Y, E229S + N672D + G753E + S754E + A769D + L775A + D801G + K875T + N892Y, E229S + N672D + P752R + G753E + S754E + A769D + L775A + D801G + K875T + N892Y, A190Q + E229S + I234V + A624E + N672D + G753E + S754E + A769D + L775A + D801G + K875T, A190Q + E229S + T631N + N672D + I703L + P752K + G753E + A769D + L775A + D801G + K875T, A190Q + E229S + I234V + S582K + N672D + G753E + S754E + S757D + A769D + L775A + D801G + K875T + N892Y, E229S + N440K + S582K + A624E + S635E + N672D + G738L + G753E + S754E + S757D + A769D + L775A + D801G + K875T + N892Y, and S100D + E229S + K360G + D458S + S582K + N672D + G753E + S754E + S757D + A769D + L775A + D801G + A843P + K875T + N892Y + N1008D, wherein said positions correspond to amino acid positions of SEQ ID NO:2.

### EXAMPLES

### Example 1: Construction, expression and purification of endoglucanase variants and xanthan lyase variants

Endoglucanase variants, as described herein, and xanthan lyase variants, as described herein, have been constructed and produced as described in WO 2018/037064, WO 2018/037065 and WO 2019/162000 (all three applications are incorporated herein by reference).

### Example 2: Detergent stability assay for endoglucanase

GH9 endoglucanase (EG) activity (EC 3.2.1.4) was determined by reducing ends on xanthan gum pre-treated with xanthan lyase using the colorimetric assay developed by Lever (Anal. Biochem. 47: 273-279, 1972). Pre-treated xanthan gum is a modified form of the xanthan sugar, where the terminal pyruvated mannose from side chains is removed (prepared according to Nankai et al. (1999) from the source Keltran). The GH9 mature parent endoglucanase and its variants cleave at beta-(1,4)-glucosyl bonds in the glucan backbone of pretreated xanthan gum releasing glucans with a reducing end which can be determined by reaction with p-Hydroxybenzoic acid hydrazide (PAHBAH). The increase of color is proportional to the enzyme activity.Enzyme sample was mixed with detergent (as exemplified in Table 1) and stored at 30°C for 8 weeks. After incubation, the enzyme-detergent sample was 100-fold diluted in assay buffer (50 mM MOPS, 4 mM CaCl₂, 0.01 % Triton X-100, pH 7.0).

To assess the enzymatic activity, 50 µl of diluted enzyme-detergent sample was mixed with 50 µl 4 mg/ml modified xanthan gum. The samples had then been incubated at 50°C for 1 hour. Finally, the level of reducing ends is estimated by adding 75 µl PAHBAH solution (15 mg/ml PAHBAH, 50 g/L potassium sodium tartrate tetrahydrate, 20 g/L NaOH), and the samples had then been incubated at 95°C for 10 minutes. After cooling down to room temperature, the absorbance at 405 nm was measured.

**Table 1. Liquid laundry detergent matrix**

| | **% Active matter raw material** | **% Active matter in formula** |
|---|---|---|
| Water demin. | 100 | 2-8 |
| 1,2-Propandiol | 100 | 3-7 |
| Glycerol | 99.5 | 7-11 |
| Monoethanolamin | | 4-8 |
| LAS (anionic surfactant) | 96 | 12-28 |
| FAEO (nonionic surfactant) | 100 | 12-28 |
| Alkanolamine alkoxylated | 100 | 3-7 |
| palm kernel oil fatty acid | 100 | 3-8 |
| Polyethylene imine | 80 | 2-6 |
| CP propylene glycol terephthalate liquid | 100 | 1-4 |
| sodium disulfite | 98.6 | 0.01-0.3 |
| DTPMP-xNa7 | 40 | 0.1-1.0 |
| Optical brightener | 90 | 0.1-1 |
| Perfume | 100 | 1-5 |
| Dye | t.q. | 0.001-0.005 |
| Without enzymes; Dosage: 1 g/L; pH = 8 | | |

### Example 3: Detergent stability assay for xanthan lyase

Enzyme sample was mixed with detergent (as exemplified in Table 1) and stored at 30°C for 8 weeks. After incubation, the enzyme-detergent sample was 50-fold diluted in dilution buffer (50 mM MOPS, 1 mM CaCl₂, pH 7.5).

To measure the enzyme activity of diluted enzyme-detergent samples, 50 µl of diluted samples were mixed with 50 µl of freshly prepared substrate solution (0.4% w/v xanthan gum was freshly prepared by dissolving 400 mg of xanthan gum in 100 ml of Milli Q water) and incubated at 40°C for 1 hour.

After incubation, 75 µl of PAHBAH reagent (1.5% PAHBAH reagent was freshly prepared by dissolving 1.5 g of p-hydroxy benzoic acid hydrazide in a stock solution mix (stock solution mix containing 1.0 M Na₂CO₃, 0.17 M potassium sodium tartrate and 5 mM (Bi(NO₃)₃ x 5H₂O) was prepared by dissolving 106.99 g of Na₂CO₃, 47.98 g of potassium sodium tartrate and 2.42 mg of (Bi(NO₃)₃ x 5H₂O) in Milli Q water for a final volume of 1000 ml)) was added, and the sample was incubated at 90°C for 10 minutes, followed by subsequent cooling at 10°C. Afterwards, absorbance was measured at 405 nm (Infinite M1000 reader; TECAN, Switzerland).

### Example 4: Residual activity

The in-detergent stability was determined by measuring the enzymatic activity present in detergent fresh, after the enzymes have been added to the detergent and also after storage of the enzymes containing detergent at 30°C.

The residual activity (RA) was calculated as the percentage of enzymatic activity remaining after incubation at 30°C relative to the enzymatic activity after freshly formulating the enzyme.

**Table 2. Residual activity**

| | Enzyme stabilizing component | Xanthan lyase activity after 8 weeks [%] | Endoglucanase activity after 8 weeks [%] |
|---|---|---|---|
| Detergent matrix S1 | None | 43% | 15% |
| Detergent matrix S2 | 5 wt.-% glycerol | 73% | 20% |
| Detergent matrix S3 | 1 wt.-% sodium formate | 76% | 61% |

| | | | |
|---|---|---|---|
| S1: Detergent matrix as shown in Table 1 with 0.7 wt.-% (t.q.) of enzyme mix containing endoglucanase and xanthan lyase, as described herein; pH = 8 S2: Detergent matrix as shown in Table 1 with additional 5 wt.-% glycerol and 0.7 wt.-% (t.q.) of enzyme mix containing endoglucanase and xanthan lyase; pH = 8 S3: Detergent matrix as shown in Table 1 with 1 wt.-% sodium formate and 0.7 wt.-% (t.q.) of enzyme mix containing endoglucanase and xanthan lyase; pH = 8 | | | |

All three samples, S1, S2 and S3, had been stored at 30°C for 8 weeks. After storage, residual activity of endoglucanase and xanthan lyase had been determined as described above.

From Table 2, it can be seen that endoglucanase as well as xanthan lyase can be stabilized by rather low sodium formate concentrations, whereas elevated glycerol concentration only has a stabilizing effect on xanthan lyase.

Using as low concentration of enzyme stabilizer as possible is especially advantageous in compacted dosage forms, like in particular unit dose, pouches and caps. In this respect, sodium formate shows surprisingly good stabilizing results while being applied in rather low concentration.

## Claims

1. A detergent composition comprising
(A) an endoglucanase variant, comprising an alteration, preferably a substitution, at one or more positions in one or more regions selected from the group consisting of: region 7 corresponding to amino acids 612 to 660 of SEQ ID NO:1, region 1 corresponding to amino acids 95 to 105 of SEQ ID NO:1, region 2 corresponding to amino acids 115 to 138 of SEQ ID NO:1, region 3 corresponding to amino acids 210 to 251 of SEQ ID NO:1, region 4 corresponding to amino acids 267 to 301 of SEQ ID NO:1, region 5 corresponding to amino acids 339 to 361 of SEQ ID NO:1, region 6 corresponding to amino acids 547 to 595 of SEQ ID NO:1, region 8 corresponding to amino acids 806 to 828 of SEQ ID NO:1, region 9 corresponding to amino acids 839 to 1042 of SEQ ID NO:1, region 10 corresponding to amino acids 1 to 94 of SEQ ID NO:1, region 11 corresponding to amino acids 106 to 114 of SEQ ID NO:1, region 12 corresponding to amino acids 139 to 209 of SEQ ID NO:1, region 13 corresponding to amino acids 252 to 266 of SEQ ID NO:1, region 14 corresponding to amino acids 302 to 338 of SEQ ID NO:1, region 15 corresponding to amino acids 362 to 546 of SEQ ID NO:1, region 16 corresponding to amino acids 596 to 611 of SEQ ID NO:1, region 17 corresponding to amino acids 661 to 805 of SEQ ID NO:1, region 18 corresponding to amino acids 829 to 838 of SEQ ID NO:1, and region 19 corresponding to amino acids 1043 to 1055 of SEQ ID NO:1; and/or
(B) a xanthan lyase variant, comprising an alteration, preferably a substitution, at one or more positions in one or more regions selected from the group consisting of: region 3 corresponding to amino acids 731 to 803 of SEQ ID NO:2, region 1 corresponding to amino acids 154 to 176 of SEQ ID NO:2, region 2 corresponding to amino acids 614 to 658 of SEQ ID NO:2, region 4 corresponding to amino acids 807 to 846 of SEQ ID NO:2, region 5 corresponding to amino acids 872 to 885 of SEQ ID NO:2, region 6 corresponding to amino acids 903 to 1004 of SEQ ID NO:2, region 7 corresponding to amino acids 1 to 153 of SEQ ID NO:2, region 8 corresponding to amino acids 177 to 613 of SEQ ID NO:2, region 9 corresponding to amino acids 659 to 730 of SEQ ID NO:2, region 10 corresponding to amino acids 804 to 806 of SEQ ID NO:2, region 11 corresponding to amino acids 847 to 871 of SEQ ID NO:2, region 12 corresponding to amino acids 886 to 902 of SEQ ID NO:2, and region 13 corresponding to amino acids 1005 to 1037 of SEQ ID NO:2; and
(C) an enzyme stabilizing component;
wherein the endoglucanase variant (A) has at least 60% and less than 100% sequence identity to SEQ ID NO:1; preferably said endoglucanase variant has activity on xanthan gum pre-treated with xanthan lyase;
wherein the xanthan lyase variant (B) has at least 60% and less than 100% sequence identity to SEQ ID NO:2, preferably said xanthan lyase variant having an activity on xanthan gum; and
wherein the enzyme stabilizing component is selected from the group consisting of polyols such as glycerol or 1,2-propylene glycol, alditols such as non-cyclic polyols having the formula HOCH₂[CH(OH)]ₙCH₂OH, mannitol, isomalt, lactit, sorbitol, xylitol, threitol, erythritol or arabitol, alkanol amines such as ethanol amine, or formates such as sodium formate, potassium formate, calcium formate or magnesium formate.

2. The detergent composition according to claim 1, wherein said endoglucanase variant has at least 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93&, 94%, 95%, 96%, 97%, 98% or 99% and less than 100% sequence identity to SEQ ID NO:1.

3. The detergent composition according to any one of claims 1 to 2, wherein said endoglucanase variant comprises an alteration, preferably a substitution, at one or more positions selected from the group consisting of 4, 17, 18, 20, 51, 53, 55, 56, 60, 63, 71, 79, 87, 92, 99, 120, 125, 126, 130, 137, 182, 186, 189, 192,213,216,221,226,228,230,231,232,233,235,240,243,247,249, 278, 279, 281, 283, 285, 289, 292, 294, 298, 302, 311, 313, 333, 346, 353, 358, 386, 387, 388, 390, 403, 408, 410, 416, 441, 448, 451, 471, 472, 476, 489, 507, 512, 515, 538, 555, 556, 557, 558, 559, 560, 561, 562, 563, 564, 567, 568, 570, 575, 578, 579, 580, 581, 583, 589, 590, 591, 592, 593, 595, 598, 599, 602, 603, 605, 607, 609, 616, 627, 630, 631, 635, 636, 638, 639, 640, 641, 642, 643, 644, 651,676,683,688,690,694,698,699,706,711,713,719,720,744,749,754,756,760,781,786, 797, 810, 811, 812, 815, 823, 824, 825, 827, 828, 833, 834, 835, 837, 843, 848, 868, 869, 870, 871, 872,873,874,880,881,883,884,885,887,888,890,892,894,898,905,906,912,920,921,924, 926,927,928,932,933,934,935,937,938,939,940,941,942,943,946,948,950,952,953,954, 956,957,960,966,971,972,980,989,991,994,995,998,999,1006,1009,1010,1011,1029,1030, 1031, 1032, 1035, 1037, 1038, 1040, 1041, 1042, 1044, 1045 and 1048, preferably at one or more positions selected from the group consisting of 17, 20, 216, 283, 302, 311, 313, 408, 476, 579, 602, 627, 636, 638, 651, 688, 697, 719, 756, 880, 881, 883, 887, 906, 921, 934, 937, and 956, wherein said positions correspond to amino acid positions of SEQ ID NO:1.

4. The detergent composition according to any one of claims 1 to 3, wherein said endoglucanase variant comprises one or more amino acid substitutions selected from the group consisting of V4T, S17A, N18G, F20P, F20N, F20G, F20Y, K51Q, K51H, E53Y, E53P, E53G, Y55M, Y55D, V56M, Y60F, S63F, A71E, T87R, T92S, K99R, A120P, I125V, A126R, S128X, N129D, K130R, F137L, H182Y, A186P, N189K, K192N, K213R, N216D, N216Q, N216R, A221R, L226K, K228E, K228I, G230F, G230L, G230A, G230H, G230N, G230W, G230T, F231Y, F231N, V232R, V232G, L233H, H235D, N240Q, G243K, G243R, D247N, A249N, A278S, G279E, K281F, K281V, K281Y, K281H, K281Q, K281N, K281W, K281T, K281R, A283D, N285G, N285L, N285M, N285S, N285P, N285T, N285Y, N285H, N285K, N285D, N285W, N285R, Q289E, T292A, T292F, T292L, T292I, T292V, T292S, T292P, T292Y, T292Q, T292N, T292K, T292D, T292G, A294V, F297L, Q298E, I302D, I302H, I302V, I302M, H311N, S313D, W333L, A346H, A346D, T353D, A386P, I387T, K388R, K390Q, I403Y, E408D, E408N, E408S, E408P, E408A, E408G, P410G, Q416S, Q416D, N441G, A448E, A448W, A448S, K451Q, K451S, G471S, S472Y, D476R, Q489P, K507R, K512P, S515V, S538C, L555Q, G556S, G557R, N558P, N558F, N558K, N558D, N558M, N558Q, N558I, N558Y, N558H, N558E, A559N, A559F, A559M, A559P, A559Y, A559H, A559Q, A559D, A559R, A559G, A559I, A559S, A559K, S560F, S560P, S560K, S560G, S560D, T561E, T561Q, T561S, T561D, T561P, G562W, G562P, G563E, A564I, A564Y, A564H, A564Q, A564K, A564E, A564D, E565M, V567F, V567P, K568R, L569F, L569Y, L569D, L569E, P570F, P570L, P570I, P570M, P570V, P570S, P570T, P570A, P570Y, P570H, P570Q, P570N, P570K, P570E, P570W, P570R, P570G, I575V, I575M, I575A, I575D, I575E, I576F, I576M, I576P, D578R, Y579F, Y579W, V580L, T581M, D583M, Q589G, P590S, P590T, P590E, E591L, G592D, S593P, S593H, S593Q, S593N, S593K, S593D, S593E, S593R, S595L, S598Q, A599S, I602T, I602D, V603P, S605T, S607C, G609E, S616G, S616D, K627L, K627M, K627V, K627S, K627T, K627Q, K627R, I630F, I630V, I630Y, K631R, K631A, T633V, D635L, D635M, D635T, D635A, D635P, D635N, D635K, D635E, D635G, D635W, S636L, S636M, S636A, S636H, S636Q, S636N, S636K, S636R, F638N, F638Y, T639D, F638I, F638V, F638T, F638L, F638H, F638M, T639V, T639S, T639L, T639I, T639M, T639A, T639E, T639W, T639G, T639Y, T639P, T640S, Y641E, S642T, S642N, N643D, N643H, N643T, T644F, A651P, A651S, D676H, Q683E, A688G, Y690F, T694A, T697G, R698W, T699A, T706Q, T711S, T711V, T711Y, K713R, W719R, K720H, K744H, K744Q, A749T, K754R, V756Y, V756H, S760G, T781M, N786K, T797S, S810Q, S810R, A811S, V812F, V812I, V812M, V812W, V812R, N815V, N815Y, N815E, N815W, N815R, S823Q, A824T, A824D, T825G, T825N, T825W, T825A, T825D, V827I, V827M, V827S, N828D, N833D, Q834E, S835A, S835D, V837I, T843V, N848D, A868E, A869V, D870F, D870L, D870I, D870M, D870V, D870S, D870T, D870Y, D870H, D870Q, D870N, D870K, D870E, D870W, D870R, D870G, P871F, P871L, P871I, P871M, P871V, P871S, P871T, P871A, P871Y, P871H, P871Q, T872S, T872F, T872A, T872Y, T872H, T872Q, T872N, T872K, T872D, T872E, T872W, T872R, T872G, D873K, D873E, T874V, T874S, T874P, T874A, T874H, T874Q, T874N, T874K, R880K, V881Q, V881T, T883R, T883V, T883C, T883K, Y884H, A885F, A885Q, A885N, T887L, T887I, T887S, T887H, T887R, T887K, L888M, V890R, T892P, T892V, K894E, R898Q, N905D, F906A, Q912V, N920P, N920D, K921R, K921E, K921D, A924D, V926F, V926P, K927R, S928D, T932A, N933V, N933S, Y934G, Y934M, Y934S, Y934A, Y934Q, Y934N, Y934E, Y934W, Y934R, T935W, A937F, A937V, A937S, A937T, A937Q, A937D, A937E, V938I, K939I, K939V, D940E, N941S, N941H, N941D, A942P, A942E, D943Y, D943H, G946R, K948R, K948E, R950V, R950H, R950N, F952S, F952W, N953Y, G954L, Q956Y, Q956S, A957L, A957P, Y960F, A964N, A964C, N966P, N966C, G971A, T972K, Q974K, Q974C, M980I, Q989I, Q991L, Q991I, Q991M, Q991V, Q991T, Q991K, Q991C, G994D, G994N, S995I, S995V, S995Q, S995R, S995C, G998V, G998A, T999R, S1006T, S1006A, S1006K, S1006R, K1009E, Y1010W, L1011M, L1011S, L1011A, L1011Q, L1011N, L1011D, L1011E, R1029N, F1030M, K10311, K1031S, K1031T, K1031H, V1032G, K1035A, A1037E, A1037W, S1038L, S1038I, S1038G, L1040N, L1040E, G1041F, G1041R, Y1042N, L1044F, L1044S, L1044N, L1044W, P1045Q, P1045W, and F1048W, preferably selected from the group consisting of S17A, F20P, N216D, N216Q, A283D, I302D, H311N, S313D, E408D, D476R, Y579W, I602T, K627R, S636N, S636K F638I, F638N, A651P, A688G, T697G, W719R, V756Y, R880K, V881Q, T883R, T887K, F906A, K921R, Y934G, A937E, and Q956Y, wherein said positions correspond to amino acid positions of SEQ ID NO:1.

5. The detergent composition according to any one of claims 1 to 4, wherein said endoglucanase variant comprises a set of alterations selected from the group consisting of A559K + N558K + S560F + T561P + G562W, A559N + P570Q, A559N + P570T, A559N + Y579F, A559N + Y579F + K627R, A559N + Y579W, A559N + Y579W+ K99R, A559N + Y579W+ S128X + K627R, A559N + Y579W + S128X + S636N, A559N + Y579W + K281R, A559N + Y579W + Q416D + S636N, A559N + Y579W + S560P, A559N + Y579W + A564I, A559N + Y579W + P570N, A559N + Y579W + P570K, A559N + Y579W + P570R, A559N + Y579W + P570A, A559N + Y579W + P570T, A559N + Y579W + P570S, A559N + Y579W + P570Q, A559N + Y579W+ P570H, A559N + Y579W+ S616D, A559N + Y579W + S616D + K627R, A559N + Y579W + S616D + S636K, A559N + Y579W + S616D + A651P, A559N + Y579W + S616D + K921R, A559N + Y579W+ S616D + K921R + Y934G, A559N + Y579W + K627R, A559N + Y579W + K627R + S636N + A651P, A559N + Y579W + K627R + S636N + K921R, A559N + Y579W + K627R + K921R, A559N + Y579W + K627M, A559N + Y579W + K627M + A651P, A559N + Y579W + S636N, A559N + Y579W + S636N + A651P, A559N + Y579W + S636N + K921R, A559N + Y579W + S636K + A651P, A559N + Y579W + S636K + K921R, A559N + Y579W + S636L, A559N + Y579W + F638I, A559N + Y579W + A651P, A559N + Y579W + A651P + K921R + Y934G, A559N + Y579W + A651P + Y934G, A559N + Y579W + D870M, A559N + Y579W + K921R, A559N + Y579W + K921R + A651P, A559N + Y579W + K921R + Y934G, A559N + Y579W + Y934G, A559N + Y579W + K948E, A559N + Y579W + K1009E, A559N + K627R, Y579F + A564E, Y579W + K99R, Y579W + G562P, Y579W+ A564D, Y579W + P570K, Y579W + P570Q, Y579W + K627R, Y579W + S636N, Y579W + F638I, Y579W + A651P, Y579W + K921R, Y579W + Y934G, Y579W + K948E, Y579W + K1009E, K627R + K51Q, K627R + K451S, K627R + P570Q, K627R + P570T, K627R + S636N, K627R + F638I, K627R + A885F, K627R + Y934G, S636N + P570T, S636N + K921R, S636N + Y934G, F638I + P570K, F6381 + P570Q, F6381 + P570T, F6381 + A651P, F6381 + Y934G, F6381 + K921R, A651P + P570Q, A651P + P570T, A885F + P570T, A885F + Y934G, K921R + P570Q, K921R + P570T, Y934G + P570T, preferably selected from the group of A559N + Y579W + T697G, K512P + A559N + Y579W + T697G, N18G + A71E + A186P + E408D + Y579W + I602T + A651P + A688G + V756Y, N18G + N189K + E408D + A559N + Y579W + A688G + T697G + V756Y + K921R + Y934G, S313D + E408D, R880K + N905D + K921R + Y934G, I302D + S313D + E408D + Y579W + I602T + A651P + T697G + R880K + K921R + Y934G, and N216Q + S313D + E408D + D476R + Y579W + I602T + F638N + A651P + T697G + W719R + R880K + T887K + K921R + Y934G, wherein said positions correspond to amino acid positions of SEQ ID NO:1.

6. The detergent composition according to any one of claims 1 to 5, wherein said endoglucanase variant has activity on xanthan gum pre-treated with xanthan lyase, preferably said activity is a xanthan gum degrading activity, further preferably said xanthan gum degrading activity is endoglucanase EC 3.2.1.4 activity.

7. The detergent composition according to any one of claims 1 to 6, wherein said xanthan lyase variant has at least 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% and less than 100% sequence identity to SEQ ID NO:2.

8. The detergent composition according to any one of claims 1 to 7, wherein said xanthan lyase variant comprises an alteration, preferably a substitution, at one or more positions selected from the group consisting of 9, 15, 18, 46, 58, 66, 89, 95, 100, 106, 109, 155, 159, 183, 188, 190, 203, 204, 221,229,234,238,240,242,243,257,258,284,291,293,316,317,320,324,329,333,339,341, 352,354,360,372,377,399,400,419,440,450,451,454,458,481,492,505,533,567,568,576, 578,579,582,620,624,626,631,635,649,650,656,664,672, 703, 722, 726, 727, 728, 738, 745, 752,753,754,757,769,775,777,779,782,786,789,792,796,799,801,819,824,843,851,855, 856, 867, 875, 887, 892, 899, 900, 901, 902, 903, 911, 912, 915, 919, 921, 923, 925, 927, 928, 930, 933, 941, 966, 991, 1008 and 1016, wherein said positions correspond to amino acid positions of SEQ ID NO:2.

9. The detergent composition according to any one of claims 1 to 8, wherein said xanthan lyase variant comprises one or more amino acid substitutions selected from the group consisting of K9R, N15T, T18D, L46D, A58L, S66H, Q89Y, K95E, S100D, N106Y, Q109R, Q109D, Q109F, Q109K, Q109A, Y155E, A159P, K183Q, K183R, V188I, A190Q, A203P, K204R, A221P, E229N, E229S, E229V, I234V, I238W, I238L, I238M, I240W, N242S, G243V, Y257W, R258E, R284G, K291R, A293G, A293P, K316R, R317K, K320R, L324Q, K329R, K333R, L339M, I341P, V352I, S354P, K360G, K360R, Q372H, F377Y, N399K, K400R, F419Y, N440K, D450P, K451E, K451R, A454V, D458S, K481R, A492H, A492L, T505I, L533I, K567R, G568A, S578K, S578N, S578R, S579R, S579K, S582K, K620R, A624E, A626Q, T631N, S635E, S635T, T649V, T649K, Q650G, I656V, T664K, N672D, I703L, I722F, P726Q, T727P, M728V, G738L, K745R, P752R, P752K, G753E, G753Q, G753S, S754E, S754L, S754Q, S754R, S757D, S757P, S757E, A769D, A769T, A769R, L775A, L775F, L775I, L775M, L775Q, L775S, L775Y, D777R, P779V, Y782I, N786K, G789R, K792W, K792Y, K792V, K792A, N796Q, A799H, D801G, K819R, K824R, A843P, S851F, K855R, E856D, P867S, K875T, K875E, K887R, N892Y, N892W, N892F, G899S, I900G, D901A, T902F, T903A, T903Q, A911M, A911V, A911S, A912T, A912I, A912Y, T915S, T915V, T915Q, T919D, T919F, T919G, T921R, T921S, T923D, T923H, T925D, T925Q, T925R, T927K, D928W, Y930F, Y930H, Y930L, D933M, G941D, G941E, A966P, N991D, V998K, N1008D and K1016T, wherein said positions correspond to amino acid positions of SEQ ID NO:2.

10. The detergent composition according to any one of claims 1 to 9, wherein said xanthan lyase variant comprises an alteration at one or more positions selected from the group consisting of 624, 631, 635, 649, 656, 738, 752, 753, 754, 757, 769, 775, 777, 800, 801, 843, 875, 911 and 915, and/or an alteration at one or more positions selected from the group consisting of 89, 100, 190, 229, 234, 352, 360, 399, 440, 458, 492, 567, 582, 664, 672, 703, 728, 892, 1008 and 1016, wherein said positions correspond to amino acid positions of SEQ ID NO:2.

11. The detergent composition according to any one of claims 1 to 10, wherein said xanthan lyase variant comprises one or more substitutions selected from the group consisting of Q89Y, S100D, A190Q, E229S, I234V, V3521, K360G, N399K, N440K, D458S, A492H, A492L, K567R, S582K, T664K, N672D, I703L, M728V, N892Y N1008D and K1016T, and/or one or more substitutions selected from the group consisting of A624E, T631N, S635E, T649K, I656V, G738L, P752K, P752R, G753E, S754E, S754R, S757D, A769D, L775A, D777R, V800P, D801G, A843P, K875T, A911V and T915A, wherein said positions correspond to amino acid positions of SEQ ID NO:2.

12. The detergent composition according to any one of claims 1 to 11, wherein said xanthan lyase variant comprises a set of alterations selected from the group consisting of: E229N + N672D + P752K + G753E + A769D + L775A + D801G + K875T + N892Y, E229S + N672D + G753E + S754E + A769D + L775A + D801G + K875T + N892Y, E229S + N672D + P752R + G753E + S754E + A769D + L775A + D801G + K875T + N892Y, A190Q + E229S + I234V + A624E + N672D + G753E + S754E + A769D + L775A + D801G + K875T, A190Q + E229S + T631N + N672D + I703L + P752K + G753E + A769D + L775A + D801G + K875T, A190Q + E229S + I234V + S582K + N672D + G753E + S754E + S757D + A769D + L775A + D801G + K875T + N892Y, E229S + N440K + S582K + A624E + S635E + N672D + G738L + G753E + S754E + S757D + A769D + L775A + D801G + K875T + N892Y, and S100D + E229S + K360G + D458S + S582K + N672D + G753E + S754E + S757D + A769D + L775A + D801G + A843P + K875T + N892Y + N1008D, wherein said positions correspond to amino acid positions of SEQ ID NO:2.

13. The detergent composition according to any one of claims 1 to 12, wherein the enzyme stabilizing component is selected from the group consisting of glycerol and formates, preferably being sodium formate.

14. The detergent composition according to any one of claims 1 to 13, comprising
(A) an endoglucanase variant having at least 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% and less than 100% sequence identity to SEQ ID NO:1 and having a set of alteration selected from the group consisting of:
(A1) A559N + Y579W + T697G,
(A2) K512P + A559N + Y579W + T697G,
(A3) N18G + A71E + A186P + E408D + Y579W + I602T + A651P + A688G + V756Y,
(A4) N18G + N189K + E408D + A559N + Y579W + A688G + T697G + V756Y + K921R + Y934G,
(A5) S313D + E408D,
(A6) R880K + N905D + K921R + Y934G,
(A7) I302D + S313D + E408D + Y579W + I602T + A651P + T697G + R880K + K921R + Y934G, and
(A8) N216Q + S313D + E408D + D476R + Y579W+ I602T + F638N + A651P + T697G + W719R + R880K + T887K + K921R + Y934G,
wherein said positions correspond to amino acid positions of SEQ ID NO:1;
(B) a xanthan lyase variant having at least 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% and less than 100% sequence identity to SEQ ID NO:2 and having a set of alteration selected from the group consisting of:
(B1) E229N + N672D + P752K + G753E + A769D + L775A + D801G + K875T + N892Y,
(B2) E229S + N672D + G753E + S754E + A769D + L775A + D801G + K875T + N892Y,
(B3) E229S + N672D + P752R + G753E + S754E + A769D + L775A + D801G + K875T + N892Y,
(B4) A190Q + E229S + I234V + A624E + N672D + G753E + S754E + A769D + L775A + D801G + K875T,
(B5) A190Q + E229S + T631N + N672D + I703L + P752K + G753E + A769D + L775A + D801G + K875T,
(B6) A190Q + E229S + I234V + S582K + N672D + G753E + S754E + S757D + A769D + L775A + D801G + K875T + N892Y,
(B7) E229S + N440K + S582K + A624E + S635E + N672D + G738L + G753E + S754E + S757D + A769D + L775A + D801G + K875T + N892Y, and
(B8) S100D + E229S + K360G + D458S + S582K + N672D + G753E + S754E + S757D + A769D + L775A + D801G + A843P + K875T + N892Y + N1008D,
wherein said positions correspond to amino acid positions of SEQ ID NO:2; and
(C) an enzyme stabilizing component, wherein the enzyme stabilizing component is selected from the group consisting of glycerol and formates, preferably being sodium formate.

15. The detergent composition according to claim 14, wherein the endoglucanase variant and/or the xanthan lyase variant do not comprise any further substitution.

16. The detergent composition according to any one of claims 1 to 15, wherein said endoglucanase variant and/or said xanthan lyase variant has improved stability in presence of said enzyme stabilizing component, in particular sodium formate and/or glycerol.

17. The detergent composition according to any one of claims 1 to 16, wherein said detergent compositions comprises 0.000001 to 10 wt.-%, such as 0.00001 to 5 wt.-%, preferably 0.0001 to 1 wt.-%, more preferably 0.0005 to 0.5 wt.-%, even more preferably 0.001 to 0.1 wt.-%, of said endoglucanase variant and/or 00.000001 to 10 wt.-%, such as 0.00001 to 5 wt.-%, preferably 0.0001 to 1 wt.-%, more preferably 0.0005 to 0.5 wt.-%, even more preferably 0.001 to 0.1 wt.-%, of said xanthan lyase variant and/or 0.001 to 20 wt.-%, such as 0.005 to 15 wt.-%, preferably 0.01 to 10 wt.-%, more preferably 0.1 to 5.5 wt.-%, even more preferably 0.25 to 1.5 wt.-%, of said enzyme stabilizing agent; and/or wherein said detergent composition comprises said endoglucanase variant and/or said xanthan lyase variant and said enzyme stabilizing agent in a weight ratio (enzyme:stabilizer) of 0.00001:1 to 0.1:2, preferably 0.0001:1 to 0.01:1, more preferably 0.0005:1 to 0.001:1.

18. The detergent composition according to any one of claims 1 to 17, wherein the detergent composition is an automatic dishwashing composition, hand dishwashing composition or laundry detergent composition, in particular a laundry detergent composition, preferably a liquid laundry detergent composition.

19. The detergent composition according to any one of claims 1 to 18, wherein said detergent compositions is preferably in unit dose, in particular pouch or caps.

20. Use of a detergent composition according to any of claims 1 to 19, wherein said use is in a laundering or dishwashing process or said use is for degrading xanthan gum.

21. Methods of degrading xanthan gum using a detergent composition according to any one of claims 1 to 19, wherein xanthan gum is on the surface of a hard surface or a textile.

22. Use of an enzyme stabilizing component for improving stability of an endoglucanase variant and/or a xanthan lyase variant and/or a mixture thereof in a detergent composition, in particular in a liquid laundry detergent composition, wherein the endoglucanase variant and/or the xanthan lyase variant is/are as defined as in any of the claims 1 to 19, and wherein the enzyme stabilizing component is selected from the group consisting of polyols such as glycerol or 1,2-propylene glycol, alditols such as non-cyclic polyols having the formula HOCH₂[CH(OH)]ₙCH₂OH, mannitol, isomalt, lactit, sorbitol, xylitol, threitol, erythritol or arabitol, alkanol amines such as ethanol amine, or formates such as sodium formate, potassium formate, calcium formate or magnesium formate.

23. Use of an enzyme stabilizing component for improving stability of an endoglucanase variant and/or a xanthan lyase variant and/or a mixture thereof in a detergent composition according to claim 22,
wherein the endoglucanase has at least 60% and less than 100% sequence identity to SEQ ID NO:1 and having a set of alteration selected from the group consisting of A559N + Y579W+ T697G, K512P + A559N + Y579W+ T697G, N18G + A71E + A186P + E408D + Y579W+ I602T + A651P + A688G + V756Y, N18G + N189K + E408D + A559N + Y579W+ A688G + T697G + V756Y + K921R + Y934G, S313D + E408D, R880K + N905D + K921R + Y934G, I302D + S313D + E408D + Y579W + I602T + A651P + T697G + R880K + K921R + Y934G, and N216Q + S313D + E408D + D476R + Y579W+ I602T + F638N + A651P + T697G + W719R + R880K + T887K + K921R + Y934G, wherein said positions correspond to amino acid positions of SEQ ID NO:1;
wherein the xanthan lyase variant has at least 60% and less than 100% sequence identity to SEQ ID NO:2 and having a set of alteration selected from the group consisting of E229N + N672D + P752K + G753E + A769D + L775A + D801G + K875T + N892Y, E229S + N672D + G753E + S754E + A769D + L775A + D801G + K875T + N892Y, E229S + N672D + P752R + G753E + S754E + A769D + L775A + D801G + K875T + N892Y, A190Q + E229S + I234V + A624E + N672D + G753E + S754E + A769D + L775A + D801G + K875T, A190Q + E229S + T631N + N672D + I703L + P752K + G753E + A769D + L775A + D801G + K875T, A190Q + E229S + I234V + S582K + N672D + G753E + S754E + S757D + A769D + L775A + D801G + K875T + N892Y, E229S + N440K + S582K + A624E + S635E + N672D + G738L + G753E + S754E + S757D + A769D + L775A + D801G + K875T + N892Y, and S100D + E229S + K360G + D458S + S582K + N672D + G753E + S754E + S757D + A769D + L775A + D801G + A843P + K875T + N892Y + N1008D, wherein said positions correspond to amino acid positions of SEQ ID NO:2; and
wherein the enzyme stabilizing component is glycerol and/or sodium formate, preferably being sodium formate.

24. Use of an enzyme stabilizing component for improving stability of an endoglucanase variant and/or a xanthan lyase variant and/or a mixture thereof in a detergent composition according to claim 22 or 23, wherein said endoglucanase variant is present in an amount of 0.001 to 20 wt.-%, such as 0.005 to 15 wt.-%, preferably 0.01 to 10 wt.-%, more preferably 0.1 to 5.5 wt.-%, even more preferably 0.25 to 1.5 wt.-%, and/or said xanthan lyase variant is present in an amount of 00.001 to 20 wt.-%, such as 0.005 to 15 wt.-%, preferably 0.01 to 10 wt.-%, more preferably 0.1 to 5.5 wt.-%, even more preferably 0.25 to 1.5 wt.-%, and/or said enzyme stabilizing agent is present in an amount of 0.001 to 20 wt.-%, such as 0.005 to 15 wt.-%, preferably 0.01 to 10 wt.-%, more preferably 0.1 to 5.5 wt.-%, even more preferably 0.25 to 1.5 wt.-%; and/or wherein said endoglucanase variant and/or said xanthan lyase variant and said enzyme stabilizing agent are present in a weight ratio (enzyme:stabilizer) of 0.00001:1 to 0.1:2, preferably 0.0001:1 to 0.01:1, more preferably 0.0005:1 to 0.001:1.
